(19) Europäisches Patentamt
European Patent Office
Office européen des brevets

(11) EP 1 938 843 A1

(12) EUROPEAN PATENT APPLICATION

(43) Date of publication:
**02.07.2008 Bulletin 2008/27**

(51) Int Cl.:
***A61K 48/00*** *(2006.01)* ***A61K 47/48*** *(2006.01)*

(21) Application number: **06256450.5**

(22) Date of filing: **19.12.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **novosom AG**
**06120 Halle (DE)**

(72) Inventors:
- **Panzner, Steffen**
**06114 Halle (DE)**
- **Reinsch, Christian**
**06110 Halle (DE)**
- **Siepe, Evgenios**
**04155 Leipzig (DE)**

(74) Representative: **Crump, Julian Richard John et al**
**Mintz Levin Cohn Ferris Glovsky & Popeo**
**Intellectual Property LLP**
**The Rectory**
**9 Ironmonger Lane**
**London EC2V 8EY (GB)**

# (54) Lipids and lipid assemblies comrising transfection enhancer elements

(57) Use of lipid assemblies comprising one or more lipids with one or more transfection enhancer elements ("TEE") for the in vivo, in vitro or ex-vivo transfection of cells wherein said lipids have the general formula (I) and said transfection enhancer elements have the general formula (II).

The pH sensitive hydrophilic element may be located distally from the link between the lipid and the TEE or centrally within the TEE. Preferably, the pH sensitive hydrophilic element comprises weak acids having a pKa of between 2 and 6.

EP 1 938 843 A1

## Description

### Field of the invention

This disclosure describes structural elements that enhance fusogenicity of lipids and lipid assemblies (e.g. liposomes) with biological membranes, in particular cell membranes, and use of such structures. The elements are pH sensitive in terms of charge and hydrophilicity and undergo a polar - apolar transition when exposed to low pH.

### Background of the invention

The fusion of membranes is a common event in biological systems and nature has developed elegant mechanisms for that. For example, the infection of a cell by a virus is one event in which fusion of membranes plays a key role.
It is therefore not surprising that the ability of viruses to promote fusion with cellular or endosomal membranes led to the development of recombinant viral gene delivery systems. The most prominent systems rely on retroviral-, adenoviral-, adeno-associated viral- or herpes simplex viral vectors, which are employed in more than 70% of clinical gene therapy trials worldwide. Although virus based gene delivery systems are very efficient, they show immune-related side effects after the injection. This major drawback limits the safety of these systems and consequently their applicability in humans (e.g. Thomas et al., Nature Reviews, Genetics, 4, 346-358, 2003).
An alternative is the use of nonviral vectors to deliver genetic material into cells. Nonviral vector systems include, for example, cationic polymers and liposomes. Liposomes are artificial single, oligo or multilamellar vesicles having an aqueous core and being formed from amphipathic molecules. The cargo may be trapped in the core of the liposome or disposed in the membrane layer or at the membrane surface. Today, liposomal vectors are the most important group of the nonviral delivery systems. More specifically, cationic liposomes or lipids have been used widely in animal trials and/or clinical studies. Although cationic systems provide high loading efficiencies, they lack colloidal stability, in particular after contact with body fluids. Ionic interactions with proteins and/or other biopolymers lead to in situ aggregate formation with the extracellular matrix or with cell surfaces. Cationic lipids have often been found to be toxic as shown by Filion, et al. in BBA, 1329(2), 345-356, 1997,; Dass in J. Pharm. Pharmacol, 54(5), 593-601, 2002; Hirko, et al. in Curr. Med. Chem., 10(14), 1185-1193, 2003.
Amphoteric liposomes represent a recently described class of liposomes having an anionic or neutral charge at pH 7.5 and a cationic charge at pH 4. WO 02/066490, WO 02/066012 and WO 03/070735, all to Panzner, et al. and incorporated herein by reference, give a detailed description of amphoteric liposomes and suitable lipids therefor. Further disclosures are made in WO 03/070220 and WO 03/066489, also to Panzner, et al. and incorporated herein by reference, which describe further pH sensitive lipids for the manufacture of such amphoteric liposomes.
Amphoteric liposomes can encapsulate nucleic acid molecules with high efficiency equal to cationic liposomes. Advantageously, amphoteric liposomes are much better tolerated upon administration in vivo and show a favourable biodistribution over cationic liposomes.
Compared to viral gene delivery vectors the non-viral systems are much safer; they are tolerated at high doses and do not elicit an immune response, therefore these systems can be administered repetitively. Still, viral systems are superior in terms of transfection efficacy. Attempts have been made to incorporate viral surface glycoproteins into liposomes (Miller N, Vile R., FASEB J, 9, 190-199, 1995) but these hybrid systems again have the drawback of immune-related side effects.
This creates a constant need for improvements of non-viral systems. The present invention relates to an improvement of non-viral carrier systems for active pharmaceutical ingredients that base on a hydrophile-hydrophobe transition in response to an acidification of the environment. Such mechanism is also known in the nature. For example, influenza viruses use a specific fusion mechanism. After the virus is internalized into the cell by receptor-mediated endocytosis, the viral envelope fuses with the endosomal membrane which leads to a release of the viral genome into the cytosol of the infected cell. This fusion event is catalyzed by the viral envelope glycoprotein hemagglutinin. The trigger of the fusion is the acidic pH within the endosomal compartment leading to a conformational change of the hemagglutinin. Concomitantly, the N-terminal fusion peptide of the HA2 chain undergoes a hydrophobic shift due to protonation of carboxyl groups in the amino acid side chain. The hydrophobic peptide can insert into the target membrane which leads to destabilization and subsequent fusion (e.g. Stegmann et al., EMBO J., 9(13), 4231-4241, 1990).

### Objects of the invention

A first object of the present invention is to provide transfection enhancer elements (TEE's) that improve uptake of lipid assemblies and sequestered active ingredients into cells.
A second object of the present invention is to provide novel lipids comprising one or more transfection enhancer elements.

A third object of the present invention is to provide lipid assemblies comprising lipids with transfection enhancer elements for the in vivo, in vitro or ex vivo transfection of cells.
An aspect of the third object of the present invention is to provide amphoteric lipid assemblies comprising lipids with transfection enhancer elements.
A fourth object of the invention is to provide pharmaceutical compositions comprising liposomes further comprising lipids with transfection enhancer elements as carriers for the delivery of active agents or ingredients, including drugs such as nucleic acid drugs, e.g., oligonucleotides and plasmids.
A fifth object of the present invention is the use of the pharmaceutical compositions for the treatment or prophylaxis of inflammatory, immune or autoimmune disorders and/or cancer of humans or non-human animals.
A sixth object of the present invention is to provide compositions and methods for the treatment of humans or non-human animals in which the pharmaceutical composition comprising an active agent is targeted to a specific organ or organs, tumours or sites of infection or inflammation.
The use of transfection enhancer elements in combination with lipid assemblies for the improvement of transfection in vivo, in vitro and ex vivo represents aspects of the objects of the present invention.
Methods for the improvement of in vivo, in vitro of ex vivo transfection of lipid assemblies or sequestered active ingredients represent further aspects of the objects of the present invention.

**Brief description of the invention**

It has been found that lipid assemblies, in particular liposomes, comprising one or more lipids with one or more transfection enhancer elements (TEE's) can be used to efficiently transfect cells *in vitro, in vivo* or *ex vivo*. According to the present invention, lipids comprising transfection enhancer elements (TEE's) have the general formula (I), the structural element of a TEE being defined as in (II):

(I) Lipid- Hydrophobic element - pH sensitive hydrophilic element

(II) hydrophobic element - pH sensitive hydrophilic element

The position of the hydrophilic element within the TEE structure may vary. In some aspects, the hydrophilic element is located distal from the link between lipid and TEE. In other aspects, the hydrophilic element is located central within the TEE.
In some embodiments said pH-responsive hydrophilic element comprises weak acids having a pKa of between 2 and 6, preferred of between 3 and 5. Said weak acids may be selected from carboxyl groups, barbituric acid and derivatives thereof, xanthine and derivatives thereof.
In other embodiments said pH-responsive hydrophilic element may be a zwitterionic structure comprising a combination of weak or strong acidic groups with weak bases, the latter having a pKa of between 3 and 8, preferred of between 4.5 and 7. Suitably said zwitterionic structures may be formed from an anionic group and a heterocyclic nitrogen atom as cationic group.
To achieve specific pKa's of said hydrophilic elements in one aspect of the invention said pH-responsive hydrophilic element may be substituted with structural elements, selected from the group comprising hydroxymethyl-, hydroxyethyl-, methoxymethyl-, methoxyethyl-, ethoxymethyl-, ethoxyethyl-, thiomethyl-, thioethyl-, methylthiomethyl-, methylthioethyl-, ethylthiomethyl-, ethylthioethyl-, chlorid-, chlormethyl- vinyl-, phenyl-, benzyl-, methyl-, ethyl- , propyl-, isopropyl- and tert-butyl or cyclohexyl groups.
In some embodiments said hydrophobic element comprise linear, branched or cyclic chains with a minimum chain length of 6 elements. In one aspect of this embodiment said hydrophobic element comprises more than 6 and up to 40 elements, in a second aspect said hydrophobic element comprises between 6 and 20 elements and in a third aspect said hydrophobic element comprises between 20 and 40 elements.
The chain elements of said hydrophobic element may be carbon atoms. In one embodiment said hydrophobic element can be saturated or may contain unsaturated bonds. In other embodiments said hydrophobic element may be substituted.
In some embodiments the branching of the main chain of said hydrophobic element may comprise rather small building blocks. Preferred building blocks comprise methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof.
Alternatively, said hydrophobic element may derive from sterols, said sterols may be further substituted.
It is possible to insert one or more heteroatoms or chemical groups into the hydrophobic element of the pH-responsive transfection enhancer elements (TEE's). Such heteroatoms or chemical groups may be selected from -O-, -S-, -N(H)C(O)-, -C(O)O-, -OC(O)N(H)-, - C(O)-, -C(O)-N(H)-, -N(H)-C(O)-O-, -CH=N-, -O-C(O)-, -N=CH- and/or -S-S-, amino acids or derivatives thereof, $\alpha$-hydroxyacids or $\beta$-hydroxy acids.

TEE's undergo a hydrophile-hydrophobe transition in response to an acidification of the environment. This transition is mediated by the hydrophilic elements described above that are responsive towards pH.

In some embodiments of the invention logD(4.0)-logD(7.4)>=1 for the transfection enhancer elements and logD at pH 7,4 is between 1 and 10.

In most aspects of the invention, the logD at pH 4 of said pH-responsive transfection enhancer elements (TEE's) exceeds 0.

Of course, said pH-responsive transfection enhancer elements (TEE's) may contain more than one pH responsive hydrophilic element.

In one aspect of the present invention the transfection enhancer elements may be chemically linked to a lipid and in one specific embodiment of this aspect the TEE's may be linked or grafted to the polar head group of said lipid. In a further embodiment of this aspect the lipids may include chemical linkers between the graft and the pH sensitive transfection enhancer elements. Furthermore the polar headgroup of the lipid may be further substituted. In yet other embodiment of this aspect said lipids may contain more than one hydrophilic polar head group or complex hydrophilic head groups that allow substitution on various positions without affecting hydrophilicity.

The lipid assemblies of the present invention may be formed from a lipid phase further comprising neutral and/or cationic and/or anionic lipids and the overall charge of said lipid assemblies can be neutral, cationic or anionic.

In one aspect of the present invention the lipid assemblies are liposomes and in a specific embodiment of this aspect the liposomes are amphoteric liposomes of various size and lamellarity. Said amphoteric liposomes may be formed from a lipid phase comprising one or more amphoteric lipids or from a lipid phase comprising (i) a stable cationic lipid and a chargeable anionic lipid, (ii) a chargeable cationic lipid and chargeable anionic lipid or (iii) a stable anionic lipid and a chargeable cationic lipid.

In a further aspect of the invention said TEE's are complexed with said lipid assemblies using ionic interactions. In one embodiment said TEE's may be linked to a polycationic element and combined with anionic lipid assemblies and in another embodiment said TEE's may be linked to a polyanionic element and combined with cationic lipid assemblies.

The lipid assemblies of the present invention may be sequester active pharmaceutical ingredients and in a specific embodiment said pharmaceutical ingredients are nucleic acid-based drugs, like oligonucleotides, polynucleotides or DNA plasmids.

A still further aspect of the present invention relates to lipids comprising one ore more transfection enhancer elements (TEE's) according to the formula (I)

Lipid- Hydrophobic element - pH sensitive hydrophilic element (I)

Said pH-responsive hydrophilic element may comprise weak acids having a pka of between 2 and 6, preferred of between 3 and 5 or is a zwitterionic structure comprising a combination of weak or strong acidic groups with weak bases having a pka of between 3 and 8, preferred of between 4.5 and 7. Said hydrophobic element may comprise linear, branched or cyclic chains with a minimum chain length of 6 elements.
Said lipids may be other than one of the following structures (III)

PE - amid linkage - X - COOH (III)

wherein X is a carbon containing linear chain having a chain length of between 3 to 20 atoms and having various degrees of saturation and/or heteroatom compositions and/or substituents and the COOH-group or (IV)

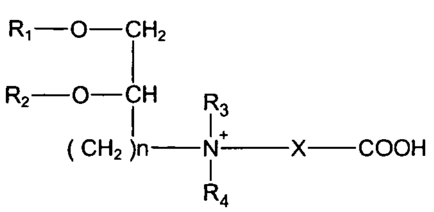

(IV)

wherein X is a straight saturated alkyl chain having a chain length of between 2 and 10 C-atoms and $R_1$, $R_2$, $R_3$ and $R_4$ are independently linear or branched, unsubstituted or substituted $C_{1-23}$ alkyl, acyl, alkylene, heteroalkyl groups having 0 to 6 sites of unsaturation, cyclic and aryl groups, the groups comprising from 0 to 5 heteroatoms, in which the substituent groups are $-O-(CH2)_x-CH_3$; $-S-(CH_2)_x-CH_3$; $X-(CH_2)_k$, wherein X is a halide, and $-N((CH_2)_k-CH_3)_2$, wherein the alkyl groups of the substituents comprise from 0-2 heteroatoms, and k is 0-4 and wherein $R_1$ and $R_2$ can further be independ-

ently H and n is 1 to 6
or(V)

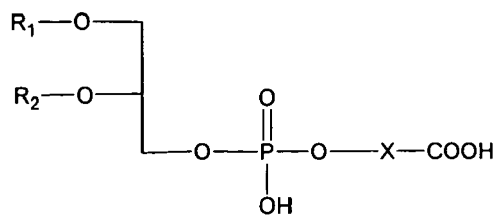

(V)

wherein $R_1$ and $R_2$ independently are hydrogen atoms or $C_1$-$C_{24}$ straight chain or branched alkyl or acyl chains optionally containing double and triple bonds and wherein X is an aliphatic and/or cycloaliphatic hydrocarbon chain with 6-20 carbon-atoms optionally substituted by aryl rests, cycloalkyls with 3-6 carbon atoms, hydroxyl and/or further carboxylic functions.

The TEE's may be chemically linked to the lipid moiety as described above. In a preferred embodiment of the invention said lipids are selected from the group comprising compounds (30) to (69).

Furthermore the invention relates to lipid assemblies comprising one or more of such lipids. In some embodiments said lipid assemblies may sequester active pharmaceutical ingredients. In one embodiment said pharmaceutical ingredients are nucleic acid-based drugs, like DNA plasmids, polynucleotides and oligonucleotides.

A still further aspect of the invention relates to amphoteric liposomes comprising one or more lipids with one or more transfection enhancer elements according to the general formula (I)

Lipid- Hydrophobic element - pH sensitive hydrophilic element (I)

In some embodiments said amphoteric liposomes may sequester active pharmaceutical ingredients. In one embodiment of this aspect said pharmaceutical ingredient include nucleic acid based drugs, like DNA plasmids, polynucleotides and oligonucleotides.

In yet another aspect of the present invention there is provided a pharmaceutical composition comprising active agent-sequestered lipid assemblies or amphoteric liposomes in accordance with the present invention and a pharmaceutically acceptable vehicle therefor.

In yet another aspect, the present invention comprehends the use of a pharmaceutical composition according to the present invention for the treatment or prophylaxis of inflammatory, immune or autoimmune disorders and/or cancer of humans or non-human animals.

For clarity, the following definitions and understandings are used for important terms of the invention:

Transfection

...is used widely to specifically describe the disappearance of a concentration gradient across a biological membrane. It comprises transport across, or diffusion through, penetration or permeation of biological membranes irrespective of the actual mechanism by which said processes occur.

LogP

...is the ratio of the respective concentrations of a compound in the 1-octanol and water partitions of a two-phase system at equilibrium. The octanol-water partition coefficient (logP) is used to describe the lipophilic or hydrophobic properties of a compound.

LogD

...is the ratio of the equilibrium concentrations of all species (unionized and ionized) of a molecule in 1-octanol to same species in the water phase.

The partition coefficient for dissociative mixtures, logD, is defined as follows:

$logD = \log (\Sigma(c_i^{H2O})/\Sigma (C_i^{org}))$, where

$c_i^{H2O}$ is the concentration of the i-th microspecies in water and

$C_i^{org}$ is the concentration of the i-th microspecies in the organic phase.

LogD differs from logP in that ionized species are considered as well as the neutral form of the molecule. LogD is therefore the logP at a given pH of the medium. LogD at pH 7.4 is often quoted to give an indication of the lipophilicity of a drug at the pH of blood plasma.

LogP and logD values can be determined experimentally by measuring the partition of a molecule or its ionized forms in octanol/water systems. Experimental values have been generated for a vast amount of individual compounds and expert systems allow extrapolating logP and logD values for novel species. One such expert system is ACD/Labs

with the modules ACD/LogP or ACD/logD and ACD/Labs 7.06 has been used for calculations within this disclosure.

"nucleic acid" or "polynucleotide"

.. as used herein refers to any nucleic acid containing molecule, including without limitation, DNA or RNA. The term polynucleotide(s) generally refers to any polyribonucleotide or polydeoxyribonucleotide, which may be unmodified RNA or DNA or modified RNA or DNA. Thus, for instance, polynucleotides as used herein refers to, among others, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions.

Oligonucleotide

... as used herein is defined as a molecule with two or more deoxyribonucleotides or ribonucleotides, often more than three, and usually more than ten. The exact size of an oligonucleotide will depend on many factors, including the ultimate function or use of the oligonucleotide. Oligonucleotides can be prepared by any suitable method, including, for example, cloning and restriction of appropriate sequences and direct chemical synthesis by a method such as the phosphotriester method of Narang et al., Meth. Enzymol., 68, 90-99, 1979; the phosphodiester method of Brown et al., Method Enzymol., 68, 109-151, 1979 the diethylphosphoramidite method of Beaucage et al., Tetrahedron Lett., 22, 1859-1862, 1981 the triester method of Matteucci et al., J. Am. Chem. Soc., 103, 3185-3191, 1981 or automated synthesis methods; and the solid support method of US 4,458,066.

Lipid assemblies

...are supramolecular assemblies comprising amphipathic molecules. In some aspects the amphipathic substances are known as lipids or as detergents, in other aspects such substances are known to form biological membranes or to insert into biological membranes. The supramolecular assemblies may further comprise oils from apolar molecules. The supramolecular assemblies of the current invention therefore comprise liposomes of various size and lamellarity, micelles, inverted micelles, cubic or hexagonal lipid phases, cochleates, emulsions, double emulsions or other multimeric assemblies that are substantially build from lipids, oils or amphiphiles.

## Detailed description of the invention

It has been found that lipid assemblies, in particular liposomes, comprising one or more lipids with one or more transfection enhancer elements can be used to efficiently transfect cells in vitro, in vivo or ex vivo. According to the present invention, lipids comprising transfection enhancer elements (TEE's) have the general formula (I), the structural element of a TEE being defined as in (II):

(I) Lipid- Hydrophobic element - pH sensitive hydrophilic element

(II) Hydrophobic element - ph sensitive hydrophilic element

The TEE (II) itself is a structure comprising the hydrophobic element and one or more pH sensitive hydrophilic elements. For the scope of this invention, pH sensitive elements become less hydrophilic when the pH is changed from neutrality to acidic conditions.

In some cases, liposomes comprising lipids under (I) have been described in the art for the purpose of coupling surface-bound molecules such as proteins or other molecules to the liposomes. In these cases, a terminal carboxyl group is employed as a coupling group and the hydrophobic element provides spatial separation between the lipid bilayer and said coupling group. The surface bound molecules may be e.g. therapeutic drugs or targeting moieties. For example, WO 86/04232 of Kung et al. discloses liposome compositions containing coupling groups in their outer bilayer region to bind such surface bound molecules. The coupling reagent disclosed by Kung et al. include a phosphatidylethanolamine lipid moiety, a carbon containing spacer arm of up to 20 atoms and a terminal carboxyl group.

A related approach is disclosed in WO 97/19675 of Wheeler. This application relates to cationic lipids of a Rosenthal inhibitor (RI) core structure having an alkyl linking group with up to 10 carbon atoms, e.g. a carboxyalkyl group. RI structures with additional carboxyalkyl groups are disclosed as intermediates for further functionalisation, e.g. with ligands for cellular uptake, therapeutic molecules or groups that can increase the polar charge density of the cationic lipids.

The prior art has also described lipids comprising long hydrophobic elements lacking a hydrophilic group (US 6,294,191). These structures are substantially different from the present disclosure in that they do not react to changes in pH.

Prior art is silent to the use and optimization of structural elements (II) or lipids (I) to enhance cellular uptake and cytosolic delivery of liposomes and sequestered active ingredient. Prior art has not taught, that the combination of hydrophobic elements with the pH sensitive hydrophilic elements provides criticality to such function.

As described above it was found that lipid assemblies comprising one or more lipids with one or more transfection

enhancer elements may be used to efficiently transfect cells. Mechanistically, the pH sensitive hydrophilic moieties of the TEE become protonated at acidic pH. This in turn leads to a decrease of the polarity of the functional group. It is possible, that the lipids (I) or parts thereof, e.g. the TEE in its entirety or fractions thereof can insert into lipid bilayers provided in trans, thereby promoting fusion events. It is also possible that the structural element (II) upon acidification inserts into its own lipid bilayer, thereby creating structural defects that improve fusogenicity and cellular transfection.

Liposomal delivery of drugs (e.g. nucleic acids) into the cytoplasm of cells very often needs a successful escape from the endosomes, which have an acidic pH. So, within the scope of the present invention such lipids with structural element (II) may enable or improve the transfection ability of liposomes, irrespective of their charge.

Although a number of explanations can be given to explain the findings of the invention, an understanding of the exact mechanism whereby the enhanced fusion or transfection is achieved is not necessary for practicing the invention and even other mechanisms, not described here, may be involved.

It is known in the prior art that the pH sensitive hydrophilic moieties can be used for engrafting ligands or active ingredients to a liposomes. Although not excluded here, the scope of the present invention is transfection with liposomes.

<u>Hydrophilic elements of TEE's</u>

In one aspect of this invention the hydrophilic elements are weak acids that provide a response in hydrophilicity between pH values of about 4 and the physiological pH of 7.4. Carboxyl groups, barbituric acid or derivatives thereof, in particular xanthine or derivatives thereof of formula (1) to (3) in table 1a represent, but do not limit such pH-responsive hydrophilic elements.

Table 1a: Compounds 1-3

| | |
|---|---|
| (1) 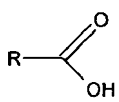 | (1) Carboxylic acids. R represents the hydrophobic element of the invention. |
| (2) 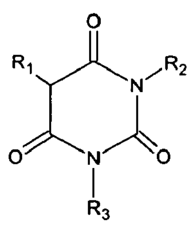 | (2) Barbituric acid derivatives. $R_1$, $R_2$ or $R_3$ may represent the hydrophobic element of the invention. |
| (3) 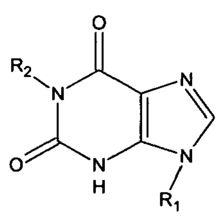 | (3) Xanthine derivatives. $R_1$ or $R_2$ represent the hydrophobic element of the invention. |

LogD values for hydrophilic head groups derived from (1) to (3) are high at low pH and low at neutral or higher pH.

Other derivatives of xanthins, pyrimidins (uracils) or barbituric acids are disclosed below in table 1b and analyzed with respect to their logD values at pH 4.0 and pH 7.4. The methoxyethyl moiety in compounds (100) to (129) represents or may be replaced by the hydrophobic elements of the TEE as described above.

Table 1b: Compounds 100-129

| Chemical structure | Compound-# | logD (pH 4) | logD (pH 7.4) |
|---|---|---|---|
| 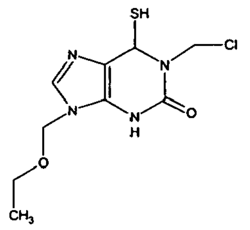 | (100) | -0,11 | -1,03 |
| 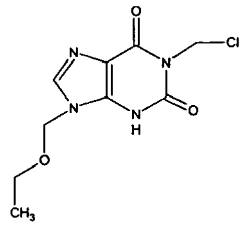 | (101) | -0,39 | -2,18 |
| 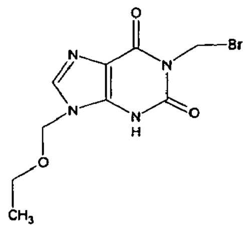 | (102) | 0,08 | -1,71 |
| 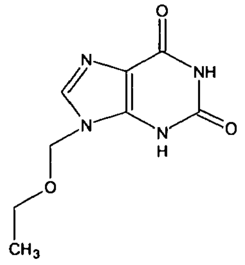 | (103) | -0,85 | -2,13 |
| 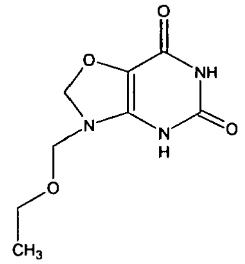 | (104) | -0,27 | -0,62 |
| 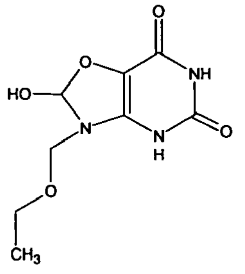 | (105) | -0,18 | -0,77 |

(continued)

| Chemical structure | Compound-# | logD (pH 4) | logD (pH 7.4) |
|---|---|---|---|
| 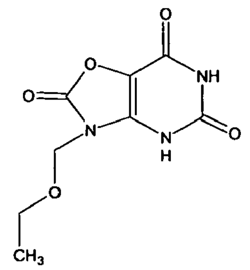 | (106) | -2,76 | -3,44 |
| 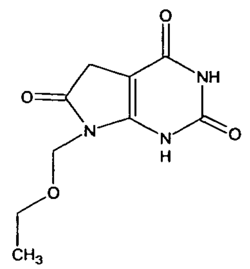 | (107) | -0,1 | -1,88 |
| 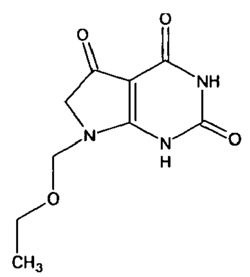 | (108) | -0,93 | -1,27 |
| 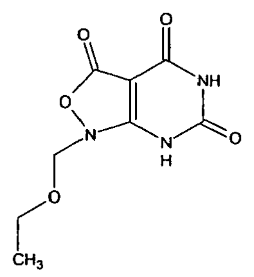 | (109) | -2,01 | -3,43 |
| 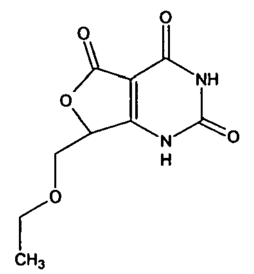 | (110) | -1,34 | -2,16 |

(continued)

| Chemical structure | Compound-# | logD (pH 4) | logD (pH 7.4) |
|---|---|---|---|
| 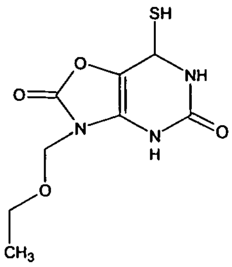 | (111) | 0,92 | -1,31 |
| 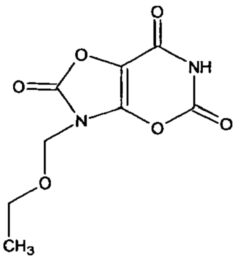 | (112) | -1,34 | -3,15 |
| 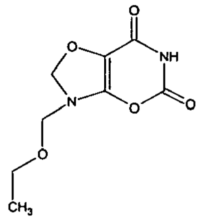 | (113) | -0,39 | -1,69 |
| 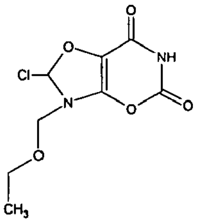 | (114) | 0,21 | -1,51 |
| 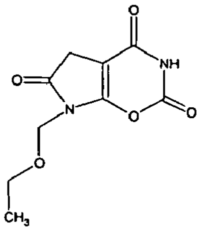 | (115) | 0,22 | -0,27 |
| 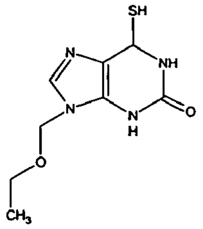 | (116) | -0,33 | -0,74 |

(continued)

| Chemical structure | Compound-# | logD (pH 4) | logD (pH 7.4) |
|---|---|---|---|
| 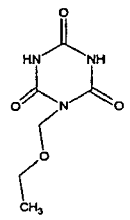 | (117) | -2,07 | -3,44 |
| 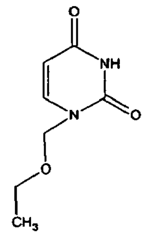 | (118) | -0,65 | -0,68 |
| 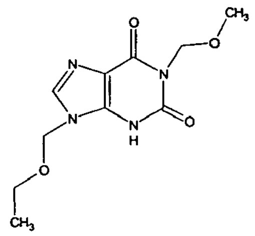 | (119) | -0,76 | -2,42 |
| 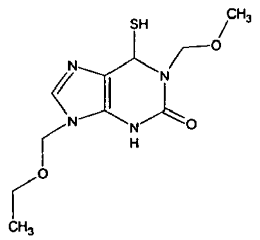 | (120) | -0,31 | -1,07 |
| 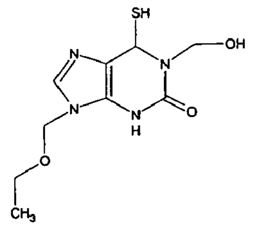 | (121) | -0,62 | -1,38 |
| 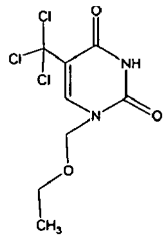 | (122) | 1,65 | 0,71 |

(continued)

| Chemical structure | Compound-# | logD (pH 4) | logD (pH 7.4) |
|---|---|---|---|
| 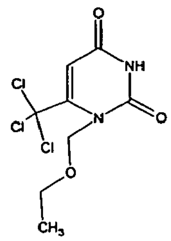 | (123) | 1,87 | 0,41 |
| 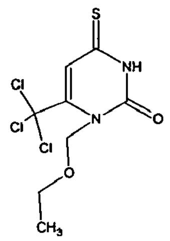 | (124) | 3,25 | 1,46 |
| 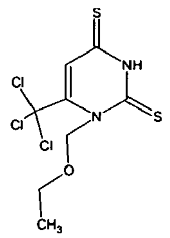 | (125) | 2,81 | 1,15 |
| 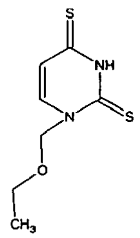 | (126) | 0,56 | -1,25 |
| 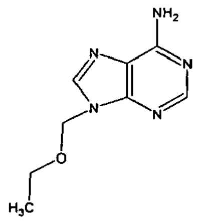 | (127) | -0,59 | -0,23 |
| 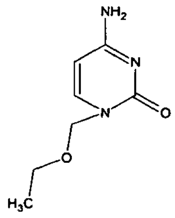 | (128) | -1,21 | -0,98 |

(continued)

| Chemical structure | Compound-# | logD (pH 4) | logD (pH 7.4) |
|---|---|---|---|
| 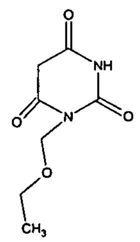 | (129) | -0,48 | -3,23 |

In another aspect of the invention, the hydrophilic elements comprise zwitterionic groups that respond to changes in the pH of the environment. Zwitterionic structures exist at pH values where both the cationic and the anionic group are charged and a generalized logD plot is shown in figure 1. It is apparent that the zwitterions have higher logD values than the charged parent groups.

The desired increase in logD upon acidification is represented by the right flank of the logD curve and depends on the pKa of the cationic charge group; it is rather independent from the pKa of the anionic group itself. As an example, the anionic group maybe a carboxyl group and the cationic group maybe a heterocyclic nitrogen atom two to five carbon atoms apart from that group (e.g. compounds 10 or 11). Pyridylcarboxylic acids, imidazolcarboxylic acids or the like are a few representations of such pH-responsive hydrophilic elements. The zwitterion exists between pH 4 and pH 7, thereby providing the pH-responsive hydrophilic headgroups of the invention. On the contrary, a simple amino group having a high pKa of about 9 (e.g. compound 13) or a quarternary ammonium group providing a constant positive charge with no effective pKa (e.g. compound 12) extends the range of pH values where the zwitterion exists and any change in hydrophilicity does no longer occur in the pH region desired (pH 2 to 9 or the more preferred ranges given above)(see figure 1 and table 2).

Table 2: Compounds 10-14

| | | |
|---|---|---|
| (10) 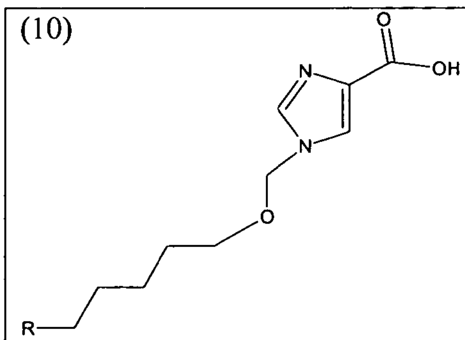 | (12) 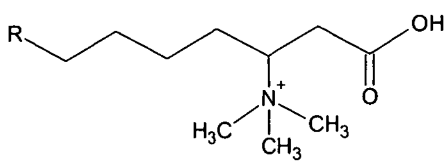 | (11) 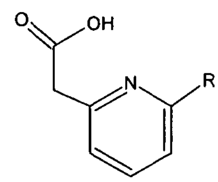 |
| (13) 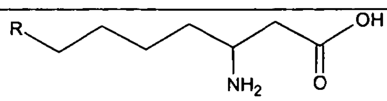 | (14) 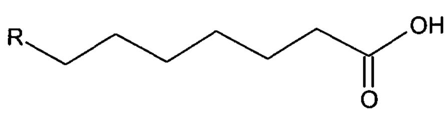 | |

The hydrophilic elements can further be substituted with polar or apolar groups. In one aspect of the invention, substitutions are selected to achieve a specific pKa of the hydrophilic element. Rules to achieve such adjustment of pKa values are known to the skilled artisan and comprise for example substitutions at nitrogen atoms of barbituric acid or xanthine with hydroxymethyl-, hydroxyethyl-, methoxymethyl-, methoxyethyl-, ethoxymethyl-, ethoxyethyl-, thiomethyl-, thioethyl-, methylthiomethyl-, methylthioethyl-, ethylthiomethyl-, ethylthioethyl-, chloro-, chloromethyl- vinyl-, phenyl- or benzyl groups or mixtures thereof to achieve a lower pK of the structure. Substitutions at the positions $R_1$, $R_2$ or $R_3$ in formula (2) or (3) are in particular suitable to achieve such shift in pK values.

Of course, pK values can be shifted towards higher values with substitutents comprising methyl-, ethyl- , propyl-, isopropyl- and tert-butyl or cyclohexyl groups or mixtures therof. An excellent overview for substituted xanthins and their

respective pK values is found in Kulikowska et al., Biochim. Acta Pol., 51, 493-531, 2004.

It is known, that the pKa value for carboxyl groups is also affected by substitutions or chemical alterations in spatial proximity. Acrylic acid derivatives, aromatic carboxylic acids such as benzoic acid, pyridinyl carboxylic acid, α- or β- hydroxycarboxylic acids or α- or β- thiocarboxylic acids but also halogenated carboxylic acids have lower pKa values than the parent compounds. In contrast, substitutions with an +I effect change the pKa of a carboxyl group towards higher values, e.g. in cyclohexylcarboxylic acids.

Specific examples of substituted hydrophilic elements include, but are not limited to formula (4) to (9) of table 3, wherein R identifies the hydrophobic element of the TEE:

Table 3: Compounds 4-9

| Substituted xanthines | | |
|---|---|---|
| 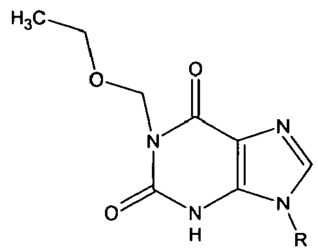 (4) | (5) 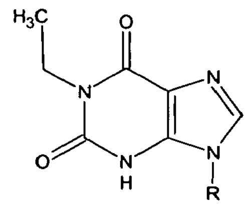 | (6) 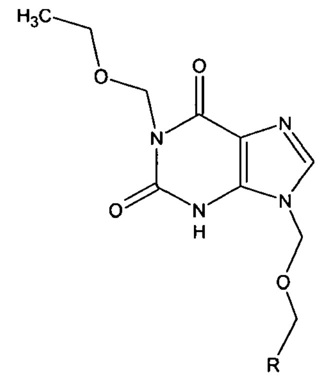 |
| **Substituted carboxylic acids** | | |
| (7) 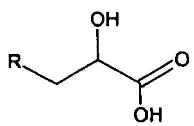 | (8) 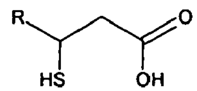 | 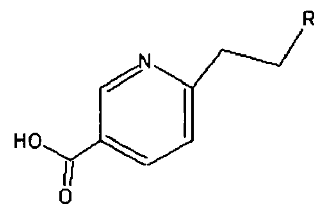 (9) |

Further chemical representations for the hydrophilic elements can be identified from the group of weak acids using the relationship between logD, pH and the pKa of the substance. For acids, this can be expressed as follows:

$$\log D = \log P + \log(1 + 10^{(pH\text{-}pKa)});$$

wherein logP is the partition coefficient for the non-ionized form. The equation reflects conditions of zero ionic strength and extremely low values for logD are calculated for acids at high pH. Under physiological conditions, where the ionic strength is about 0,15M, salt formation is limiting such extremes in logD.

Figure 2 shows the logD calculations for a number of hydrophilic elements.

Further analysis reveals identical shifts in logD when curves are plotted against pH-pKa (see figure 3).

Once standardized with respect to their pKa values the logD plots become similar for all hydrophilic elements analyzed here. A maximum difference of 3.75 units in logD can be achieved for the ionization of a single hydrophilic element. The maximum amplitude for zwitterion formation is about 2.5 units in logD.

The full amplitude requires a rather large shift of about 6 units in pH. Within the practical range of ΔpH ~ 3.4 (pH 7.4 - pH 4) considered for many aspects of this invention, the maximum difference in logD is about 3 units for pKa ~ 4. The logD reacts very sensitive whenever the pH is 0 to 4 units above the pKa, the most sensitive reaction is at pH values between 1 and 2.5 units above pKa. This relation is also analyzed in the figure 4.

In practical terms, the ideal pKa for hydrophilic elements is about 4. Preferred are hydrophilic elements having a pKa between 2 and 6 (maximum amplitude about 1.5 units), more preferred are hydrophilic elements having a pKa between 3 and 5 (maximum amplitude about 2.5 units). Other hydrophilic elements within the scope of the invention may have pKa values between 1 and 7.

Hydrophobic elements of the TEE's

TEE's of this invention comprise hydrophobic elements which contribute to the penetration of biological lipid membranes. Chemical representations of such hydrophobic elements include linear, branched or cyclic chains with a minimum chain length of 6 elements. In many aspects of the invention the chain elements are carbon atoms. In some aspects of the invention the chain elements may comprise heteroatoms being able to form covalent bonds with more than one other chain element. Hydrogen or halogen atoms can substitute the chain, but are not elements of the chain. The hydrophobic elements can comprise more than 6 elements and may comprise up to 40 elements. In some aspects of the invention the hydrophobic elements comprise between 6 and 12 elements. In other aspects of the invention the hydrophobic element comprise between 12 and 20 elements. In still other aspects of the invention the hydrophobic element does comprise between 20 and 40 elements.

In one aspect, the branching of the main chain comprises one or more rather small building blocks such as methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof.

Hydrophobic elements can be saturated or may contain unsaturated bonds.

In another aspect, more complex branched and or cyclic ring systems may be chemical representations of the hydrophobic element. In one embodiment of such aspect, hydrophobic elements are derived from sterols. Of course, the sterols may further be substituted with methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof. In another aspect of the invention, the hydrophobic elements may comprise sterols that are substituted with one or more hydrophilic groups such as hydroxyl groups. In a preferred embodiment, sterols contain hydroxyl groups at one or more of the positions 3, 7 and 12. In a preferred aspect, the sterol is a cholestane and in a further preferred embodiment the cholestane is hydroxylated in one or more of the positions 3, 7 or 12.

It is possible to insert one or more heteroatoms or chemical groups into the hydrophobic element. In one embodiment of the invention the hydrophobic element does comprise no more than 5 and in another embodiment no more than 2 heteroatoms or chemical groups.

The heteroatoms or chemical groups may be selected from the group comprising -O-, -S-, - N(H)C(O)-, -C(O)O-, -OC(O)N(H)-, -C(O)-, -C(O)-N(H)-, -N(H)-C(O)-O-, -CH=N-, -O-C(O)-, -N=CH- and/or -S-S-.

In some aspects, said heteroatoms and/or chemical groups derive from amino acids, α-hydroxyacids or β-hydroxy acids.

In one embodiment said amino acid building block is selected from the group of proline, glycine, alanine, leucine, isoleucine, valine, tyrosine, tryptophane, phenylalanine or methionine or peptides thereof and said α- and β-hydroxyacids are selected from the group comprising glycolic acid, lactic acid or hydroxybutyric acid.

In another embodiment of such an aspect, more than a single ether group is present and the spacing between

the ether bonds is two carbon atoms, representing the monomer elements in poly-ethylenglycol or poly-propylenglycol.

Architecture of TEE's

Construction of TEE's is governed by its physicochemical parameters; TEE's undergo a hydrophile-hydrophobe transition in response to an acidification of the environment. This transition is mediated by the hydrophilic elements described above that are responsive towards pH. Such response should have minimum amplitude of 0.5 logD whereby the higher absolute value of logD is achieved at the lower pH. In one aspect, such amplitude is higher than 1 logD which means that a distribution of such TEE from the water phase to the hydrophobic interior of the membrane becomes 10 times more preferred. In another aspect, such amplitude is higher than 1.5 and in some aspects the amplitude between the hydrophilic and hydrophobic form is more than 2 logD units.

In one aspect of the invention, the hydrophilic elements respond to pH values between the physiological pH and slightly acidic conditions of about pH 4. Such slightly acidic conditions can be found within cell organelles like endosomes or lysosomes. Therefore TEEs may be capable of mediating the endosomal escape of lipid aggregates or liposomes after endocytotic uptake into the cell. Tumor tissue or areas of ongoing inflammation also provide a slightly acidic environment and consequently TEEs may be useful to accumulate lipid aggregates or liposomes in these areas. Accumulation may occur specifically in tumor or stroma cells or in cells of the immune system or fibroblasts that are present in inflammatory regions.

As described above, the governing pKa for the shift in hydrophilicity can be the pKa of a weak acidic group such as carboxylic acids, barbituric acids or xanthines. In a preferred embodiment the pK of the TEE essentially driven by weak acids can be optimized to maximize the difference between hydrophilicity at pH 7.4 and hydrophobicity at pH 4. In preferred aspects, the pKa of such hydrophilic elements is between 2 and 6. In more preferred aspects this pKa is between 3 and 5.

In cases where a shift in hydrophilicity is caused by zwitterion formation, the governing pKa is the pKa of a weak base such as pyridine, imidazole, morpholine or piperazine. For zwitterions, base pKa are between 3 and 8, preferred between 4.5 and 7 and more preferred between 5 and 6.5.

TEE's may contain one or more hydrophilic elements and the relative and absolute positioning of the hydrophilic elements may vary. In some cases, neighbouring effects may occur. Effects within the hydrophilic groups include, amongst others, pK shifts and zwitterion formation. Effects between hydrophilic groups may also include shifts in the respective pK values. This is known to the skilled artisan and frequently observed between carboxylic acids in close proximity, e.g. when the spacing between groups is between 2 to 5 carbon atoms.

Some examples for more complex hydrophilic elements are shown below (table 4).

Table 4: Compounds 15 and 16

| | |
|---|---|
| (15) 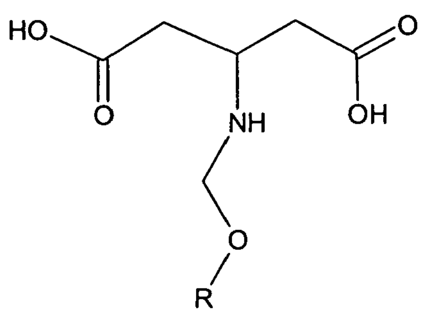 | (15) β-Glutamic acid derivatives. R represents the hydrophobic element of the invention. The ether bond between R and the hydrophilic element is optional and lowers the pK of the amino group. |

(continued)

| | |
|---|---|
| (16) 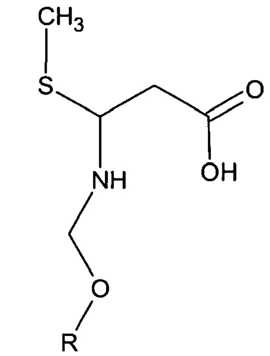 | (16) 3-Amino-3-(methylthio)propanoic acid derivatives R represents the hydrophobic element of the invention. Again, the ether bond between R and the amino group is optional but shifts the pK downwards and the same holds true for the thioether. The hydrophobic element may also be bound other positions including the methyl group at the thioether. |

TEE's with more than one hydrophilic element have larger amplitudes of hydrophilicity between their neutral and slightly acidic state. Of course, mixtures of hydrophilic elements can be combined with a single hydrophobic element. Such mixture may allow more precise adjustments in the amplitude and pH-sensitivity of the hydrophobic shift. However, too large of a number of hydrophilic elements increases the hydrophilicity of the TEE to values that can no longer be compensated with the hydrophobic shift.

Therefore, besides the amplitude of hydrophilicity between different pH values, the absolute hydrophilicity of the TEE at a first pH represents a very important aspect of the invention.

In terms of absolute hydrophilicity, the log D of the TEE itself may be vary between slightly hydrophilic at conditions of neutral or physiological pH and somewhat hydrophobic. In other words the TEE's have a logD at pH7.4 between -2 and 10.

In preferred aspects, the logD (7.4) is greater than 1 and in some aspects the logD(7.4) is greater than 3. In other aspects of the invention, the logD(7.4) of the TEE is smaller than 10, in some aspects the logD(7.4) is smaller than 7.

Independent from its absolute logD at pH7.4, the logD of the TEE at pH4.0 needs to exceed 0.

TEE's with a negative logD(7.4) require high amplitudes of the hydrophobic shift and such high amplitudes can be provided hydrophilic elements comprising one or more carboxyl groups, xanthine groups or barbituric acid groups.

Specific examples of preferred TEE's

The following chemical representations of TEE's should further illustrate the teachings of the invention. However, the scope of the invention is by no means limited to the specific examples given below. Preferred TEE's have the following attributes:

| | |
|---|---|
| Number of chain elements in the hydrophobic element | : 8...40 |
| logD(7.4) | : 1...10 |
| logD(7,4)-logD(4) pKa- | : >1 |
| for weak acids | : 2 ... 6 |
| for weak bases with ability for zwitterion formations | : 4.5 ... 7 |

A. TEE's based on carboxylic acids

In one aspect of the invention the TEE comprises one or more carboxylic acid groups as the hydrophilic element.

In some embodiments of such aspect, the hydrophobic element comprises a straight chain of carbon atoms. In some representations, such chain is a straight alkyl chain.

Table 5 below is analyzing logD at pH4 and pH7.4 for different chain length of the carboxylic acids.

Table 5:

| # of C | pH 4,0 | pH 7,4 | Δ |
|---|---|---|---|
| 4 | 0,71 | -1,83 | -2,54 |
| 6 | 1,77 | -0,75 | -2,52 |
| 8 | 2,84 | 0,31 | -2,53 |
| 10 | 3,9 | 1,38 | -2,52 |

(continued)

| # of C | pH 4,0 | pH 7,4 | Δ |
|---|---|---|---|
| 12 | 4,96 | 2,44 | -2,52 |
| 14 | 6,02 | 3,5 | -2,52 |
| 16 | 7,09 | 4,56 | -2,53 |
| 18 | 8,15 | 5,62 | -2,53 |
| 20 | 9,21 | 6,69 | -2,52 |

It becomes apparent that chain elongation by an methylene group increases the logD by about 0,5 units. Carboxylic acids with 6 to 26 C atoms represent preferred TEE's according to the selection criteria given above.

Position effects of the carboxylic acid group are less important and the carboxylic group is not mandatory the terminal group of the hydrophobic element.

In some aspects, one or more positions of the main chain of the hydrophobic element can be substituted (R-) and the impact of some substitutions is analyzed below for hexadecanoic acid (palmitic acid) derivatives (table 6).

Table 6:

| methyl side chain | | | | ethyl side chain | | | | propyl side chain | | | |
|---|---|---|---|---|---|---|---|---|---|---|---|
| # subs | pH 4,0 | pH7,4 | Δ | # subs | pH 4,0 | pH7,4 | Δ | # subs | pH 4,0 | pH 7,4 | Δ |
| 0 | 7,09 | 4,56 | -2,53 | 0 | 7,09 | 4,56 | -2,53 | 0 | 7,09 | 4,56 | -2,53 |
| 1 | 7,43 | 4,91 | -2,52 | 1 | 7,96 | 5,44 | -2,52 | 1 | 8,5 | 5,98 | -2,52 |
| 2 | 7,78 | 5,26 | -2,52 | 2 | 8,84 | 6,32 | -2,52 | 2 | 9,91 | 7,38 | -2,53 |
| 3 | 8,13 | 5,6 | -2,53 | 3 | 9,72 | 7,2 | -2,52 | 3 | 11,32 | 8,79 | -2,53 |

If R= methyl, each R results in a gain in logD of about 0.35 units. If R= ethyl, such gain is about 0.88 and for R=propyl the gain is about 1.41 per substitution. It becomes apparent that addition of methylene groups in side chain also increase logD by about 0.5 units as it is the case in the main chain.

In other aspects, R comprises heteroatoms, in particular oxygen atoms and the impact for some substitutions is analyzed below for hexadecanoic acid (palmitic acid) derivatives (table 7).

Table 7:

| methoxy side chain | | | | ethoxy side chain | | | | MOE side chain | | | |
|---|---|---|---|---|---|---|---|---|---|---|---|
| # subs | pH 4,0 | pH7,4 | Δ | # subs | pH 4,0 | pH7,4 | Δ | # subs | pH 4,0 | pH7,4 | Δ |
| 0 | 7,09 | 4,56 | -2.53 | 0 | 7,09 | 4,56 | -2,53 | 0 | 7,09 | 4,56 | -2,53 |
| 1 | 6,03 | 3,35 | -2,68 | 1 | 6,56 | 3,9 | -2.66 | 1 | 6,89 | 4,31 | -2,58 |
| 2 | 4,48 | 1,8 | -2,68 | 2 | 5,54 | 2,87 | -2,67 | 2 | 6,4 | 3,81 | -2,59 |
| 3 | 2,92 | 0,24 | -2,68 | 3 | 4,52 | 1,85 | -2,67 | 3 | 5,91 | 3,32 | -2.59 |

For R= methoxy each R results in a decrease of logD of about -1.4 units. If R= ethoxy, the extra methylene group contributes about 0,5 units of logD and the resulting effect is about - 0,9 units of logD. If R= methoxyethyl, the average impact per substitution is about -0,4 units of logD.

Other substitutions on the side chain may further change the logD of the chain with different impact and some examples for R are given in table 8 below (analysis based on hexadecanoic acid).

Table 8:

| R= | D (log(D)) | R= | D(log(D)) |
|---|---|---|---|
| Vinyl | +0.4 | Methoxymethyl- | -1 |
| Chloro | -0.2....-0.5 | Ethoxymethyl- | -0.5 |
| Fluoro | -0.9 | Ethoxyethyl- | 0 |
| Bromo | -0.35....+0.2 | Keto- | -2.2....-1.7 |

(continued)

| R= | D (log(D)) | R= | D(log(D)) |
|---|---|---|---|
| Hydroxyl | -1.2 ... 2.2 | | |

The substituents itself are not pH-responsive and therefore do not contribute to the pH-response of the TEE. Also, the impact of R is independent of the pH. However, as pointed out before R may influence the pKa of the hydrophilic element, thereby changing the amplitude of log(D) between physiological pH and pH4...5 .

In still other aspects, heteroatoms may be part of the main chain of the hydrophobic element. In other aspects, the main chain may comprise non-saturated bonds. In still other aspects, the main chain may comprise heteroatoms in combination with substitutions in the side chain. Such changes may influence the logD of the TEE and some variations for the main chain have been analyzed for hexadecanoic acid (table 9).

Table 9:

| **double bonds (-2H)** | | | | **other In main chain (PEG)** | | | | **ether In main chain (PPG)** | | | |
|---|---|---|---|---|---|---|---|---|---|---|---|
| **#subs** | **pH 4,0** | **pH7,4** | **Δ** | **#subs** | **pH 4,0** | **pH7,4** | **Δ** | **# subs** | **PH 4,0** | **pH7,4** | **Δ** |
| 0 | 7,09 | 4,56 | -2,53 | 0 | 7,09 | 4,56 | -2,53 | 0 | 7,09 | 4,56 | -2,53 |
| 1 | 7,11 | 4,59 | -2,52 | 1 | 5,16 | 2,54 | -2,62 | 1 | 5,51 | 2,88 | -2,63 |
| 2 | 6,58 | 3,99 | -2,59 | 2 | 3,21 | 0,57 | -2,64 | 2 | 3,9 | 1,26 | -2,64 |
| 3 | 5,95 | 3,09 | -2,86 | 3 | -0,69 | -3,33 | -2,64 | 3 | 2,3 | -0,34 | -2,64 |

A double bond therefore reduces logD by about -0.4 to 0.5 units, ether bonds in the main chain as found in repetitive structures derived from poly-ethylenglycol reduce logD by about 2.5 units, said effect being reduced to about 1.6 units by addition of an additional methyl group next to the ether bond, such as in the repetitive structures from polypropyleneglycol.

Other substitutions on the main chain may further change the logD of the chain with different impact and some examples are given in table 10 below (analysis based on hexadecanoic acid).

Table 10:

| element | D (log(D)) | element | D(log(D)) |
|---|---|---|---|
| $-CH_2-$ | Reference | -S- | -1,5 |
| -NH- (Aza) | -6....-9 | -S-S- | -1.1 |
| -NH-CO- (Amide) | -7.5 ... -11 | | |
| -O-CO- (Ester) | -2,7 | | |

The substituents itself are not pH-responsive and therefore do not contribute to the pH-response of the TEE. Also, the impact of R is independent of the pH. However, as pointed out before R may influence the pKa of the hydrophilic element, thereby changing the amplitude of log(D) between physiological pH and pH4...5 .

The examples and analysis presented above give guidance for practicing the invention, in particular to identify structural elements as TEE's. Isoforms and position isomers are within the disclosure of the current invention. As mentioned earlier in this disclosure, neighbouring effects between substituents may occur. However, these effects are known to the skilled artisan and are described in the standard chemical literature, in databases or are part of chemical software such as ACD/Labs and others.

Ring systems

In some aspects, the hydrophobic element may form a cyclic structure such as cycloalkanes, cycloalkenes or cycloalkynes or aromatic ring structures.

A few representations of cyclic elements have been analyzed in the table below and more cyclic elements can be developed from known structural contributions of other elements. It is of course possible to further substitute the cyclic elements, in particular with the groups analyzed above.

Table 11:

| | Cyclic backbone | | | |
|---|---|---|---|---|
| | cpd | pH4,0 | pH7,4 | Δ |
| stearic acid | | 8,15 | 5,62 | -2,53 |
| 4-undecyl cyclohexanoic acid | | 7,54 | 5,22 | -2,32 |
| 4-undecyl cyclohexenoic acid | | 7,12 | 4,39 | -2,73 |
| p-undecyl benzoic acid | | 7,51 | 4,87 | -2,64 |

Sterols

In a specific variant of the invention, the ring systems of the hydrophobic elements may be more complex ring systems such as in sterols. Again, further substitutions may be present at the sterol backbone and the analysis shown below is detailing some aspects of logD for natural occurring derivatives of sterols, e.g. hydroxyl group substitutions at positions 3,7 and 12 of the sterane backbone.

Also, the orientation of the sterol in the TEE may be different and a presence of the carboxyl group at position 26 such as in bile acids or grafted onto position 3 such as in cholesterol hemisuccinate (CHEMS) represent some instant examples (tables 12 and 13).

Table 12: Analysis of logD for bile acid derivatives used as TEE's. For analytical purposes, the 3' hydroxyl group was assumed to be methylated to model a potential drug linkage

| bile acids | Oil in backbone | | | |
|---|---|---|---|---|
| (3 cholesterols) | # subs | pH 4,0 | pH7,4 | Δ |
| 3' methoxy cholate | 3 | 3,18 | 0,64 | -2,54 |
| 3' methoxy deoxycholate | 2 | 5,23 | 2,69 | -2,54 |
| 3' methoxy dideoxycholate | 1 | 7,27 | 4,73 | -2,54 |

Table 13: Analysis of logD for CHEMS derivatives used for TEE's. For analytical purposes, the carboxyl group in position 26 was esterified with methanol to model a potential drug linkage. In this analysis, the 3' position is esterified with succinic acid, thereby providing an unsubstituted carboxyl group as the hydrophilic element of the TEE.

| CHEMS derivatives | OH in backbone | | | |
|---|---|---|---|---|
| (26 cholesterols) | # subs | pH 4,0 | pH7,4 | Δ |
| 26'methyl cholate | 3 | 3,65 | 1,01 | -2,64 |
| 26' methyl deoxycholate | 2 | 5,69 | 3,06 | -2,63 |
| 26' methyl dideoxycholate | 1 | 7,73 | 5,1 | -2,63 |

B. TEE's based on barbituric acids and xanthines

The considerations above can of course be transferred to TEE's comprising structurally different hydrophilic elements. Contributions of structural elements in the hydrophobic section of the TEE are close if not identical irrespective of the chemical nature of the hydrophilic element or elements. Some specific chemical representations of TEE's comprising barbituric acid or xanthine may illustrate the construction of such TEE's without limiting this part of the disclosure (compounds 17-23).

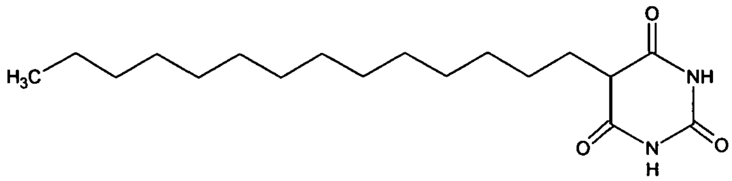

(17)

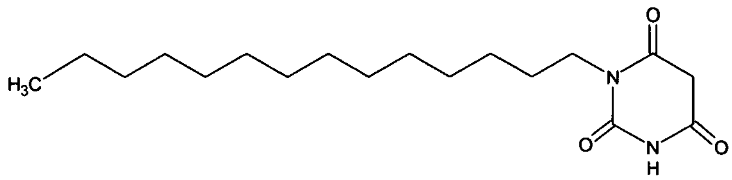

(18)

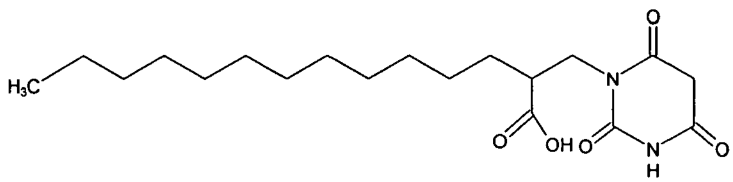

(19)

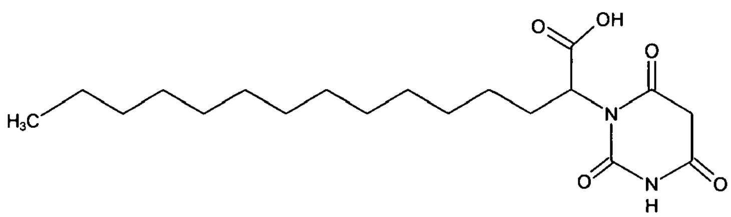

(20)

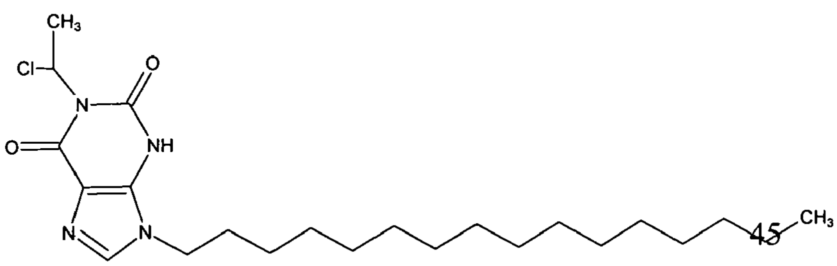

(21)

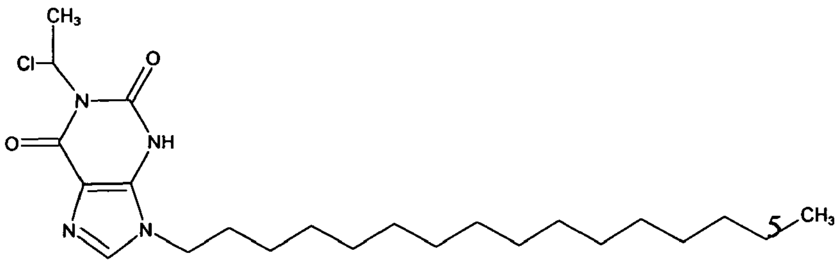

(22)

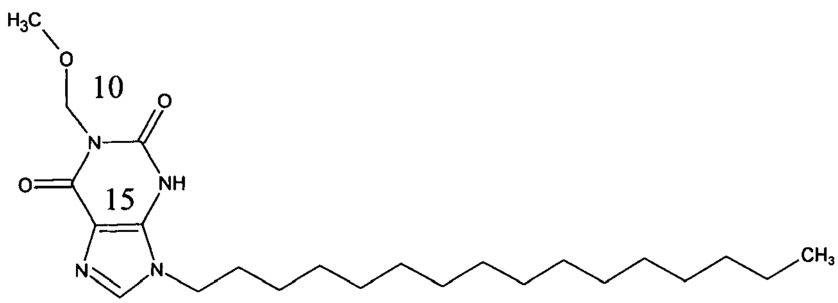

(23)

The position of the hydrophilic element within the TEE structure may vary. In some aspects, the hydrophilic element is located distal from the link between molecule and TEE. In other aspects, the hydrophilic element is located central within the TEE.

<u>Use of TEE's</u>

TEE's need to be combined to a lipid or a liposomes or other supramolecular aggregates such as micelles, lipoplexes, cochleates, emulsion droplets and the like, altogether called lipid assemblies within this invention.

For the combination of TEE's with lipid assemblies a number of different approaches can be taken including but not limited to chemical bonds, physical attractions or by other interactions, e.g. chelate bonds.

In one aspect of the invention, the TEE's can directly be linked to a lipid by means of chemical bonds; lipids comprising TEE's are described in detail further below.
In another aspect of the invention, TEE's can be grafted on the lipid assembly after such assembly has been produced. Chemical conjugation techniques and methods for carrying out such couplings have extensively been reported in the literature, e.g. by G. T. Hermanson, Bioconjugate Techniques, Academic Press, 1996.

In another aspect of the invention, TEE's may be complexed with the lipid assembly systems using molecular recognition, e.g. between biotin and biotin binding proteins, between two complementary or partially complementary oligonucleotides or between cyclodextrins and molecules fitting the binding pockets of the cyclodextrin such as various lipids or detergents as disclosed in DeGrip et al., Biochem. J., 330, 667-674, 1998, sterols or adamantane units as disclosed in WO 06/105361, other sterols such as steroids or adamantane units as disclosed in WO 06/105361.

In another aspect, TEE's may be complexed with lipid assemblies using ionic or electrostatic interaction. In one embodiment of such aspect, TEE's further comprising a polycationic element are combined with anionic lipid assemblies. In another embodiment, TEE's further comprising a polyanionic element are combined with cationic lipid assemblies. Examples for polycationic elements include, but are not limited to polyethylenimine, spermine, thermine, spermidine, putrescine or polymers or oligomers from lysine, ornithine or arginine and the like. Examples for polyanionic elements include, but are not limited to polymers or oligomers of acrylic acid, methacrylic acid, glutamic acid, aspartic acid and the like. Techniques and experimental protocols for the combination of lipid assemblies with polyions are disclosed in WO 01/64330.

TEE derivatives for such use include, but are not limited to the compounds 24 - 29.

Table 14: Compounds 24-29

| | |
|---|---|
| 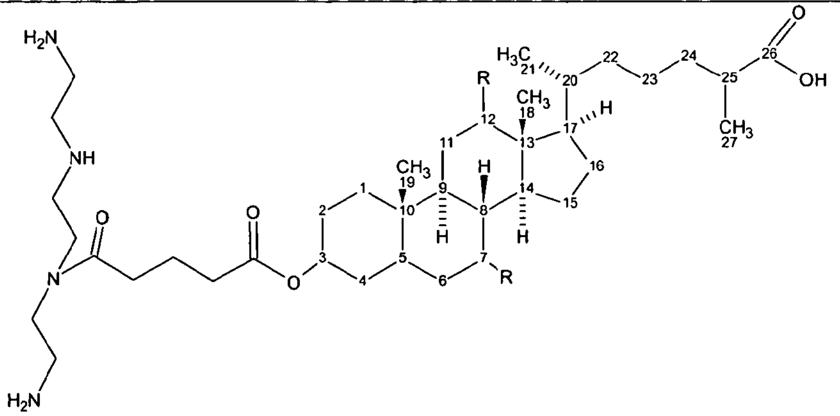<br>(24), R is -H or -OH | |

| | |
|---|---|
| 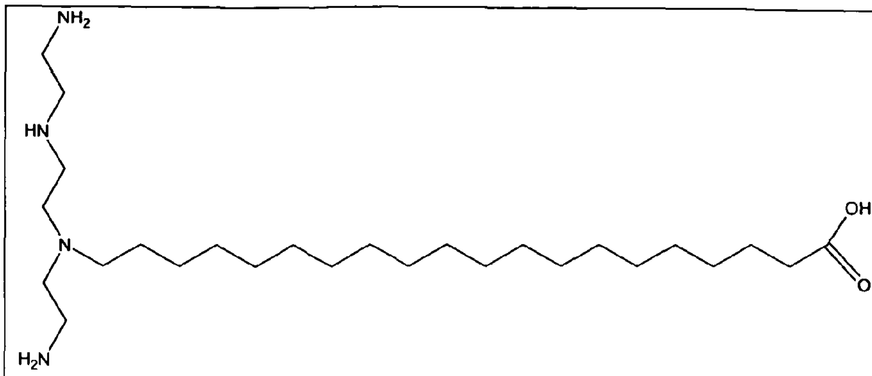 (25) | |
| 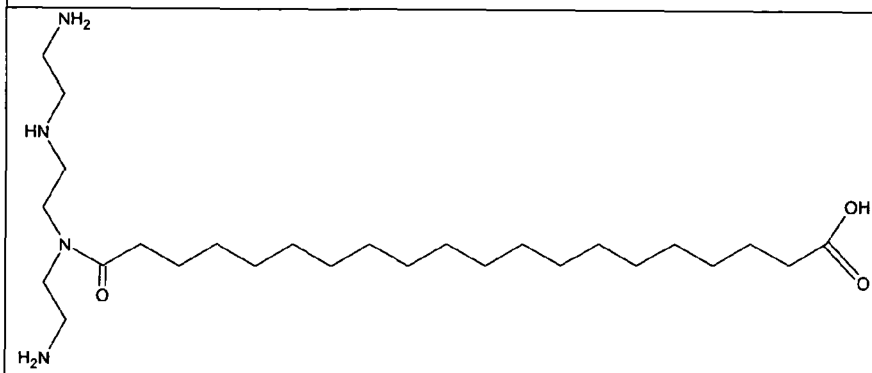 (26) | |
| 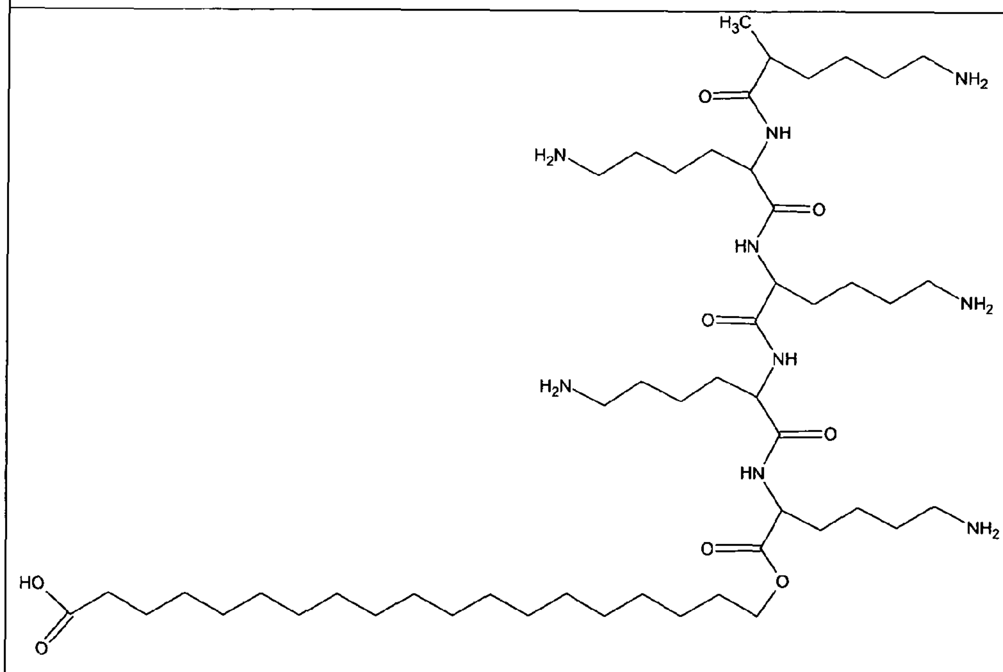 (27) | |
| | |

| | |
|---|---|
| 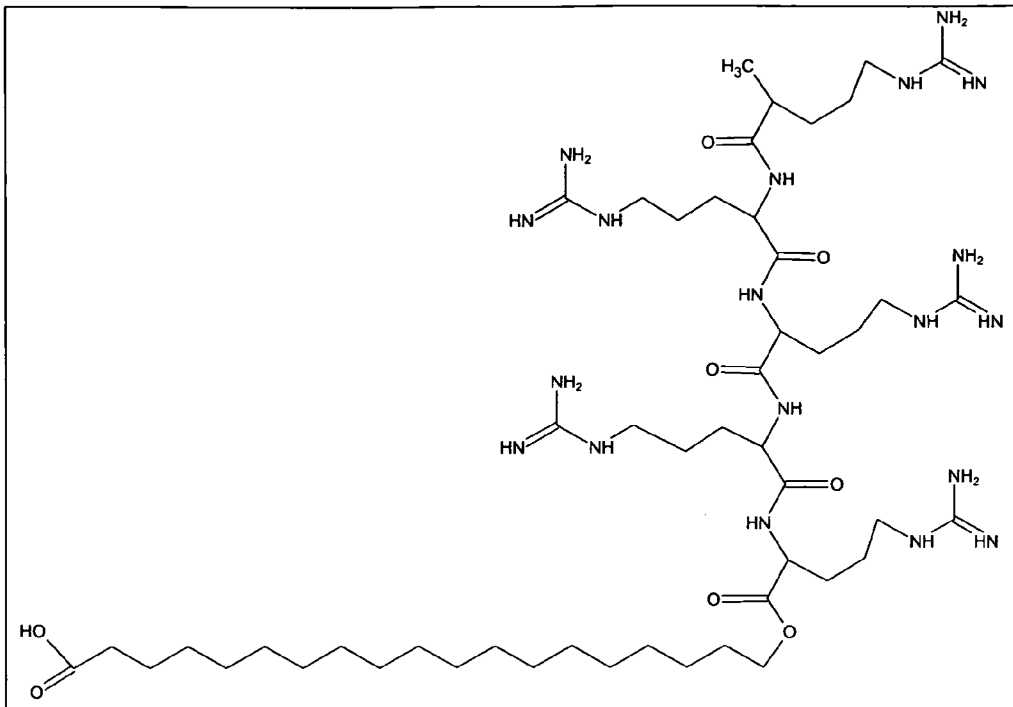 (28) | |
| 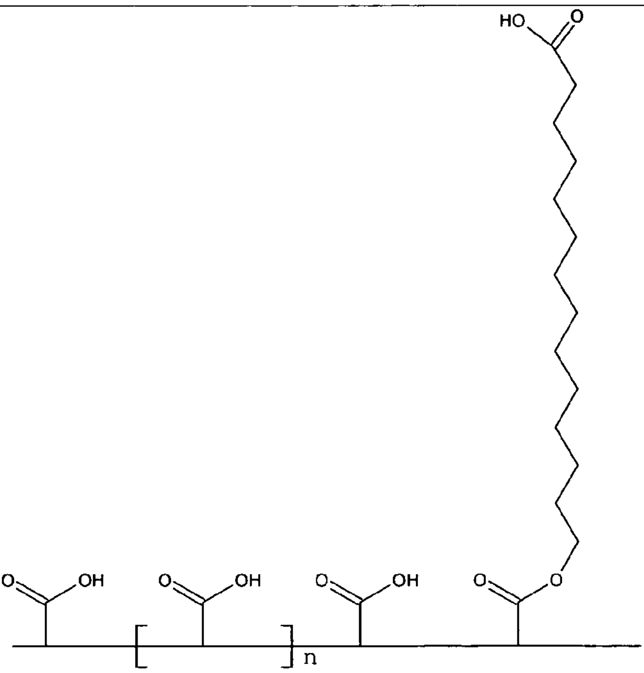 (29) n = 1 - 50 | |

Lipids comprising TEE's

Lipids to which the TEE may be attached according to the present invention may be selected without limitation from the group comprising phospholipids and their lyso forms, sphingolipids and their lyso forms, sterols like cholesterols

and derivatives thereof, diacylglycerols/ dialkylglycerols, monacylglycerols/ monoalkylglycerols, monoester, diesters, monoethers or diethers of glyceric acid, sphingosines/ phytosphingosines/ sphinganines and N-substituted derivatives thereof, ceramides, 1,2-Diacyl-3-aminopropanes/ 1,2 -Dialkyl-3-aminopropanes, 1- or 2- monoacyl - 3 aminopropanes/ 1-or 2- monoalkyl-3-aminopropanes, dialkylamines, monoalkylamines, fatty acids, dicarboxylic acid alkyl ester, tricarboxylic acid dialkyl ester optionally substituted with - OH groups or esters of tartaric acid with long chain alcohols or esters of tartaric acid with long chain carboxylic acids

In a preferred embodiment the acyl- and alkyl-chains of the amphiphilic lipid substances comprise independently 8-30 carbon atoms and 0, 1 or 2 ethylenically unsaturated bonds.

In other preferred embodiments the amphiphilic lipid substances comprise cholestane derivatives.

Graft positions

The TEE's of the invention can be chemically linked or grafted onto a lipid. In many aspects, the water exposed, polar lipid head group is the preferred position for grafting one or more TEE's.

Chemical linkers between the graft and the TEE may include and comprise but are not limited to -O-, -S-, -N(H)C(O)-, -C(O)O-, -OC(O)N(H)-, -C(O)-, -C(O)-N(H)-, -N(H)-C(O)-O-, -CH=N-, -O-C(O)-, -N=CH-, -S-S-, non-branched, branched or cyclic alkyl, alkylene or alkynyl with 1 to 6 C-atoms and optionally substituted with one or more -OH, $-NR_2$, -COOH or sugars or mixtures thereof; $-PO_4^-$-; $-PO_3^-$-; pyrophosphate; $-SO_4$-; $-SO_3$-; -NH-; -NR-; sugars and derivatives thereof; amino acids; Di- or Tripeptides, $\alpha$-hydroxyacids or $\beta$-hydroxy acids or dihydroxyacids.

In preferred embodiments of this invention the structural unit of the lipid head group and the chemical linker group maintains substantial polarity. In a number of aspects this structural unit is carrying a charge. This might be a preserved charge from the former head group or a newly generated charge, e.g. an amine group resulting from reductive amination.

In other aspects, the polar region of (I) is defined as the (continuous) structural unit outside the membrane anchors of the lipid and also outside of the actual TEE and said structural unit maintains substantial polarity. Substantial polarity describes the ability to form hydrogen bonds in water. Substantial polarity means a logD < 0, in some aspects logD <-2 and in other aspects a logD<-4.

In some embodiments of the invention the TEE is grafted onto a lipid comprising a charged polar head group, wherein the graft position is said charged polar head group and keeps the charge of that group at least to a substantial extent. Examples for such hydrophilic polar head groups of lipids comprise, but are not limited to phosphoric acid (in lipids like DOPA), phosphoethanol (in lipids like DOPE), phosphoglycerol (in lipids like POPG), phosphoglycerolaldehyde, phosphoglyceric acid, Amino groups (e.g. derived from 1,2-diacyl or 1,2 dialkyl - 3 aminopropanes).

Chemical representations of such lipids comprising TEE's include without limitation compounds 30-43. $R_1$ and $R_2$ independently are $C_8$-$C_{30}$ alkyl or acyl chains with 0, 1 or 2 ethylenically unsaturated bonds.

Table 15: Compounds 30-43

| | |
|---|---|
| (30) 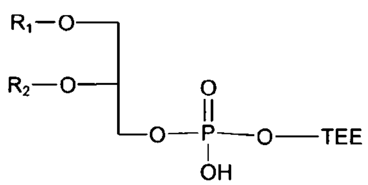 | (31) 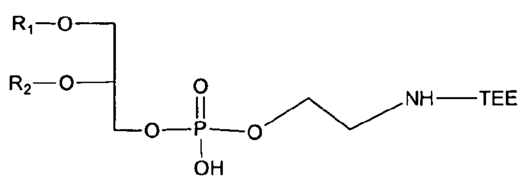 |

| | |
|---|---|
| (32) 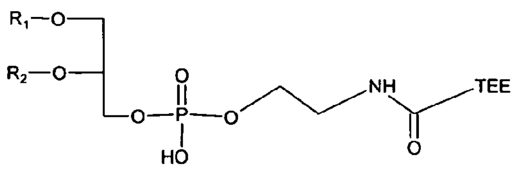 | (33) 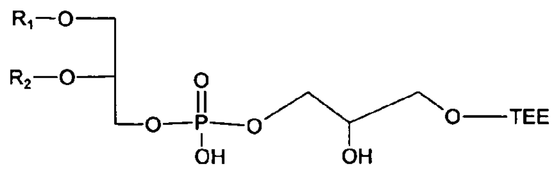 |
| (34) 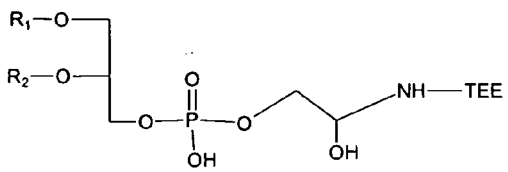 | (35) 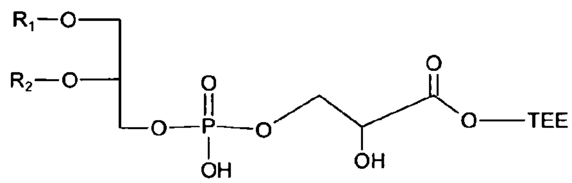 |
| (36) 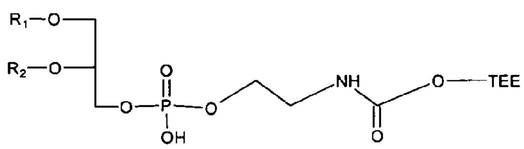 | (37) 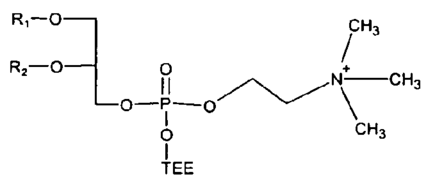 |
| (38) 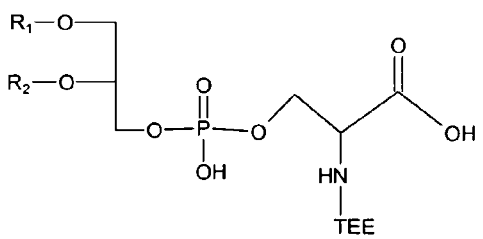 | (39) 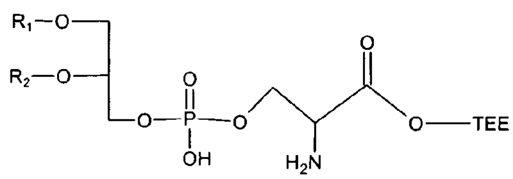 |
| (40) 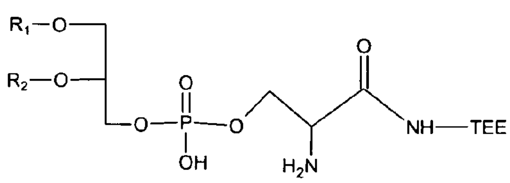 | (41) 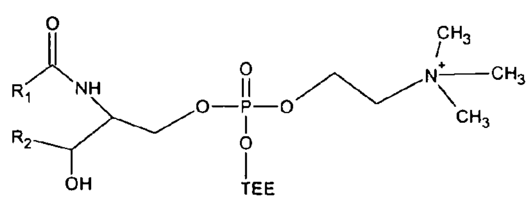 |
| (42) 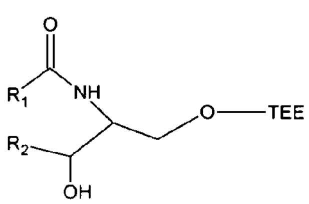 | (43) 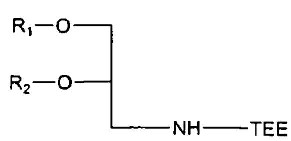 |

In another embodiment of the invention the TEE is grafted on a lipid comprising a hydrophilic polar head group, wherein the graft position is said hydrophilic group but the chemical linkage between TEE and lipid renders the polar head group uncharged. Examples include but are not limited to -N(H)-C(O)-, -N(H)-C(O)O-.

Chemical representations of such lipids comprising TEE's include without limitation compounds 44-47. $R_1$ and $R_2$ independently are $C_8$-$C_{30}$ alkyl or acyl chains with 0, 1 or 2 ethylenically unsaturated bonds.

Table 16:

| | |
|---|---|
| (44) 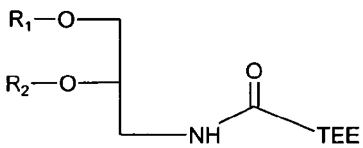 | (45) 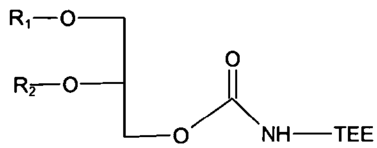 |
| (46) 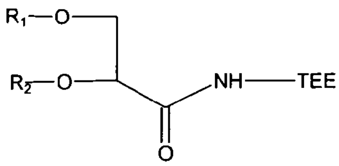 | |
| (47) 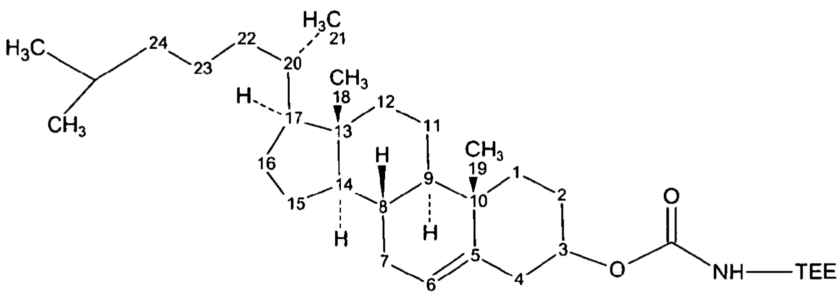 | |

Of course, in all cases substituents may be attached to the lipid headgroups. Furthermore, as mentioned above, linking groups may be inserted between the lipid headgroup and the attached TEE. In some aspects the linking groups are substituted as well.

In a preferred embodiment substituents or linking groups may be used when the attachment of a TEE to a lipid headgroup leads to a decrease of the polarity of the headgroup and subsequent to a loss of the amphiphilic character of the lipid. For example, the esterification of cholesterol with a $C_{16}$-dicarboxylic acid as TEE results in an apolar ester bond and is therefore not preferred. However, the insertion of a polar linking group, e.g. tartaric acid, between the cholesterol and the $C_{16}$-dicarboxylic acid as TEE preserves the amphiphilic structure of the lipid, also after the attachment of the TEE.

In some embodiments the substituents that may be attached to the lipid headgroups are polar and include, but are not limited to groups like -OH, -COOH, $-NH_2$, -NHR, $-NR_2$, sugars and derivatives thereof, amino acids and derivatives thereof, $-OPO_3^{2-}$, $OPO_2^{2-}$, - $OSO_3^-$; $-OSO_2^-$ or mixtures thereof.

Chemical representations of such lipids having linking groups between the headgroup and the TEE include without limitation compounds 48-49. $R_1$ and $R_2$ independently are $C_8$-$C_{30}$ alkyl or acyl chains with 0, 1 or 2 ethylenically unsaturated bonds

Table 17: Compounds 48 and 49

| |
|---|
| (48) 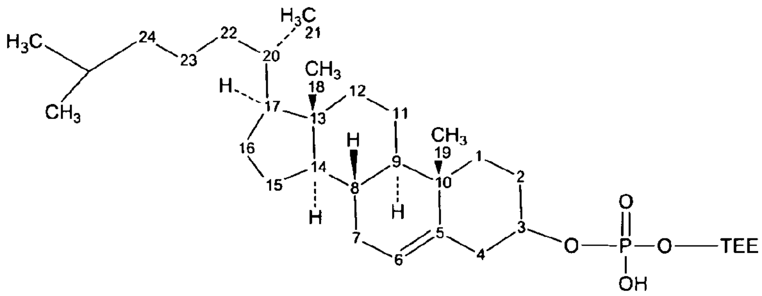 |
| (49) 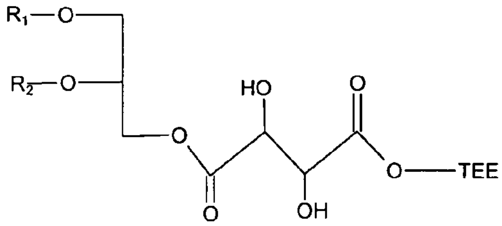 |

In some variants of the invention, the lipid may contain more than one hydrophilic polar head group or complex hydrophilic head groups that allow substitution on various positions without affecting hydrophilicity. Examples include, but are not limited to esters of tartaric acid with long chain alcohols, derivatives of maleic acid or esters of fatty acids with sugars such as glucose, sucrose or maltose. Further examples include, without limitation, alkyl glycosides and derivatives thereof, such as dodecyl-β-D-glucopyranoside or dodecyl-β-D-maltoside, derivatives of alkyl-ethylenglycol detergents (such as Brij35, Genapol series, Thesit and the like) or Phosphatidylinositols and derivatives thereof. Isomers wherein the TEE is grafted onto different positions of such polar head groups are within the scope of this invention.

Chemical representations of such lipids with more than one hydrophilic headgroups or complex headgroups comprising a TEE include without limitation compounds 50-55. $R_1$ and $R_2$ independently are $C_8$-$C_{30}$ alkyl or acyl chains with 0, 1 or 2 ethylenically unsaturated bonds. R are $C_8$-$C_{30}$ alkyl chains with 0, 1 or 2 ethylenically unsaturated bonds.

Table 18: Compounds 50-55

| | |
|---|---|
| (50) 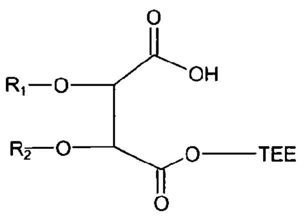 | (51) 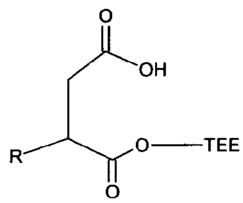 |
| (52) 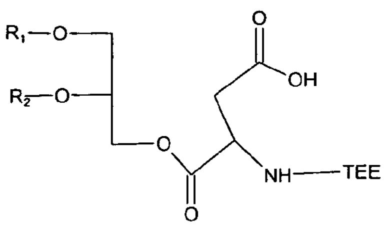 | (53) 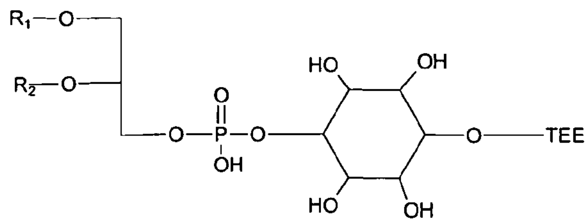 |
| (54) $H_3C—(CH_2)x—O—(CH_2CH_2O)y—TEE$ <br> x=8-30, optionally comprising unsaturated bonds and y=2-30 | |

(55)

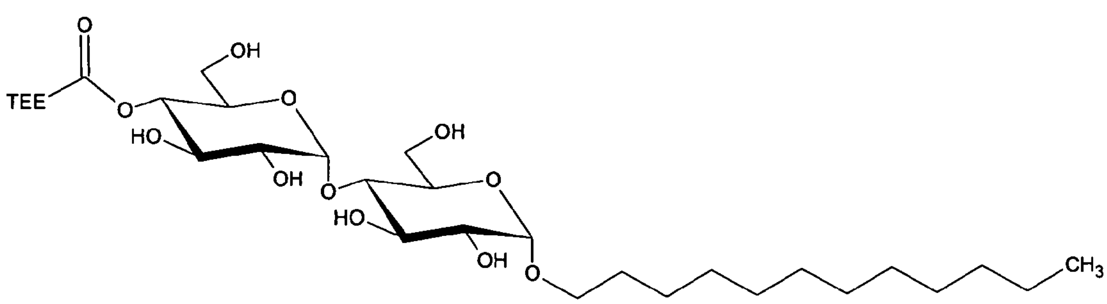

In some variants of the invention, more than a single TEE may be attached to one lipid.

Chemical representations of such lipids comprising more than one TEE include without limitation compounds 52-56. $R_1$ and $R_2$ independently are $C_8$-$C_{30}$ alkyl or acyl chains with 0, 1 or 2 ethylenically unsaturated bonds.

Table 19: Compounds 56-60

| | |
|---|---|
| (56) 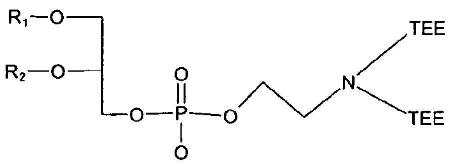 | (57) 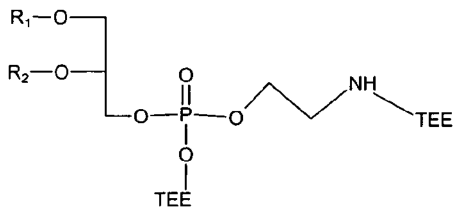 |
| (58) 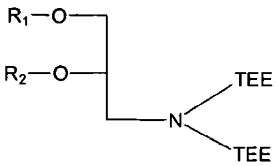 | (59) 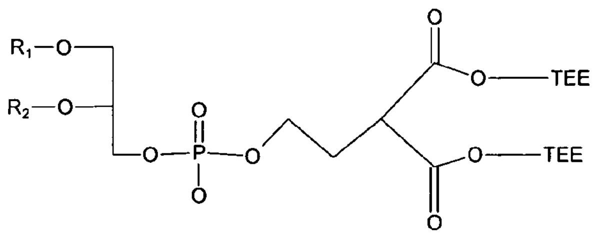 |
| | |

| | |
|---|---|
| (60) 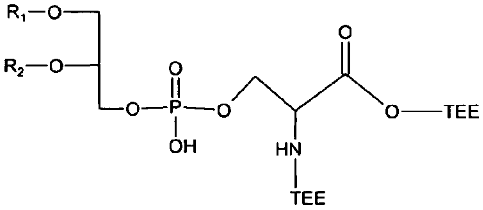 | |

In some aspects of the invention the lipids-TEE (structures according to the general formula (I)) may be other than one of the following structures (III):

PE-amid linkage-X-COOH (III)

wherein X is a carbon containing linear chain having a chain length of between 3 to 20 atoms and having various degrees of saturation and/or heteroatom compositions and/or substituents and the COOH-group

(IV)

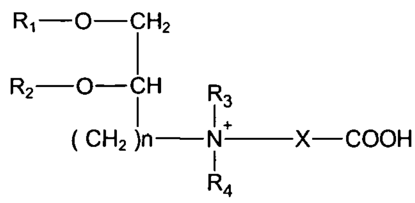

(IV)

wherein X is a straight saturated alkyl chain having a chain length of between 2 and 10 C-atoms and $R_1$, $R_2$, $R_3$ and $R_4$ are independently linear or branched, unsubstituted or substituted $C_{1-23}$ alkyl, acyl, alkylene, heteroalkyl groups having 0 to 6 sites of unsaturation, cyclic and aryl groups, the groups comprising from 0 to 5 heteroatoms, in which the substituent groups are $-O-(CH_2)_x-CH_3$; $-S-(CH_2)_x-CH_3$; $X-(CH_2)_k$, wherein X is a halide, and $-N((CH_2)_k-CH_3)_2$, wherein the alkyl groups of the substituents comprise from 0-2 heteroatoms, and k is 0-4 and wherein $R_1$ and $R_2$ can further be independently H and n is 1 to 6
or (V)

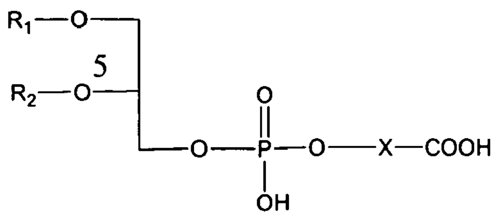

(V)

wherein $R_1$ and $R_2$ independently are hydrogen atoms or $C_1$-$C_{24}$ straight chain or branched alkyl or acyl chains optionally containing double and triple bonds and wherein X is an aliphatic and/or cycloaliphatic hydrocarbon chain with 6-20 carbon-atoms optionally substituted by aryl rests, cycloalkyls with 3-6 carbon atoms, hydroxyl and/or further carboxylic functions.

In other aspects of the invention the use of lipid assemblies comprising the compounds (III), (IV) or (V) for the in vivo, in vitro or ex-vivo transfection of cells may be preferred.

Specific examples of phospholipids in accordance with the present invention include, but are not limited to:

<u>Phospholipids and Sphingolipids:</u>

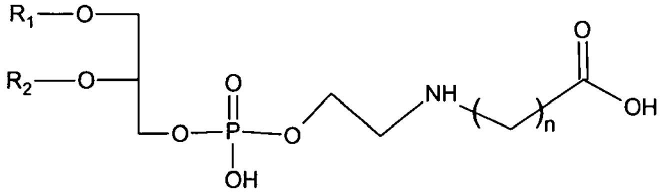

(61)

wherein $R_1$ and $R_2$ independently are $C_8$-$C_{30}$ alkyl or acyl chains with 0, 1 or 2 ethylenically unsaturated bonds and n=6-40.

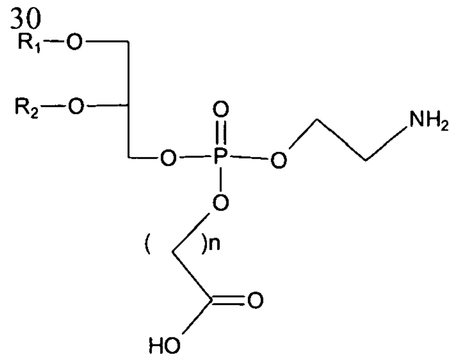

(62)

wherein $R_1$ and $R_2$ independently are $C_8$-$C_{30}$ alkyl or acyl chains with 0, 1 or 2 ethylenically unsaturated bonds and n=6-40.

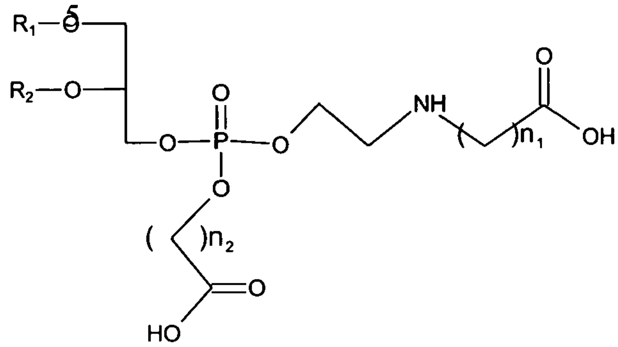

(63)

wherein $R_1$ and $R_2$ independently are $C_8$-$C_{30}$ alkyl or acyl chains with 0, 1 or 2 ethylenically unsaturated bonds and n1 and n2 are independently 6-40.

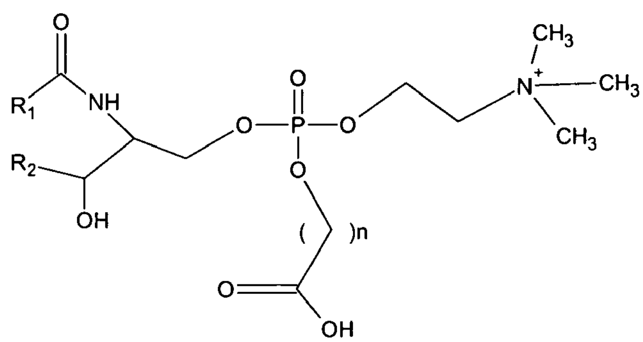

(64)

wherein $R_1$ and $R_2$ independently are $C_8$-$C_{30}$ alkyl chains with 0, 1 or 2 ethylenically unsaturated bonds and n=6-40.

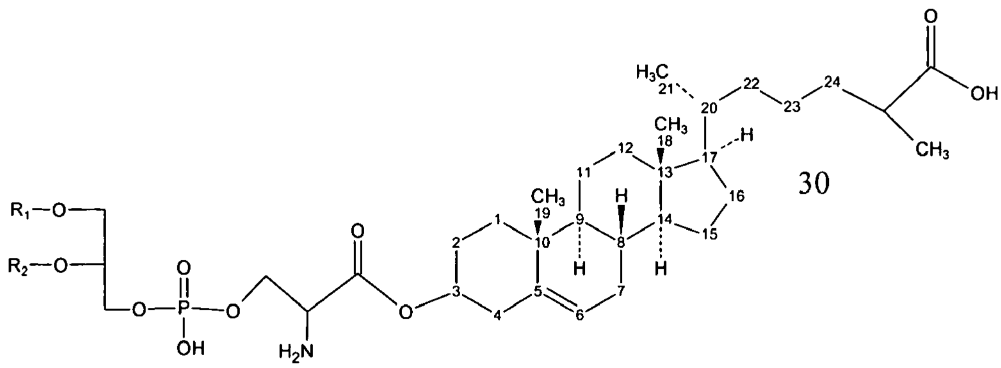

(65)

wherein $R_1$ and $R_2$ independently are $C_8$-$C_{30}$ alkyl or acyl chains with 0, 1 or 2 ethylenically unsaturated bonds.

Specific examples of sterol based lipids in accordance with the present invention include, but are not limited to:

Sterol based lipids:

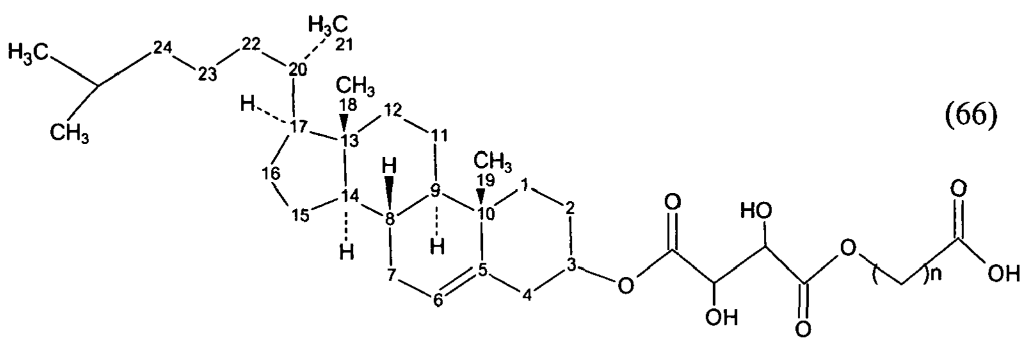

(66)

wherein n=6-40

Specific examples of diacylglycerol/ dialkylglycerols based lipids in accordance with the present invention include, but are not limited to:

Diacylglycerol/ dialkylglycerol based lipids:

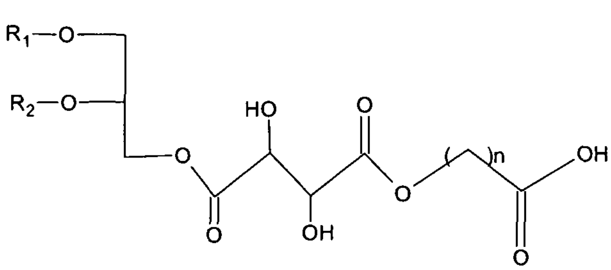

(67)

wherein $R_1$ and $R_2$ independently are $C_8$-$C_{30}$ alkyl or acyl chains with 0, 1 or 2 ethylenically unsaturated bonds and n=6-40.

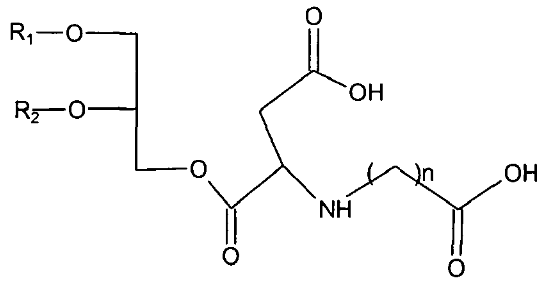

(68)

wherein $R_1$ and $R_2$ independently are $C_8$-$C_{30}$ alkyl or acyl chains with 0, 1 or 2 ethylenically unsaturated bonds and n=6-40.

Specific examples of 1,2-Diacyl-3-aminopropanes/ 1,2 -Dialkyl-3-aminopropanes based lipids in accordance with the present invention include, but are not limited to:

1,2-Diacyl-3-aminopropanes/ 1,2 -Dialkyl-3-aminopropanes:

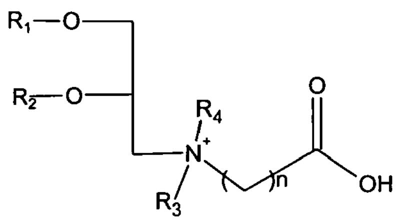

(69)

wherein $R_1$ and $R_2$ independently are $C_8$-$C_{30}$ alkyl or acyl chains with 0, 1 or 2 ethylenically unsaturated bonds, $R_3$ and $R_4$ are independently H or $C_1$-$C_6$ alkyls and n=11-40.

Methods of synthesising lipids comprising one or more transfection enhancer element as described above include coupling reactions that are well-known to those skilled in the art and may vary depending on the starting material and coupling component employed. Typical reactions are esterification, amidation, etherification, or reductive amination.

Particularly preferred molecules may be prepared by following processes, without being limited on these coupling reactions:

i) reductive amination of a ω-Carboxy-ketone with phosphatidylethanolamine

ii) esterification of the phosphate group in a phospholipid, e.g. PC, PE, PS

iii) esterification of a ω-Dicarboxylic acid with free hydroxyl groups of a lipid

iv) esterification of phosphatidylserine with the 3' hydroxyl group of a cholesterol derivative which has a carboxyl group within the terminal isopropyl group

v) amidation or alkylation of 3-amino-1,2-propanediol diesters,

vi) oxidation of phosphatidyl glycerols and subsequent reductive amination

Lipid assemblies

Lipid assemblies are supramolecular assemblies comprising amphipathic molecules. In some aspects the amphipathic substances are known as lipids or as detergents, in other aspects such substances are known to form biological membranes or to insert into biological membranes. The supramolecular assemblies may further comprise oils from apolar molecules. The supramolecular assemblies of the current invention therefore comprise liposomes of various

size and lamellarity, micelles, inverted micelles, cubic or hexagonal lipid phases, cochleates, emulsions, double emulsions or other multimeric assemblies that are substantially build from lipids, oils or amphiphiles.

In some embodiments of this invention the lipid assemblies comprise one or more lipids with one or more transfection enhancer elements may be formed from a lipid phase further comprising neutral and/or cationic and/or anionic lipids. The overall charge of the lipid assemblies can be neutral, cationic or anionic.

Lipids, that may be used to form lipid assemblies according to the present invention may include without limitation lipids listed in table 20 and 21 below. All used abbreviations for lipids refer primarily to standard use in the literature and are included in table 20 as a helpful reference.

In one embodiment of the invention the lipid assemblies include one or more neutral lipids selected from the group comprising natural or synthetic phosphatidylcholines, phosphatidylethanolamines, sphingolipids, ceramides, cerebrosides, sterol-based lipids, e.g. cholesterol, and derivatives of such lipids. Specific examples of neutral lipids include, without limited to DMPC, DPPC, DSPC, POPC, DOPC, DMPE, DPPE, DSPE, POPE, DOPE, Diphythanoyl-PE, sphingomyelein, ceramide and cholesterol.

In another embodiment the lipid assemblies include one or more cationic lipids, alone or in combination with neutral and/or anionic lipids. The cationic lipids may include without limitation DOTAP, DMTAP, DPTAP, DC-Chol, DAC-Chol, DODAP, DOEPC, TC-Chol, DOTMA, DORIE, DDAP, CTAB, CPyC, DPIM, CHIM, MoChol, HisChol, BGSC, BGTC, DOSPER, DOSC, DOGSDO and derivatives thereof.

In still another embodiment of the present invention the lipid assemblies include one or more anionic lipids, alone or in combination with neutral and/or cationic lipids. The anionic lipids may include, without limitation, phosphatidylglycerols, phosphatidylserins, phosphatidylinositols, phosphatidic acids, CHEMS and further anionic sterol-derivatives, cetylphosphate, diacylglycerol hemisuccinates and cardiolipines and derivatives thereof. Specific examples include without limited to DMPG, DPPG, DSPG, DOPG, POPG, DMPS, DPPS, DOPS, POPS, DMPA, DPPA, DOPA, POPA, CHEMS, cetylphosphate, DMG-Succ, DPG-Succ, DSG-Succ, DOG-Succ, POG-Succ, Chol-Sulfate, Chol-Phospate.

In one aspect, lipid assemblies for use with the present invention may include fusogenic lipids, such as for example DOPE, lysolipids or free fatty acids or mixtures of said fusogenic lipids with neutral and/or cationic and/or anionic lipids mentioned above.

Of course, lipid assemblies known in the art can be used with lipids comprising TEE's of the current invention. Some of such lipid assemblies are disclosed for example in WO OS/105152; WO 06/069782; Morrissey et al., Nature Biotechnology, 23(8), 1002-1007, 2005; WO OS/007196; Wheeler et al., Gene Therapy, 6(2), 271-281, 1999; WO 02/34236; Budker et al., Nature Biotechnology, 14(6), 760-764, 1996; US 5,965,434; US 5,635,487; Spagnou et al., Biochemistry, 43(42), 13348-13356, 2004; US 6,756,054; WO 06/016097 and US 5,785,992; WO 04/035523.

Alternatively, the lipid assemblies comprising one or more lipids with one or more transfection enhancer elements may be formed from a lipid phase having an amphoteric character. In one aspect said lipid assemblies are amphoteric liposomes. Amphoteric liposomes represent a recently described class of liposomes having anionic or neutral charge at about pH 7.5 and cationic charge at pH 4. WO 02/066490, WO 02/066120 and WO 03/070220 give a detailed description of amphoteric liposomes and suitable lipids therefor.

By "amphoteric" herein is meant a substance, a mixture of substances or a supra-molecular complex (e.g., a liposome) comprising charged groups of both anionic and cationic character wherein:

(i) at least one of the charged groups has a pK between 4 and 8,

(ii) the cationic charge prevails at pH 4, and

(iii) the anionic charge prevails at pH 8,

resulting in an isoelectric point of neutral net charge between pH 4 and pH 8. Amphoteric character is by this definition different from zwitterionic character, as zwitterions do not have a pK in the range mentioned above. In consequence, zwitterions are essentially neutrally charged over a range of pH values; phosphatidylcholines and phosphatidylethanolamines are neutral lipids with zwitterionic character.

In some embodiments of the present invention, said amphoteric liposomes may be formed from a lipid phase comprising one or more amphoteric lipids.

Suitable amphoteric lipids are disclosed in WO 02/066489 and WO 03/070735. Preferably, said amphoteric lipid is selected from the group consisting of HistChol, HistDG, isoHistSuccDG, Acylcarnosin, HCChol, Hist-PS and EDTA-Chol.

In yet another embodiment the lipid phase may comprise a plurality of charged amphiphiles which in combination with one another have amphoteric character.

In one aspect of this embodiment said one or more charged amphiphiles comprise a pH sensitive anionic lipid and a pH sensitive cationic lipid. Herein, such a combination of a chargeable cation and chargeable anion is referred

to as an "amphoteric II" lipid pair. Suitably, said chargeable cations have pK values of between about 4 and about 8, preferably of between about 5.5 and about 7.5. Suitably, said chargeable anions have pK values of between about 3.5 and about 7, preferably of between about 4 and about 6.5. Examples include, but are not limited to MoChol/CHEMS, DPIM/CHEMS and DPIM/DGSucc.

In a second aspect of this embodiment said one or more charged amphiphiles comprise a stable cation and a chargeable anion and is referred to as "amphoteric I" lipid pair.

Examples include, without limited to DDAB/CHEMS, DOTAP/CHEMS and DOTAP/DMG-Succ.

In a third aspect of this embodiment said one or more charged amphiphiles comprise a stable anion and a chargeable cation and is referred to as "amphoteric III" lipid pair. Examples include, but not limited to MoChol/DOPG and MoChol/Chol-SO4.

It is of course possible to use amphiphiles with multiple charges such as amphipathic dicarboxylic acids, phosphatidic acid, amphipathic piperazine derivatives and the like. Such multicharged amphiphiles might fall into pH sensitive amphiphiles or stable anions or cations or might have mixed character.

The amount of lipids containing said transfection enhancer elements is preferably between 0,1% and 90% of the total lipid phase of the liposomes. In one embodiment the amount of lipids containing said transfection enhancer elements is between 1% and 50% of the total lipid phase. In another embodiment the amount of said of lipids containing said transfection enhancer elements is between 2% and 20% of the total lipid phase of the liposomes.

Specific examples of liposomes in accordance with the present invention include, but are not limited to:

| | |
|---|---|
| POPC/DOPE/Pal-PE | 22:68:10 |
| POPC/DOPE/Pal-PE | 17:53:30 |
| POPC/DOTAP/Chems/Pal-PE | 15:28:47:10 |
| POPC/DOPE/Mochol/Chems/Pal-PE | 12:38:20:20:10 |
| POPC/DOPE/Mochol/Chems/Pal-PE | 5:22:42:21:10 |
| POPC/DOPE/Mochol/DOG-Succ/Pal-PE | 7:20:21:42:10 |
| DOPE/Mochol/Chems/Pal-PE | 50:20:20:10 |

| | |
|---|---|
| POPC/DOPE/Deca-PE | 22:68:10 |
| POPC/DOPE/Deca-PE | 17:53:30 |
| POPC/DOTAP/Chems/Deca-PE | 15:28:47:10 |
| POPC/DOPE/Mochol/Chems/Deca-PE | 12:38:20:20:10 |
| POPC/DOPE/Mochol/Chems/Deca-PE | 5:22:42:21:10 |
| POPC/DOPE/Mochol/DOG-Succ/Deca-PE | 7:20:21:42:10 |
| DOPE/Mochol/Chems/Deca-PE | 50:20:20:10 |

The liposomes of the invention may be manufactured using suitable methods that are known to those skilled in the art. Such methods include, but are not limited to, extrusion through membranes of defined pore size, injection of an alcoholic lipid solution into a water phase containing the cargo to be encapsulated, or high pressure homogenisation.

A solution of the drug (e.g. an oligonucleotide) may be contacted with the lipid phase at a neutral pH, thereby resulting in volume inclusion of a certain percentage of the solution. High concentrations of the lipids, ranging from about 50 mM to about 150 mM, are preferred to achieve substantial encapsulation of the active agent.

Amphoteric liposomes offer the distinct advantage of binding nucleic acids at or below their isoelectric point, thereby concentrating these active agents at the liposome surface. This process, called advanced loading procedure, is described in more detail in WO 02/066012.

In one embodiment of the invention amphoteric liposomes comprising one or more lipids with one or more transfection enhancer elements may be prepared by using said advanced loading procedure combined with a lipid film extrusion process. Briefly, lipids are dissolved in an organic solvent and a lipid film is produced by evaporating the solvent to dryness. The lipid film is hydrated with an aqueous phase having a pH $< 6$, preferred between 3 and 5.5, containing for example a nucleic acid. The multilamellar liposome suspension is subsequently extruded through membranes (e.g. polycarbonate) with defined pore size. Afterwards the pH of the suspension is increased to $> 7$.

In another embodiment of the invention amphoteric liposomes comprising one or more lipids with one or more transfection enhancer elements may be prepared by using said advanced loading procedure combined with an injection of an alcoholic lipid solution into a water phase containing for example a nucleic acid. This process may comprise several steps:

a) Providing a solution of a lipid mixture in a water-miscible solvent, preferably an alcohol, wherein said solution may be optionally acidified

b) Providing an aqueous solution of a nucleic acid drug, wherein said solution may be optionally acidified
At least one of the solutions in a) and b) must be acidified to a pH < 6, preferably a pH between 3 and 5.5.

c) Mix of defined amounts of the solutions of a) and b) by injecting the alcoholic solution of the lipid mixture into the aqueous solution of the nucleic acid or vice versa or by combining two controlled flows of the solutions of a) and b), optional using one or more mixing devices.

d) Dilution step, which is optional

e) Dissolve of the interactions between the amphoteric liposomes and the nucleic acids by increasing the pH to higher than 7 or by increasing the ionic strange and subsequent increasing of the pH to higher than 7.

f) Removing of non-encapsulated drug and/or concentrating the liposomal suspension and or changing the aqueous phase and/or removing the water miscible solvent, wherein each of these steps is independently optional.

g) Sterile filtration of the liposomes, which is optional

Between the steps c) and d) and/or between d) and e) and/or between e) and f) and/or between f) and g) an extrusion of the liposomes may be part of the process.
Between the steps e) and f) and/or between f) and g) one or more freeze/thaw cycles of the liposomes may be part of the process.
After step g) one or more freeze/thaw cycles and/or a lyophilisation of the liposomes may be part of the process.
Preferably, said alcohol may be selected from, without limited to, ethanol, propanol or isopropanol, optionally acidified using a buffer or an acid.
The pH of the acidic solutions used in the advanced loading procedure may be adjusted with known buffer substances like acetate-buffer or citrate-buffer. Alternatively, the pH may be adjusted using an acid (e.g. HCl, acetic acid or citric acid). Preferably, pharmaceutical acceptable buffers, like acetic acid, citric acid or glycine are used.
Irrespective of the actual production process used to make the liposomes of the invention, in some embodiments, non-encapsulated drug may be removed from the liposomes after the initial production step in which the liposomes are formed as tight containers. Again, the technical literature and the references included herein describe such methodology in detail and suitable process steps may include, but are not limited to, size exclusion chromatography, sedimentation, dialysis, ultrafiltration and diafiltration.
However, the removal of any non-encapsulated drug is not required for performance of the invention, and in some embodiments the liposomal composition may comprise free as well as entrapped drug.
The liposomes according to the present invention may be unilamellar, oligolamellar or multilamellar.
In one aspect of the invention the size of the liposomes may vary between 50 and 1000 nm, preferably between 50 and 500 nm and more preferred between 70 and 250 nm.
In other aspects the size of the liposomes may vary between 70 and 150 nm and in still other aspects the size of the liposomes may vary between 130 and 250 nm.

Table 20: Abbreviations for lipids refer primarily to standard use in the literature and are included here as a helpful reference:

| | |
|---|---|
| DMPC | Dimyristoylphosphatidylcholine |
| DPPC | Dipalmitoylphosphatidylcholine |
| DSPC | Distearoylphosphatidylcholine |
| POPC | Palmitoyl-oleoylphosphatidylcholine |
| DOPC | Dioleoylphosphatidylcholine |
| DOPE | Dioleoylphosphatidylethanolamine |
| DMPE | Dimyristoylphosphatidylethanolamine |
| DPPE Diphytanoly-PE | Dipalmitoylphosphatidylethanolamine Diphytanolyphosphatidylethanolamine |
| DOPG | Dioleoylphosphatidylglycerol |
| POPG | Palmitoyl-oleoylphosphatidylglycerol |

(continued)

| | |
|---|---|
| DMPG | Dimyristoylphosphatidylglycerol |
| DPPG | Dipalmitoylphosphatidylglycerol |
| DMPS | Dimyristoylphosphatidylserine |
| DPPS | Dipalmitoylphosphatidylserine |
| DOPS | Dioleoylphosphatidylserine |
| POPS | Palmitoyl-oleoylphosphatidylserine |
| DMPA | Dimyristoylphosphatidic acid |
| DPPA | Dipalmitoylphosphatidic acid |
| DOPA | Dioleoylphosphatidic acid |
| POPA | Palmitoyl-oleoylphosphatidic acid |
| DMPI | Dimyristoylphosphatidylinositol |
| DPPI | Dipalmitoylphosphatidylinositol |
| DOPI | Dioleoylphosphatidylinositol |
| POPI | Palmitoyl-oleoylphosphatidylinositol |
| CHEMS | Cholesterolhemisuccinate |
| Chol-Sulfate Chol-Phosphate | Cholesterolsulfate Cholesterolphosphate |
| DC-Chol | 3-β-[N-(N',N'-dimethylethane) carbamoyl]cholesterol |
| Cet-P | Cetylphosphate |
| DODAP | (1,2)-dioleoyloxypropyl)-N,N-dimethylammonium chloride |
| DOEPC | 1,2-dioleoyl-sn-glycero-3-ethylphosphocholine |
| DAC-Chol | 3-β-[N-(N,N'-dimethylethane) carbamoyl]cholesterol |
| TC-Chol | 3-β-[N-(N',N',N'-trimethylaminoethane) carbamoyl] cholesterol |
| DOTMA chloride)(Lipofectin®) | (1,2-dioleyloxypropyl)-N,N,N-trimethylammonium- |
| DOGS (Transfectam®) | ((C18)2GlySper3+) N,N-dioctadecylamido-glycyl-spermine |
| CTAB | Cetyl-trimethylammoniumbromide |
| CPyC | Cetyl-pyridiniumchloride |
| DOTAP | (1,2-dioleoyloxypropyl)-N,N,N-trimethylammonium salt |
| DMTAP | (1,2-dimyristoyloxypropyl)-N,N,N-trimethylammonium salt |
| DPTAP | (1,2-dipalmitoyloxypropyl)-N,N,N-trimethylammonium salt |
| DOTMA | (1,2-dioleyloxypropyl)-N,N,N-trimethylammonium chloride) |
| DORIE | (1,2-dioleyloxypropyl)-3 dimethylhydroxyethyl ammoniumbromide) |
| DDAB | Dimethyldioctadecylammonium bromide |
| DPIM | 4-(2,3-bis-palmitoyloxy-propyl)-1-methyl-1H-imidazole |
| CHIM | Histaminyl-Cholesterolcarbamate |
| MoChol | 4-(2-Aminoethyl)-Morpholino-Cholesterolhemisuccinate |
| HisChol | Histaminyl-Cholesterolhemisuccinate |
| HCChol | Nα-Histidinyl-Cholesterolcarbamate |
| HistChol | Nα-Histidinyl-Cholesterol-hemisuccinate |
| AC | Acylcarnosine, Stearyl- & Palmitoylcarnosine |
| HistDG hemisuccinate, & | 1,2-Dipalmitoylglycerol-hemisuccinat-N_-Histidinyl- Distearoyl- , Dimyristoyl, Dioleoyl or palmitoyl-oleoylderivatives |
| IsoHistSuccDG | 1,2-ipalmitoylglycerol-O_-Histidinyl-Nα-hemisuccinat, & |
| Distearoyl-, Dimyristoyl, Dioleoyl or palmitoyl-oleoylderivatives | |
| DGSucc 1,2-Dipalmitoyglycerol-3-hemisuccinate & Distearoyl-, dimyristoyl- Dioleoyl or palmitoyl-oleoylderivatives | |
| EDTA-Chol | cholesterol ester of ethylenediaminetetraacetic acid |
| Hist-PS | Nα-histidinyl-phosphatidylserine |
| BGSC | bisguanidinium-spermidine-cholesterol |
| BGTC | bisguanidinium-tren-cholesterol |
| DOSPER | (1.3-dioleoyloxy-2-(6-carboxy-spermyl)-propylamide |
| DOSC | (1,2-dioleoyl-3-succinyl-sn-glyceryl choline ester) |

(continued)

| | |
|---|---|
| DOGSDO ornithine) | (1,2-dioleoyl-sn-glycero-3-succinyl-2-hydroxyethyl disulfide |
| DOGSucc | 1,2-Dioleoylglycerol-3-hemisucinate |
| POGSucc | Palimtolyl-oleoylglycerol-oleoyl-3-hemisuccinate |
| DMGSucc | 1,2-Dimyristoylglycerol-3-hemisuccinate |
| DPGSucc | 1,2-Dipalmitoylglycerol-3-hemisuccinate |
| Pal-PE | 16- {2-[2,3-Bis-hexadecanoyloxy-propoxy)-hydroxy-phosphoryloxy]-ethylamino}-hexadecanoic acid |
| Deca-PE | 16- {2-[2,3-Bis-hexadecanoyloxy-propoxy)-hydroxy-phosphoryloxy]-ethylamino}-decanoic acid |

Table 21: Non-limiting examples of lipids that are suitable for use in the compositions in accordance with the present invention. The membrane anchors of the lipids are shown exemplarily and serve only to illustrate the lipids of the invention and are not intended to limit the same.

| | |
|---|---|
| MoChol<br>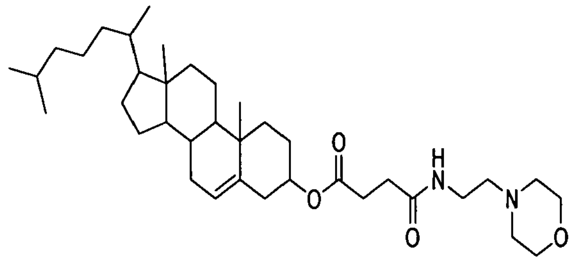 | DG-Succ<br>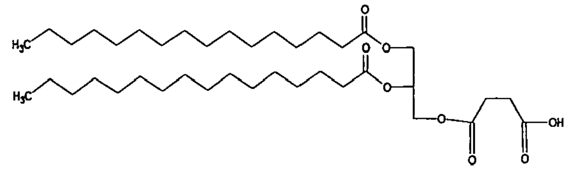 |
| DOTAP<br>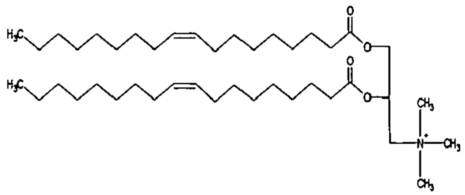 | IsohistsuccDG<br>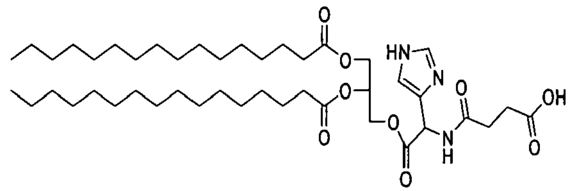 |
| HisChol<br>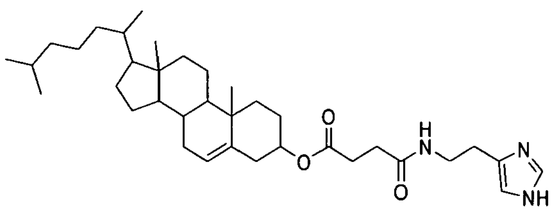 | HCChol<br>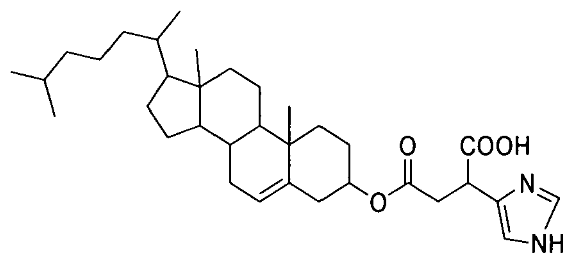 |
| AC | Hist-Chol |

(continued)

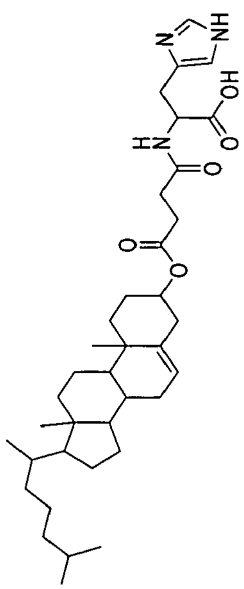

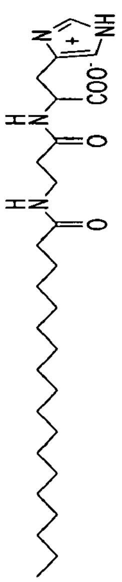

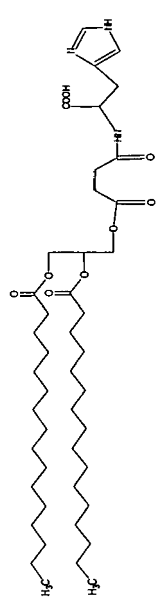

Hist-DG

Transfection

According to the present invention lipid assemblies comprising lipids with a transfection enhancer element may be used to transfect cells in vitro, in vivo or ex vivo. Without being limited to such use, the lipid assemblies (e.g. the liposomes) described in the present invention are well suited for use as carriers for nucleic acid-based drugs such for example as oligonucleotides, polynucleotides and DNA plasmids. These drugs are classified into nucleic acids that encode one or more specific sequences for proteins, polypeptides or RNAs and into oligonucleotides that can specifically regulate protein expression levels or affect the protein structure through interference with splicing, artificial truncation and others.

In some embodiments of the present invention, therefore, the nucleic acid-based therapeutic may comprise a nucleic acid that is capable of being transcribed in a vertebrate cell into one or more RNAs, which RNAs may be mRNAs, shRNAs, miRNAs or ribozymes, wherein such mRNAs code for one or more proteins or polypeptides. Such nucleic acid therapeutics may be circular DNA plasmids, linear DNA constructs, like MIDGE vectors (Minimalistic Immunogenically Defined Gene Expression) as disclosed in WO 98/21322 or DE 19753182, or mRNAs ready for translation (e.g., EP 1392341).

In another embodiment of the invention, oligonucleotides may be used that can target existing intracellular nucleic acids or proteins. Said nucleic acids may code for a specific gene, such that said oligonucleotide is adapted to attenuate or modulate transcription, modify the processing of the transcript or otherwise interfere with the expression of the protein. The term "target nucleic acid" encompasses DNA encoding a specific gene, as well as all RNAs derived from such DNA, being pre-mRNA or mRNA. A specific hybridisation between the target nucleic acid and one or more oligonucleotides directed against such sequences may result in an inhibition or modulation of protein expression. To achieve such specific targeting, the oligonucleotide should suitably comprise a continuous stretch of nucleotides that is substantially complementary to the sequence of the target nucleic acid.

Oligonucleotides fulfilling the abovementioned criteria may be built with a number of different chemistries and topologies. The oligonucleotides may comprise naturally occurring or modified nucleosides comprising but not limited to DNA, RNA, locked nucleic acids (LNA's), 2'O-methyl RNA (2'Ome), 2' O-methoxyethyl RNA (2'MOE) in their phosphate or phosphothioate forms or Morpholinos or peptide nucleic acids (PNA's). Oligonucleotides may be single stranded or double stranded.

The mechanisms of action of oligonucleotides may vary and might comprise effects on splicing, transcription, nuclear-cytoplasmic transport and translation, amongst others.

In a preferred embodiment of the invention single stranded oligonucleotides may be used including but are not limited to DNA-based oligonucleotides, locked nucleic acids, 2'-modified oligonucleotides and others, commonly known as antisense oligonucleotides. Backbone or base or sugar modifications may include but are not limited to Phosphothioate DNA (PTO), 2'O-methyl RNA (2'Ome), 2' O- methoxyethyl-RNA (2'MOE), peptide nucleic acids (PNA), N3'-P5' phosphoamidates (NP), 2'fluoroarabino nucleic acids (FANA), locked nucleic acids (LNA), Morpholine phosphoamidate (Morpholino), Cyclohexene nucleic acid (CeNA), tricyclo-DNA (tcDNA) and others. Moreover, mixed chemistries are known in the art, being constructed from more than a single nucleotide species as copolymers, block-copolymers or gapmers or in other arrangements.

In addition to the aforementioned oligonucleotides, protein expression can also be inhibited using double stranded RNA molecules containing the complementary sequence motifs. Such RNA molecules are known as siRNA molecules in the art (e.g., WO 99/32619 or WO 02/055693). Again, various chemistries were adapted to this class of oligonucleotides. Also, DNA / RNA hybrid systems are known in the art.

In another embodiment of the present invention, decoy oligonucleotides can be used. These double stranded DNA molecules and chemical modifications thereof do not target nucleic acids but transcription factors. This means that decoy oligonucleotides bind sequence-specific DNA-binding proteins and interfere with the transcription (e.g. Cho-Chung, et al. in Curr. Opin. Mol. Ther., 1999).

In a further embodiment of the invention oligonucleotides that may influence transcription by hybridizing under physiological conditions to the promoter region of a gene may be used. Again various chemistries may adapt to this class of oligonucleotides.

In a further alternative of the invention, DNAzymes may be used. DNAzymes are single-stranded oligonucleotides and chemical modifications thereof with enzymatic activity.

Typical DNAzymes, known as the "10-23" model, are capable of cleaving single-stranded RNA at specific sites under physiological conditions. The 10-23 model of DNAzymes has a catalytic domain of 15 highly conserved deoxyribonucleotides, flanked by 2 substrate-recognition domains complementary to a target sequence on the RNA._Cleavage of the target mRNAs may result in their destruction and the DNAzymes recycle and cleave multiple substrates.

In another embodiment of the invention, ribozymes can be used. Ribozymes are single-stranded oligoribonucleotides and chemical modifications thereof with enzymatic activity. They can be operationally divided into two components, a conserved stem-loop structure forming the catalytic core and flanking sequences which are reverse complementary to sequences surrounding the target site in a given RNA transcript. Flanking sequences may confer specificity and may generally constitute 14-16 nt in total, extending on both sides of the target site selected.

In a further embodiment of the invention aptamers may be used to target proteins. Aptamers are macromolecules composed of nucleic acids, such as RNA or DNA, and chemical modifications thereof that bind tightly to a specific molecular target and are typically 15-60 nt long. The chain of nucleotides may form intramolecular interactions that fold the molecule into a complex three-dimensional shape. The shape of the aptamer allows it to bind tightly against the surface of its target molecule including but not limited to acidic proteins, basic proteins, membrane proteins, transcription factors and enzymes. Binding of aptamer molecules may influence the function of a target molecule.

All of the above-mentioned oligonucleotides may vary in length between as little as 5, preferably between 8 and 50 nucleotides. The fit between the oligonucleotide and the target sequence is preferably perfect with each base of the oligonucleotide forming a base pair with its complementary base on the target nucleic acid over a continuous stretch of the abovementioned number of oligonucleotides. The pair of sequences may contain one or more mismatches within the said continuous stretch of base pairs, although this is less preferred. In general the type and chemical composition of such nucleic acids is of little impact for the performance of the inventive liposomes as vehicles be it in vivo or in vitro and the skilled artisan may find other types of oligonucleotides or nucleic acids suitable for combination with the liposomes.

A further aspect of the invention relates to pharmaceutical compositions comprising lipid assemblies comprising one or more lipids with one or more transfection enhancer elements as a carrier for the targeted delivery of active agents or ingredients, including drugs such as nucleic acid drugs, e.g., oligonucleotides and plasmids. The pharmaceutical composition of the present invention may be formulated in a suitable pharmacologically acceptable vehicle. Vehicles such as water, saline, phosphate buffered saline and the like are well known to those skilled in the art for this purpose.

In some embodiments said pharmaceutical compositions may be used for the treatment or prophylaxis of inflammatory, immune or autoimmune disorders and/or cancer of humans or non-human animals.

A yet further aspect of the present invention relates to methods for the treatment of human or non-human animals in which said pharmaceutical composition comprising liposomes, which have lipids with a transfection enhancer element in their membrane, as a carrier for active agents or ingredients is targeted to a specific organ or organs, tumours or sites of infection or inflammation.

In the drawings:

Figure 1 shows the logD response of compound 11-14

Figure 2 shows the relation between logD and pH for different hydrophiles in TEE structures. All hydrophilic elements are located at the terminal position of the alkyl chain. LogD starts to decrease at pH values roughly equal to pKa, such decrease being limited by ion pair formation at physiological ionic strength.

Figure 3 shows standardized curves for logD wherein pH-pKa is used as x-axis for different hydrophiles in TEE structures. All hydrophilic elements are located at the terminal position of the alkyl chain. 3-cholor octanoic acid is identical with octanoic acid and not shown in the plot.

Figure 4 shows an analysis of the increment of logD vs. pH. The curve was standardized using pH-pKa as a common descriptor that is independent from the pKa of the individual hydrophilic elements.

Figure 5 shows fluorescence microscopy images of Hela-cells tranfected with formulations L-1, L-2 and L-3 after 24 hours(40x).

Figure 6 shows fluorescence microscopy images of Hela-cells tranfected with formulations L-4, L-5 and L-6 after 24 hours(40x).

Figure 7 shows fluorescence microscopy images of Hela cells transfected with formulations L-7 - L-10 (40x).

Figure 8 shows fluorescence microscopy images of Hela cells transfected with formulations L-12 - L-14 (40x).

Figure 9 shows fluorescence microscopy images of Hela cells transfected with formulations L-17 - L-20 (40x).

Figure 10 shows fluorescence microscopy images of Hela cells transfected with formulations L-21 - L-24 (40x).

Figure 11 shows fluorescence microscopy images of Hela cells transfected with free carboxyfluorescein (C1) or an anionic liposomal standard formulation (DPPC: DPPG: Chol 50:10:40) (C2),(10x).

Figure 12 shows the cryosected tumor tissue of tumor-bearing mice received an intravenous injection of liposomal formulations L-25 and L-26.

## Examples

The invention will be further described in the following examples, which do not limit the scope of the invention.

Example 1:

Synthesis of (16-{2-[2,3-Bis-hexadecanoyloxy-propoxy)-hydroxy-phosphoryl-oxy]-ethylamino}-hexadecanoic acid)(Pal-PE)

Reaction scheme:

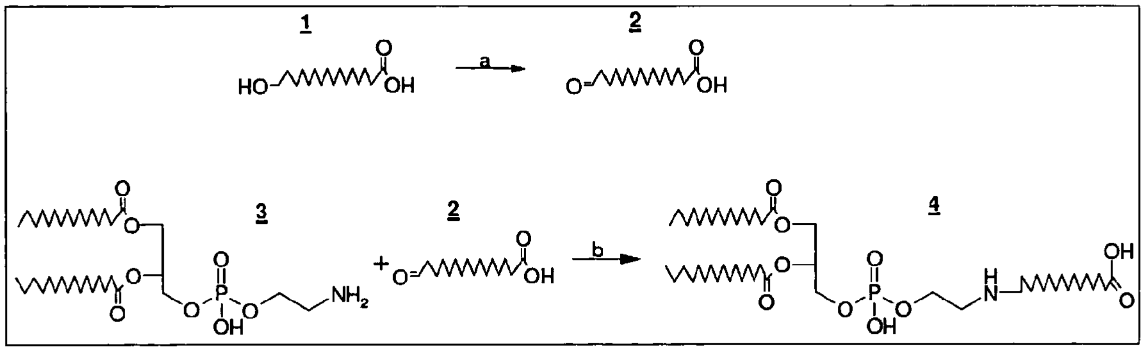


First step (a): Oxidation of 16-Hydroxy-hexadecanoic acid to 16-Oxo-hexadecanoic acid 10 g of compound 1 (1 eq.) was oxidized with 16 g PCC (Pyridinium chlorochromate) in dichloromethane for 15 min at 40°C. The solvent was removed and the residue was resolved in acetic acid ethyl ester followed by a chromatography step on silica gel.

Second step (b): Reductive amination of compound 2 with dipalmitoyl phosphatidylethanolamine (DPPE) 11,8 g of DPPE (3) and 16 g sodium cyano-boronhydride ($NaCNBH_3$) were dissolved in 800 ml $CHCl_3/CH_3OH$ 1:1 and heated to 65 °C. 9,44 g pyridine was added to the mixture. 4.6 g of compound 2 was dissolved in 400 ml $CHCl_3/CH_3OH$ 1:1 and finally also added drop wise to the reaction mixture. After three hours the solvent was removed and the solid was washed two times with water before resolved in 200 ml $CHCl_3/CH_3OH/H_2O$ 500:100:4. The product was purified by a chromatography on silica gel.

Example 2:

Synthesis of compound III (16-{2-[2,3-Bis-hexadecanoyloxy-propoxy)-hydroxy-phosphoryloxy]-ethylamino}-decanoic acid) (Deca-PE)

This synthesis was performed according to example 1 instead of using 10-Hydroxydecanoic acid in step a.

Example 3:

Preparation of amphoteric liposomes encapsulating Cy3-labelled antisense oligonucleotides

Following liposomal preparations were prepared as described below:

Table 22: Formulations L-1 - L-5

| Formulation ID | Lipids | Mol% |
|---|---|---|
| L-1 | DOPE/MoChol/Chems/Pal-PE | 50:20:20:10 |
| L-2 | DOPE/MoChol/Chems/Deca-PE | 50:20:20:10 |
| L-3 | DOPE/MoChol/Chems | 50:20:30 |
| L-4 | POPC/DOPE/MoChol/Chems/Pal-PE | 12:38:20:20:10 |
| L-5 | POPC/DOPE/MoChol/Chems/Deca-PE | 12:38:20:20:10 |

Stock solutions of lipids in chloroform were mixed and finally evaporated in a round bottom flask to dryness under vacuum. Lipid films were hydrated with 1 ml Cy3-labelled antisense oligonucleotide solution in PBS pH 7.5 (0.5 mg/ml). The resulting lipid concentration was 50 mM. The suspensions were hydrated for 45 minutes in a water bath at room

temperature, sonicated for 5 minutes following by three freeze/thaw cycles at - 70˚C. After thawing the liposomal suspensions were extruded 19 times through polycarbonate membranes with a pore size of 800/200/800 or 200/200 nm. Non-encapsulated oligonucleotide was removed by sedimentation. One formulation was prepared by a modified process:

Table 23: Formulation L-6

| Formulation ID | Lipids | Mol% |
|---|---|---|
| L-6 | POPC/DOPE/MoChol/Chems | 15:45:20:20 |

Briefly, lipids were weighed into a round bottom flask and solved in chloroform. Then a lipid film was produced by evaporating the solvent using a rotary evaporator. Chloroform residues were removed in vacuum overnight. Multilamellar vesicles were formed during the hydratisation of the lipid film with 1 ml Cy3-labelled antisense oligonucleotide solution in 10 mM NaAc, 50mM NaCl pH 4.5 (0,35 mg/ml). After a freeze thaw step multilamellar liposomes were extruded 19 times through 800/200/800 or nm polycarbonate membranes. After the extrusion the pH of the liposome suspension was immediately shifted with 1/10 volume 1M Hepes pH 8. Non-encapsulated oligonucleotide was removed by sedimentation.

Lipid recovery and concentration was analysed by organic phosphate assay. Particle size was measured by dynamic light scattering on a Malvern Zetasizer 3000 HSA. Encapsulation efficiency was measured by fluorescence spectroscopy.

Results:

Table 24: Size, PI (Polydispersity index) and encapsulation efficiencies of formulations L-1 - L-6

| Formulation ID | Size [nm] / PI | Encapsulation efficiency [%] |
|---|---|---|
| L-1 | 275/0.183 | 12 |
| L-2 | 251/0387 | 18 |
| L-3 | 173/0.068 | 11 |
| L-4 | 244/0.186 | 20 |
| L-5 | 269/0.396 | 15 |
| L-6 | 227/0.052 | 69 |

Example 4:

Preparation of amphoteric liposomes encapsulating Carboxyfluorescein

Following liposomal preparations were prepared as described below:

Table 25: Formulations L-7 - L10, L-12 - L-15 and L-17 - L-24

| Formulation ID | Lipids | Mol% |
|---|---|---|
| L-7 | DOPE/MoChol/Chems/Pal-PE | 50:20:20:10 |
| L-8 | DOPE/MoChol/Chems/Pal-PE | 56:20:20:4 |
| L-9 | DOPE/MoChol/Chems/Deca-PE | 50:20:20:10 |
| L-10 | DOPE/MoChol/Chems/Deca-PE | 56:20:20:4 |
| L-12 | POPC/DOPE/MoChol/Chems/Pal-PE | 12:38:20:20:10 |
| L-13 | POPC/DOPE/MoChol/Chems/Pal-PE | 15:41:20:20:4 |
| L-14 | POPC/DOPE/MoChol/Chems/Deca-PE | 12:38:20:20:10 |
| L-15 | POPC/DOPE/MoChol/Chems/Deca-PE | 15:41:20:20:4 |
| L-17 | POPC/DOTAP/Chems/ Pal-PE | 50:10:30:10 |
| L-18 | POPC/DOTAP/Chems/ Pal-PE | 56:10:30:4 |

(continued)

| Formulation ID | Lipids | Mol% |
|---|---|---|
| L-19 | POPC/DOTAP/Chems/ Deca-PE | 50:10:30:10 |
| L-20 | POPC/DOTAP/Chems/ Deca-PE | 56:10:30:4 |
| L-21 | POPC/DOTAP/Chems/ Pal-PE | 15:28:47:10 |
| L-22 | POPC/DOTAP/Chems/ Pal-PE | 21:28:47:4 |
| L-23 | POPC/DOTAP/Chems/ Deca-PE | 15:28:47:10 |
| L-24 | POPC/DOTAP/Chems/ Deca-PE | 21:28:47:4 |

Stock solutions of lipids in chloroform were mixed and finally evaporated in a round bottom flask to dryness under vacuum. Lipid films were hydrated with 100 mM CF in PBS pH 7.5. The resulting lipid concentration was 20 mM. The suspensions were hydrated for 45 minutes in a water bath at room temperature, sonicated for 5 minutes following by three freeze/thaw cycles at -70°C. After thawing the liposomal suspensions were extruded 15 times through polycarbonate membranes with a pore size of 800/200/800 nm. Non-encapsulated CF was removed by gel filtration, whereas the liposomes were diluted by a factor three. Lipid recovery and concentration was analysed by organic phosphate assay. Particle size was measured by dynamic light scattering on a Malvern Zetasizer 3000 HSA.

Results:

Table 26: Size and PI of formulations L-7 - L10, L-12 - L-15 and L-17 - L-24

| Formulation ID | Size [nm] / PI |
|---|---|
| L-7 | 254/0.317 |
| L-8 | 192/0.114 |
| L-9 | 185/0.154 |
| L-10 | 175/0.074 |
| L-12 | 225/0.292 |
| L-13 | 181/0.117 |
| L-14 | 165/0.265 |
| L-15 | 169/0.113 |
| L-17 | 190/0.271 |
| L-18 | 205/0.211 |
| L-19 | 194/0.288 |
| L-20 | 143/0.190 |
| L-21 | 211/0.190 |
| L-22 | 212/0.244 |
| L-23 | 166/0.166 |
| L-24 | 141/0.117 |

Example 5:

In vitro transfection experiment with liposomes encapsulating Cy3-labelled antisense oligonucleotides or carboxyfluorescein

HeLa-Cells were obtained from DSMZ (German Collection of Micro Organism and Cell Cultures) and maintained in DMEM. Media were purchased from Gibco-Invitrogen and supplemented with 10% FCS at 37 OC under 5% CO2. The cells were plated at a density of 2 x $10^4$ cells/ml and cultivated in 100 μl medium. After 16 h the media were replaced with Opti-MEM I (Gibco) containing liposomes (L1-L24) with a total amount of 50 to 200 ng Cy3-labelled oligonucleotides

or 0,5 mM CF / well. Cell culture dishes were centrifuged for 1 h at 450 g at 37 °C, followed by an incubation of 3 h hours at 37 °C under 5% CO2. The transfection mixture was replaced by the above mentioned medium and cells were incubated for further 24-48 h hours. Transfection efficiency and cellular distribution was determined after 4 or 24 hours by light and fluorescence microscopy (Axionvert S 100, Carl Zeiss Inc.).

Results:

Transfection of HeLa-cells with liposomes encapsulating Cy3-labelled oligonucleotides: Figure 5 shows fluorescence microscopy images of Hela-cells transfected with formulations L-1, L-2 and L-3. It is shown that the transfection efficiency of the liposomal formulation L-3 can be improved by inclusion of a lipid with a transfection enhancer element in the liposomal membrane (L-1 and L-2).
Figure 6 shows fluorescence microscopy images of Hela-cells transfected with formulations L-4, L-5 and L-6. It is shown that the transfection efficiency of the liposomal formulation L-6 can be improved by inclusion of a lipid with a transfection enhancer element in the liposomal membrane (L-4 and L-5).
Transfection of HeLa-cells with liposomes encapsulating carboxyfluorescein:
Carboxyfluorescein is a pH-sensitive fluorescence probe which do not show a fluorescence signal in acidic environment (e.g. endosomes).
Figure 7 shows the fluorescence microscopy images of Hela cells transfected with formulations L-7 - L-10. All formulations show fluorescence signals in the cytosol or nucleus. The fluorescence signals are improved with formulation having 10% Pal-PE or Deca-PE in their membrane (L-7 and L-9).
Figure 8 shows the fluorescence microscopy images of Hela cells transfected with formulations L-12 - L-14. In this case the fluorescence signal of the carboxyfluorescein is more intensive for formulations L-12 and L-13 having Pal-PE in their membrane. The amount of Pal-PE (4% or 10%) does not influence the fluorescence signal.
Figure 9 shows the fluorescence microscopy images of Hela cells transfected with formulations L-17 - L-20. In this case the fluorescence signal of the carboxyfluorescein is more intensive for formulations L-17 and L-19 having Pal-PE or 10 % Deca-PE in their membrane.
Figure 10 shows the fluorescence microscopy images of Hela cells transfected with formulations L-21 - L-24. All formulations show fluorescence signals in the cytosol or nucleus. The fluorescence signals are improved with formulation having 10% Pal-PE or Deca-PE in their membrane (L-21 and L-23).
Figure 11 shows fluorescence microscopy images of Hela cells transfected with free carboxyfluorescein or an anionic liposomal standard formulation (DPPC: DPPG: Chol 50:10:40). Both formulations do not show any fluorescence signals.

<u>Example 6:</u>

Biodistribution of amphoteric liposomes in mice bearing tumor xenografts

Preparation of liposomes encapsulating C5.5 antisense oligonucleotides
Liposomes were manufactured by an alcohol-injection method. Lipid mixtures were dissolved in the appropriate alcohol (ethanol for L-25 and isopropanol/1% HCl for L-26) at a volume of 8 ml and an appropriate concentration (40 mM for L-25 and 20 mM for L-26) according to the distinct formulation. A volume of 72 ml of Cy5.5 labelled antisense oligonucleotide solution in 20 mM HAc, 300 mM sucrose, buffer, pH 4.5 at the appropriate concentration (57 μg/ml for L-25 and 67 μg/ml for L-26), was transferred to a roundbottom flask. Both solutions were mixed using an injection device with pumps. The resulting liposomal suspensions were shifted to pH7.5 with 1/10 of the total volume with 1 M tris(hydroxymethyl) aminomethane (Tris) HCl, pH 8, dialyzed against PBS to remove non-encapsulated Cy 5.5 labelled antisense oligonucleotides and subsequent concentrated.

Table 27: Formulations L-25 and L-26

| Form.ID | Lipids | Mol% | Size [nm]/PI | Encapsulation efficiency [%] |
|---|---|---|---|---|
| L-25 | POPC/DOTAP/Chems | 25:28:47 | 122/0.211 | 91 |
| L-26 | POPC/DOTAP/Chems/ Pal-PE | 15:28:47:10 | 165/0.194 | 67 |

Preparation of empty liposomes

Lipid mixtures for empty liposomes were dissolved in either ethanol (L-27) or isopropanol/1% HCL (L-28) with a concentration of 50mM. Empty liposomes were prepared by mixing the lipids in organic solvent with an aqueous buffer

(PBS or 10mM Hac, 300mM Sucrose, 100mM Tris, pH7.5). Liposomal suspension were dialyzed against PBS and subsequent concentrated.

Table 28: Formulations L-27 and L-28

| Form.ID | Lipids | Mol% | Size [nm]/PI |
|---|---|---|---|
| L-27 | POPC/DOTAP/Chems | 25:28:47 | 62/0.311 |
| L-28 | POPC/DOTAP/Chems/ Pal-PE | 15:28:47:10 | 174/0.124 |

Cy 5.5 antisense oligonucleotide loaded liposomes and empty liposomes were mixed to a final lipid concentration of 60 mM.

Biodistribution study

50 μg of liposomal Cy 5.5 labelled antisense oligonucleotides (2 mg/kg) were injected intravenously into the tail vein of CD1 nude mice having a xenograft tumor (human hepatoma) (diameter of 6-8 mm). The group size was 2 mice per formulation. A control group received 150 μl PBS intravenously. 24 hours later mice were sacrificed and tumors were collected and frozen.

Deep-frozen tumors were partially embedded into OCT (Tissue-Tek) and 10μm sections were made with the Cryostat Cryo-Star HM560 (Microm-International, Walldorf, Germany) at -20˚C. Sections were collected and dried at Super Frost® Plus Gold slides (Menzel, Braunschweig, Germany) and stored at 4˚C.

Imaging of cryosected organ slices was performed with LI-COR Odyssey Infrared Imaging Systems (LI-COR Biosciences GmbH, Bad Homburg, Germany)

Results:
After an intravenous application of Cy5.5 labelled antisense oligonucleotides, encapsulated into liposomal formulations L-25 and L-26, an accumulation in tumor tissue was found. Figure 12 shows the cryosected tumor tissue of both animals of a group. Compared to formulation L-25 the Pal-PE formulation L-26 shows an improved accumulation in tumor tissue by a factor 5.4. The tumor tissue of the animals received PBS buffer do not show any fluorescence signal.

**Claims**

1. Use of lipid assemblies comprising one or more lipids with one or more transfection enhancer elements for the in vivo, in vitro or ex-vivo transfection of cells wherein said lipids have the general formula (I) and said transfection enhancer elements have the general formula (II).

Lipid- Hydrophobic element - pH sensitive hydrophilic element (I)

Hydrophobic element - pH sensitive hydrophilic element (II)

2. Use of lipids assemblies as claimed in claim 1 wherein said pH sensitive hydrophilic element is located distal from the link between said lipid and TEE.

3. Use of lipid assemblies as claimed in claim 1 wherein said pH sensitive hydrophilic element is located central within the TEE.

4. Use of lipid assemblies as claimed in any of claims 1 to 3 wherein said pH sensitive hydrophilic element comprises weak acids having a pKa of between 2 and 6, preferred of between 3 and 5.

5. Use of lipid assemblies as claimed in claim 4 wherein said weak acids are selected from the group comprising carboxyl groups, barbituric acid and derivatives thereof, xanthine and derivatives thereof.

6. Use of lipid assemblies as claimed in any of claims 1 to 3 wherein said pH sensitive hydrophilic element is a zwitterionic structure comprising a combination of weak or strong acidic groups with weak bases having a pKa of between 3 and 8, preferred of between 4.5 and 7.

7. Use of lipid assemblies as claimed in claim 6 wherein said zwitterionic structure may be formed from an anionic

group and a heterocyclic nitrogen atom as cationic group.

**8.** Use of lipid assemblies as claimed in any of claim 1 to 7 wherein said pH-responsive hydrophilic element may comprise further polar or apolar groups, selected from the group of hydroxymethyl-, hydroxyethyl-, methoxymethyl-, methoxyethyl-, ethoxymethyl-, ethoxyethyl-, thiomethyl-, thioethyl-, methylthiomethyl-, methylthioethyl-, ethylthiomethyl-, ethylthioethyl-, chloro-, chlormethyl- vinyl-, phenyl-, benzyl-, methyl-, ethyl- , propyl-, isopropyl- and tert-butyl or cyclohexyl groups.

**9.** Use of lipid assemblies as claimed in any of claim 1 to 8 wherein said hydrophobic element comprises linear, branched or cyclic chains with a minimum chain length of 6 elements.

**10.** Use of lipid assemblies as claimed in any of claim 1 to 9 wherein said hydrophobic element comprises more than 6 and up to 40 chain elements.

**11.** Use of lipid assemblies as claimed in any of claims 1 to 10 wherein said hydrophobic element comprises between 6 and 20 chain elements.

**12.** Use of lipid assemblies as claimed in any of claims 1 to 10 wherein said hydrophobic element comprises between 20 and 40 chain elements.

**13.** Use of lipid assemblies as claimed in any of claims 1 to 12 wherein the chain elements of said hydrophobic element are carbon atoms.

**14.** Use of lipid assemblies as claimed in any of claims 1 to 13 wherein said hydrophobic element can be saturated or may contain unsaturated bonds.

**15.** Use of lipid assemblies as claimed in any of claims 1 to 14 wherein said hydrophobic element may be substituted.

**16.** Use of lipid assemblies as claimed in any of claims 1 to 15 wherein the branching of the main chain of said hydrophobic element comprises one or more rather small building blocks such as methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof.

**17.** Use of lipid assemblies as claimed in any of claims 1 to 16 wherein said hydrophobic element derives from sterols.

**18.** Use of lipid assemblies as claimed in claim 17 wherein said sterols are further substituted with substituents selected from the group comprising methyl-, ethyl-, propyl-, isopropyl-, methoxy-, ethoxy-, methoxymethyl-, ethoxymethyl-, methoxyethyl, ethoxyethyl- and vinyl-, halogen- or hydroxyl-groups or mixtures thereof.

**19.** Use of lipid assemblies as claimed in any of claims 1 to 18 wherein said hydrophobic element comprises one or more heteroatoms or chemical linking groups, selected from the group comprising -O-, -S-, -N(H)C(O)-, -C(O)O-, -OC(O)N(H)-, -C(O)-, -C(O)-N(H)-, -N(H)-C(O)-O-, -CH=N-, -O-C(O)-, -N=CH- and/or -S-S-, amino acids or derivatives thereof, $\alpha$-hydroxyacids or $\beta$-hydroxy acids.

**20.** Use of lipid assemblies as claimed in any of claims 1 to 19 wherein said pH-responsive transfection enhancer elements (TEE's) have a difference in logD(4.0)- logD(7.4) that is greater than 1 logD unit.

**21.** Use of lipid assemblies as claimed in any of claims 1 to 19 wherein said pH-responsive transfection enhancer elements (TEE's) have a logD at pH 7,4 of between 1 and 10.

**22.** Use of lipid assemblies as claimed in any of claims 1 to 19 wherein the logD at pH 4 of said pH-responsive transfection enhancer elements (TEE's) exceeds 0.

**23.** Use of lipid assemblies as claimed in any of claims 1 to 22 wherein said pH-responsive transfection enhancer elements (TEE's) comprise more than one pH-responsive hydrophilic element.

**24.** Use of lipid assemblies as claimed in any of claims 1 to 23 wherein said lipid assemblies are selected from the group comprising liposomes of various size and lamellarity, micelles, inverted micelles, cubic or hexagonal lipid

phases, cochleates, emulsions, double emulsions or other multimeric assemblies that are substantially build from lipids, oils or amphiphiles.

**25.** Use of lipid assemblies as claimed in any of claims 1 to 24 wherein said TEE's are chemically linked to said lipid which is selected from the group comprising phospholipids and their lyso forms, sphingolipids and their lyso forms, sterols like cholesterols and derivatives therof, diacylglycerols/ dialkylglycerols, monacylglycerols/ monoalkylglycerols, monoester, diesters, monoethers or diethers of glyceric acid, sphingosines/ phytosphingosines/ sphinganines and N-substituted derivatives thereof, ceramides, 1,2-Diacyl-3-aminopropanes/ 1,2 -Dialkyl-3-aminopropanes, 1- or 2- monoacyl - 3 aminopropanes/ 1-or 2- monoalkyl-3-aminopropanes, dialkylamines, monoalkylamines, fatty acids, dicarboxylic acid alkyl ester, tricarboxylic acid dialkyl ester optionally substituted with -OH groups or esters of tartaric acid with long chain alcohols or esters of tartaric acid with long chain carboxylic acids.

**26.** Use of lipid assemblies as claimed in claim 25 wherein (i) the acyl- and alkyl-chains of said amphiphilic lipid substances comprise independently 8-30 carbon atoms and 0, 1 or 2 ethylenically unsaturated bonds or (ii) said sterols are cholestane derivatives.

**27.** Use of lipid assemblies as claimed in any of claims 1 to 26 wherein said pH sensitive transfection enhancer elements are chemically linked or grafted on the polar head group of said lipid.

**28.** Use of lipid assemblies as claimed in any of claims 1 to 27 wherein said lipids include chemical linkers between the graft and the pH sensitive transfection enhancer elements selected from the group comprising -O-, -S-, -N(H)C(O)-, -C(O)O-, -OC(O)N(H)-, - C(O)-, -C(O)-N(H)-, -N(H)-C(O)-O-, -CH=N-, -O-C(O)-, -N=CH-, -S-S-, non-branched, branched or cyclic alkyl, alkylene or alkynyl with $C_1$-$C_6$ atoms and optionally substituted with one or more -OH, $-NR_2$, -COOH or sugars or mixtures thereof; $-PO_4$--; $-PO_3$--; pyrophosphate; $-SO_4$-; $-SO_3$-; -NH-; -NR-; sugars and derivatives thereof; amino acids; Di- or Tripeptides, $\alpha$-hydroxyacids or $\beta$-hydroxy acids or dihydroxyacids.

**29.** Use of lipid assemblies as claimed in any of claims 1 to 28 wherein said polar headgroup of the lipid is substituted.

**30.** Use of lipid assemblies as claimed in claim 29 wherein said substituents are polar and selected from the group comprising -OH, -COOH, $-NH_2$, -NHR, $-NR_2$, sugars and derivatives thereof, amino acids and derivatives thereof, $-OPO_3^{2-}$, $-OPO_2^{2-}$, $-OSO_3^-$; - $OSO_2$- or mixtures thereof.

**31.** Use of lipid assemblies as claimed in any of claims 1 to 30 wherein said lipids contain more than one hydrophilic polar head group or complex hydrophilic head groups that allow substitution on various positions.

**32.** Use of lipid assemblies as claimed in claim 31 wherein said lipids are selected from the group comprising esters of tartaric acid with long chain alcohols; derivatives of maleic acid; esters of fatty acids with sugars such as glucose, sucrose or maltose; alkyl glycosides and derivatives thereof, such as dodecyl-$\beta$-D-glucopyranoside or dodecyl-$\beta$-D-maltoside; derivatives of alkyl-ethylenglycol detergents, such as Brij35, Genapol series, Thesit or Phosphatidylinositols and derivatives thereof.

**33.** Use of lipid assemblies as claimed in any of claims 1 to 32 wherein said lipid assemblies are formed from a lipid phase further comprising neutral and/or cationic and/or anionic lipids.

**34.** Use of lipid assemblies as claimed in any of claims 1 to 33 wherein the overall charge of said lipid assemblies is neutral, cationic or anionic.

**35.** Use of lipid assemblies as claimed in claim 33 or claim 34 wherein said neutral lipids are selected from the group comprising phosphatidylcholines; phosphatidylethanolamines; sphingolipids; ceramides; cerebrosides; sterol-based lipids, e.g. cholesterol; and/or derivatives of such lipids.

**36.** Use of lipid assemblies as claimed in claim 35 wherein said neutral lipids are selected from the group comprising DMPC, DPPC, DSPC, POPC, DOPC, DMPE, DPPE, DSPE, POPE, DOPE, Diphythanoyl-PE, sphingomyelein, ceramide and/or cholesterol.

**37.** Use of lipid assemblies as claimed in claim 33 or claim 34 wherein said cationic lipids are selected from the group comprising DOTAP, DMTAP, DPTAP, DC-Chol, DAC-Chol, DODAP, DOEPC, TC-Chol, DOTMA, DORIE, DDAP, CTAB, CPyC, DPIM, CHIM, MoChol, HisChol, BGSC, BGTC, DOSPER, DOSC, DOGSDO and derivatives of such

lipids.

**38.** Use of lipid assemblies as claimed in claim 33 or claim 34 wherein said anionic lipids are selected from the group comprising phosphatidylglycerols, phosphatidylserins, phosphatidylinositols, phosphatidic acids, chems and further anionic sterol-derivatives, , cetylphosphate, diacylglycerol hemisuccinates and cardiolipins and/or derivatives of such lipids.

**39.** Use of lipid assemblies as claimed in any of claims 1 to 38 wherein said lipid assemblies include fusogenic lipids, selected from the group comprising DOPE, lysolipids or free fatty acids or mixtures thereof.

**40.** Use of lipid assemblies as claimed in any of claims 1 to 39 wherein said lipid assemblies are formed from a lipid phase having an amphoteric character.

**41.** Use of lipid assemblies as claimed in any of claims 1 to 40 wherein said lipid assemblies are liposomes or amphoteric liposomes of various size and lamellarity.

**42.** Use of lipid assemblies as claimed in claim 41 wherein said amphoteric liposomes are unilamellar, oligolamellar or multilamellar and wherein the size of said amphoteric liposomes vary between 50 and 1000 nm, preferred between 50 and 500 nm and more preferred between 70 and 250 nm.

**43.** Use of lipid assemblies as claimed in claim 42 wherein said amphoteric liposomes are formed from a lipid phase comprising one or more amphoteric lipids.

**44.** Use of lipid assemblies as claimed in claim 43 wherein said amphoteric lipids are selected from the group comprising HistChol, HistDG, isoHistSuccDG, Acylcarnosin, HCChol, Hist-PS and EDTA-Chol.

**45.** Use of lipid assemblies as claimed in claim 42 wherein said amphoteric liposomes are formed from a lipid phase comprising (i) a stable cationic lipid and a chargeable anionic lipid, (ii) a chargeable cationic lipid and chargeable anionic lipid or (iii) a stable anionic lipid and a chargeable cationic lipid.

**46.** Use of lipid assemblies as claimed in any of claims 1 to 45 wherein the amount of said one or more lipids with one or more transfection enhancer elements is between 0.1 % and 90 % of the total lipid phase.

**47.** Use of lipid assemblies as claimed in any of claims 41 to 46 wherein said lipid assemblies are liposomes and the lipid phase comprises combinations selected from the group comprising

| | |
|---|---|
| POPC/DOPE/Pal-PE | 22:68:10 |
| POPC/DOPE/Pal-PE | 17:53:30 |
| POPC/DOTAP/Chems/Pal-PE | 15:28:47:10 |
| POPC/DOPE/Mochol/Chems/Pal-PE | 12:38:20:20:10 |
| POPC/DOPE/Mochol/Chems/Pal-PE | 5:22:42:21:10 |
| POPC/DOPE/Mochol/DOG-Succ/Pal-PE | 7:20:21:42:10 |
| DOPE/Mochol/Chems/Pal-PE | 50:20:20:10 |

| | |
|---|---|
| POPC/DOPE/Deca-PE | 22:68:10 |
| POPC/DOPE/Deca-PE | 17:53:30 |
| POPC/DOTAP/Chems/Deca-PE | 15:28:47:10 |
| POPC/DOPE/Mochol/Chems/Deca-PE | 12:38:20:20:10 |
| POPC/DOPE/Mochol/Chems/Deca-PE | 5:22:42:21:10 |
| POPC/DOPE/Mochol/DOG-Succ/Deca-PE | 7:20:21:42:10 |
| DOPE/Mochol/Chems/Deca-PE | 50:20:20:10 |

**48.** Use of lipid assemblies as claimed in any of claims 1 to 24 wherein said TEE's are complexed with said lipid

assemblies using ionic interactions.

**49.** Use of lipid assemblies as claimed in 48 wherein said TEE's are linked to a polycationic element and combined with anionic lipid assemblies.

**50.** Use of lipid assemblies as claimed in claim 49 wherein said polycationic elements are selected from the group comprising polyethylenimine, spermine, thermine, spermidine, putrescine or polymers or oligomers from lysine, ornithine or arginine.

**51.** Use of lipid assemblies as claimed in 48 wherein said TEE's are linked to a polyanionic element and combined with cationic lipid assemblies.

**52.** Use of lipid assemblies as claimed in claim 51 wherein said polyanionic elements are selected from the group comprising polymers or oligomers of acrylic acid, methacrylic acid, glutamic acid, aspartic acid.

**53.** Use of lipid assemblies as claimed in any of claims 1 to 52 wherein said lipid assemblies sequester active pharmaceutical ingredients.

**54.** Use of lipid assemblies as claimed in claim 53 wherein said sequestered active pharmaceutical ingredients are nucleic acid-based drugs.

**55.** Use of lipid assemblies as claimed in claim 54 wherein said nucleic acids are oligonucleotides, polynucleotides or DNA plasmids.

**56.** Use of lipid assemblies as claimed in claim 55 wherein said nucleic acids are capable of being transcribed in a vertebrate cell into one or more RNAs, said RNAs being mRNAs, shRNAs, miRNAs or ribozymes, said mRNAs coding for one or more proteins or polypeptides.

**57.** Use of lipid assemblies as claimed in claim 56 wherein said nucleic acid is a circular DNA plasmid, a linear DNA construct or an mRNA.

**58.** Use of lipid assemblies as claimed in claim 55 wherein said nucleic acid is an oligonucleotide.

**59.** Use of lipid assemblies as claimed in claim 58 wherein said oligonucleotide is a decoy oligonucleotide, an antisense oligonucleotide, a siRNA, an agent influencing transcription, an agent influencing splicing, Ribozymes, DNAzymes or Aptamers.

**60.** Use of lipid assemblies as claimed in claim 58 or claim 59 wherein said oligonucleotides comprise naturally occurring or modified nucleosides such as DNA, RNA, locked nucleic acids (LNA's), 2'O-methyl RNA (2'Ome), 2' O-methoxyethyl RNA (2'MOE) in their phosphate or phosphothioate forms or Morpholinos or peptide nucleic acids (PNA's).

**61.** Use of lipid assemblies as claimed in claim 58, claim 59 or claim 60 wherein said oligonucleotide is an antisense oligonucleotide of 8 to 50 basepairs length.

**62.** Use of lipid assemblies as claimed in claim 58, claim 59 or claim 60 wherein said oligonucleotide is a siRNA of 15 to 30 basepairs length.

**63.** Use of lipid assemblies as claimed in claim 58, claim 59 or claim 60 wherein said oligonucleotide is a decoy oligonucleotide of 15 to 30 basepairs length.

**64.** Use of lipid assemblies as claimed in claim 58, claim 59 or claim 60 wherein said oligonucleotide is an agent influencing the transcription of 15 to 30 basepairs length.

**65.** Use of lipid assemblies as claimed in claim 58, claim 59 or claim 60 wherein said oligonucleotide is a DNAzyme of 25 to 50 basepairs length.

**66.** Use of lipid assemblies as claimed in claim 58, claim 59 or claim 60 wherein said oligonucleotide is a Ribozyme of 25 to 50 basepairs length.

**67.** Use of lipid assemblies as claimed in claim 58, claim 59 or claim 60 wherein said oligonucleotide is a Aptamer of 15 to 60 basepairs length.

**68.** Lipids comprising one or more transfection enhancer elements according to the general formula (I)

Lipid- Hydrophobic element - pH sensitive hydrophilic element (I)

wherein said pH-responsive hydrophilic element
comprises weak acids having a pKa of between 2 and 6, preferred of between 3 and 5
or
is a zwitterionic structure comprising a combination of weak or strong acidic groups with weak bases having a pKa of between 3 and 8, preferred of between 4.5 and 7.
and
wherein said hydrophobic element comprises linear, branched or cyclic chains with a minimum chain length of 6 elements.
and wherein said lipids are other than one of the following structures (III)

PE - amid linkage - X - COOH (III)

wherein X is a carbon containing linear chain having a chain length of between 3 to 20 atoms and having various degrees of saturation and/or heteroatom compositions and/or substituents and the COOH-group
(IV):

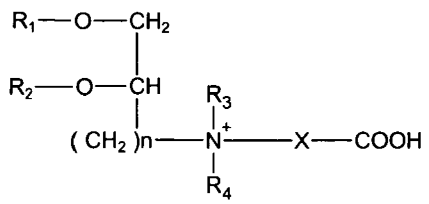

(IV)

wherein X is a straight saturated alkyl chain having a chain length of between 2 and 10 C-atoms and $R_1$, $R_2$, $R_3$ and $R_4$ are independently linear or branched, unsubstituted or substituted $C_{1-23}$ alkyl, acyl, alkylene, heteroalkyl groups having 0 to 6 sites of unsaturation, cyclic and aryl groups, the groups comprising from 0 to 5 heteroatoms, in which the substituent groups are $-O-(CH_2)_x-CH_3$; $-S-(CH_2)_x-CH_3$; $X-(CH_2)_k$, wherein X is a halide, and $-N((CH_2)_k-CH_3)_2$, wherein the alkyl groups of the substituents comprise from 0-2 heteroatoms, and k is 0-4 and wherein $R_1$ and $R_2$ can further be independently H and
n is 1 to 6
or (V):

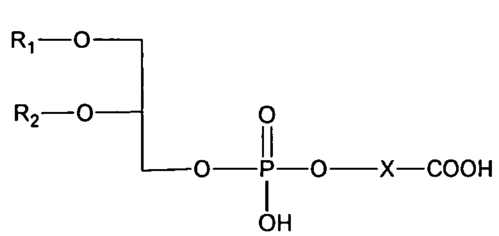

(V)

wherein $R_1$ and $R_2$ independently are hydrogen atoms or $C_1$-$C_{24}$ straight chain or branched alkyl or acyl chains optionally containing double and triple bonds and wherein X is an aliphatic and/or cycloaliphatic hydrocarbon chain with 6-20 carbon-atoms optionally substituted by aryl rests, cycloalkyls with 3-6 carbon atoms, hydroxyl and/or further carboxylic functions.

**69.** Lipids comprising one or more transfection enhancer elements as claimed in claim 68 wherein the lipid moiety to which the pH sensitive transfection enhancer elements are linked is selected from the group comprising phospholipids and their lyso forms, sphingolipids and their lyso forms, sterols like cholesterols and derivatives thereof, diacylglycerols/ dialkylglycerols, monacylglycerols/ monoalkylglycerols, monoester, diesters, monoethers or diethers of glyceric acid, sphingosines/ phytosphingosines/ sphinganines and N-substituted derivatives thereof, ceramides, 1,2-Diacyl-3-aminopropanes/ 1,2 -Dialkyl-3-aminopropanes, 1- or 2- monoacyl - 3 aminopropanes/ 1-or 2- monoalkyl-3-aminopropanes, dialkylamines, monoalkylamines, fatty acids, dicarboxylic acid alkyl ester, tricarboxylic acid dialkyl ester optionally substituted with -OH groups or esters of tartaric acid with long chain alcohols or esters of tartaric acid with long chain carboxylic acids.

**70.** Lipids comprising one or more transfection enhancer elements as claimed in claim 69 wherein (i) the acyl- and alkyl-chains of said amphiphilic lipid substances comprise independently 8-30 carbon atoms and 0, 1 or 2 ethylenically unsaturated bonds (ii) said sterols are cholestane derivatives.

**71.** Lipids comprising one or more transfection enhancer elements as claimed in any of claims 68 to 70 wherein said pH sensitive transfection enhancer elements are chemically linked or grafted on the polar head group of said lipid.

**72.** Lipids comprising one or more transfection enhancer elements as claimed in any of claims 68 to 71 wherein said lipids include chemical linkers between the graft and the pH sensitive transfection enhancer elements selected from the group comprising -O-, - S-, -N(H)C(O)-, -C(O)O-, -OC(O)N(H)-, -C(O)-, -C(O)-N(H)-, -N(H)-C(O)-O-, - CH=N-, -O-C(O)-, -N=CH-, -S-S-, non-branched, branched or cyclic alkyl, alkylene or alkynyl with $C_1$-$C_6$ atoms and optionally substituted with one or more -OH, -$NR_2$, - COOH or sugars or mixtures thereof; -$PO_4$--; -$PO_3$--; pyrophosphate; -$SO_4$-; -$SO_3$-; - NH-; -NR-; sugars and derivatives thereof; amino acids; Di- or Tripeptides, α-hydroxyacids or β-hydroxy acids or dihydroxyacids.

**73.** Lipids comprising one or more transfection enhancer elements as claimed in any of claims 68 to 72 wherein said polar headgroup of the lipid is substituted.

**74.** Lipids comprising one or more transfection enhancer elements as claimed in claim 73 wherein said substituents are polar and selected from the group comprising -OH, - COOH, -$NH_2$, -NHR, -$NR_2$, sugars and derivatives thereof, amino acids and derivatives thereof, -$OPO_3^{2-}$, -$OPO_2^{2-}$, -$OSO_3$-; -$OSO_2^-$ or mixtures thereof.

**75.** Lipids comprising one or more transfection enhancer elements as claimed in any of claims 68 to 74 wherein said lipids contain more than one hydrophilic polar head group or complex hydrophilic head groups that allow substitution on various positions.

**76.** Lipids comprising one or more transfection enhancer elements as claimed in claim 75 wherein said lipids are selected from the group comprising esters of tartaric acid with long chain alcohols; derivatives of maleic acid; esters of fatty acids with sugars such as glucose, sucrose or maltose; alkyl glycosides and derivatives thereof, such as dodecyl-β-D-glucopyranoside or dodecyl-β-D-maltoside; derivatives of alkyl-ethylenglycol detergents, such as Brij35, Genapol series, Thesit or Phosphatidylinositols and derivatives thereof.

**77.** Lipids comprising one or more transfection enhancer elements as claimed in any of claims 68 to 76 wherein said lipids are selected from the group comprising following compounds:

| | |
|---|---|
| 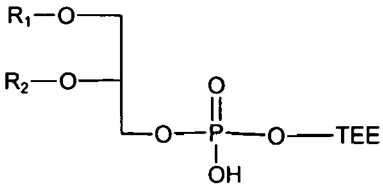 | 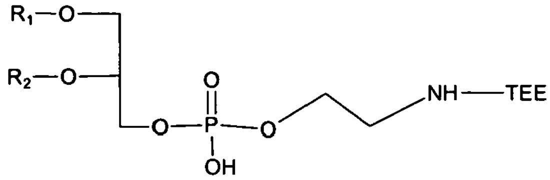 |
| 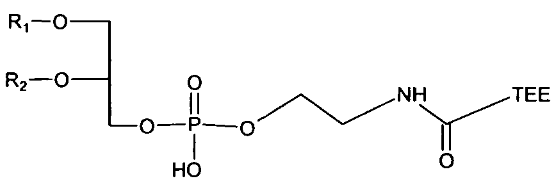 | 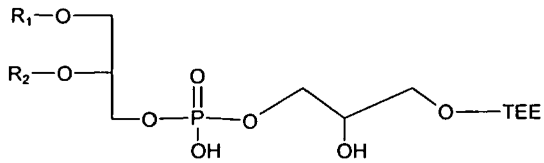 |
| 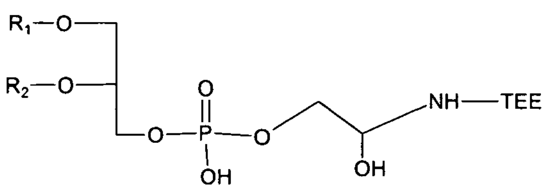 | 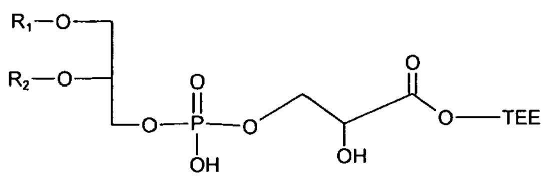 |
| 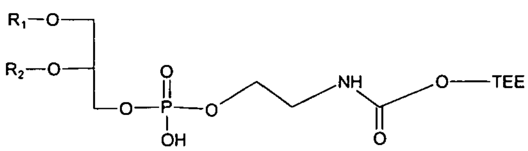 | 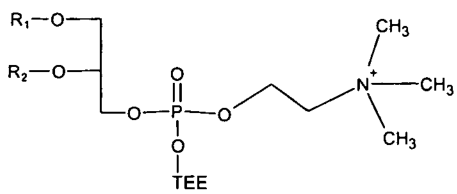 |
| 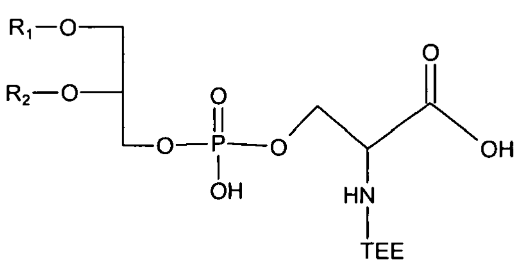 | 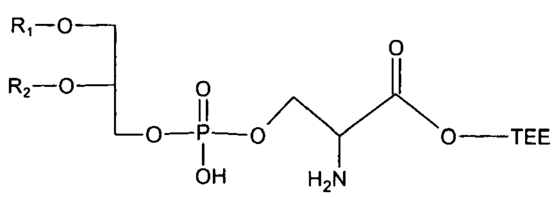 |

| | |
|---|---|
| 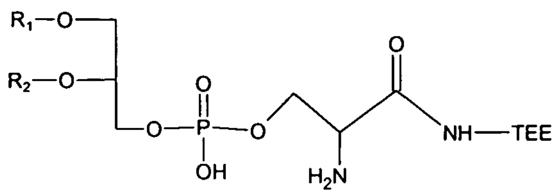 | 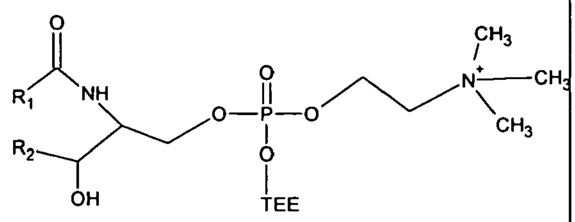 |
| 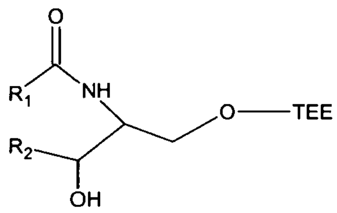 | 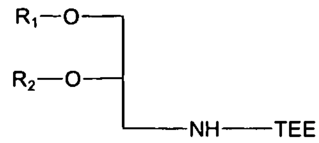 |

**78.** Lipids comprising one or more transfection enhancer elements as claimed in any of claims 68 to 76 wherein said lipids are selected from the group comprising the following compounds:

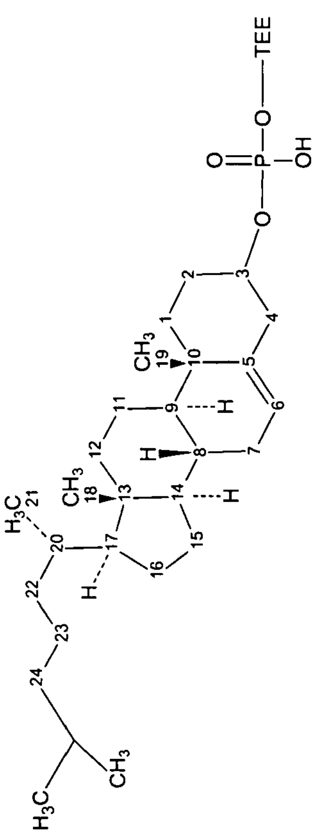

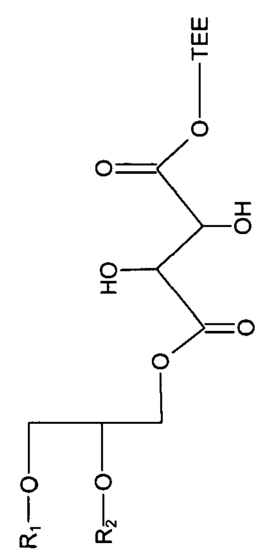

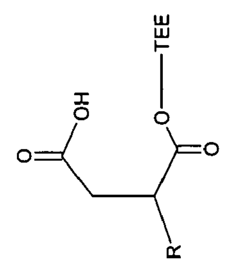

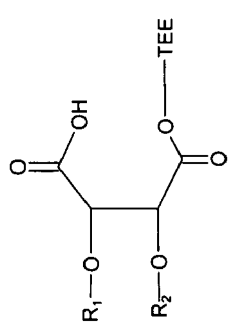

(continued)

| | |
|---|---|
| 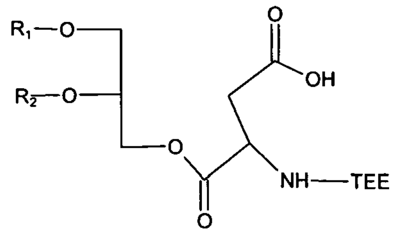 | 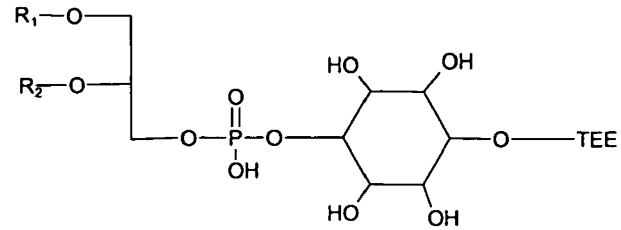 |
| $H_3C-(CH_2)_x-O-(CH_2CH_2O)_y-TEE$<br>x=8-30, optionally comprising unsaturated bonds and y=2-30 | |
| 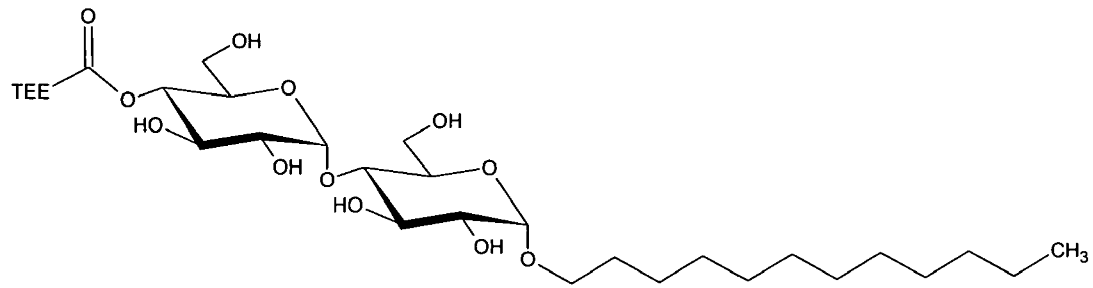 | |

**79.** Lipids comprising one or more transfection enhancer elements as claimed in any of claims 68 to 76 wherein said lipids are selected from the group comprising the following compounds:

| | |
|---|---|
| 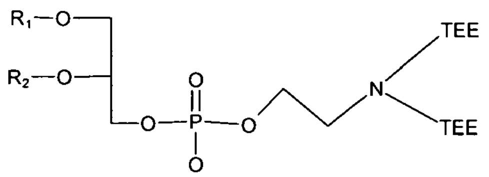 | 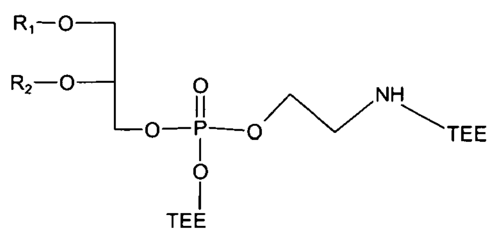 |
| 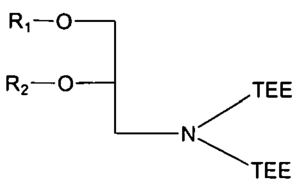 | 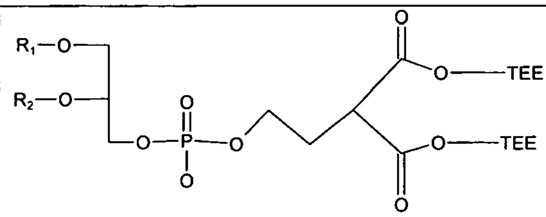 |

| | |
|---|---|
| 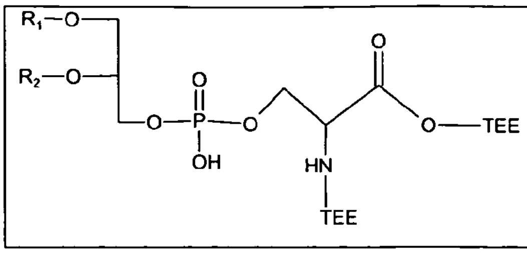 | |

**80.** Lipids comprising one or more transfection enhancer elements as claimed in any of claims 68 to 76 wherein said lipids are selected from the group comprising the following compounds:

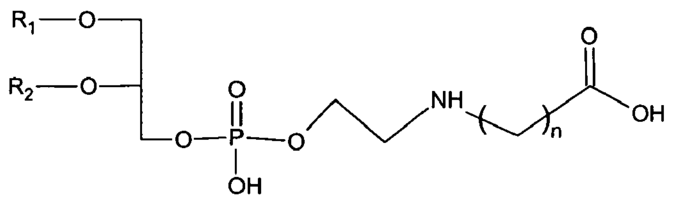

wherein $R_1$ and $R_2$ independently are $C_8$-$C_{30}$ alkyl or acyl chains with 0, 1 or 2 ethylenically unsaturated bonds and n=6-40,

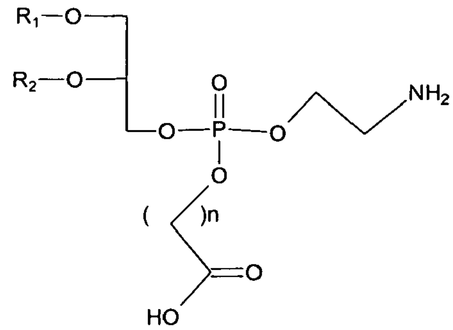

wherein $R_1$ and $R_2$ independently are $C_8$-$C_{30}$ alkyl or acyl chains with 0, 1 or 2 ethylenically unsaturated bonds and n=6-40,

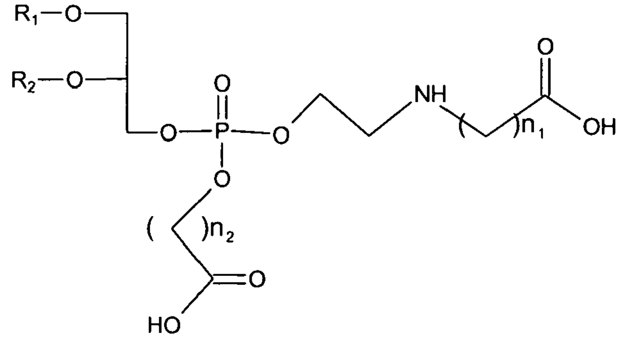

wherein $R_1$ and $R_2$ independently are $C_8$-$C_{30}$ alkyl or acyl chains with 0, 1 or 2 ethylenically unsaturated bonds and n=6-40,

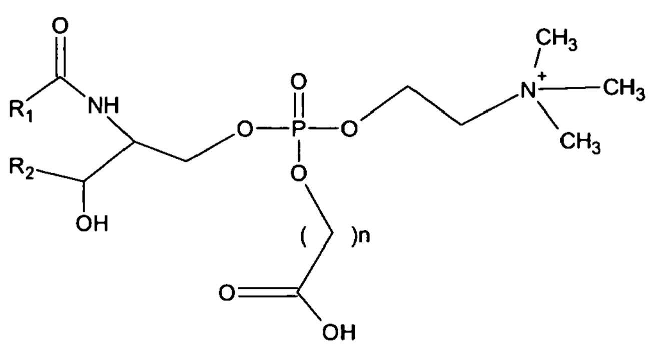

wherein $R_1$ and $R_2$ independently are $C_8$-$C_{30}$ alkyl or acyl chains with 0, 1 or 2 ethylenically unsaturated bonds and n=6-40,

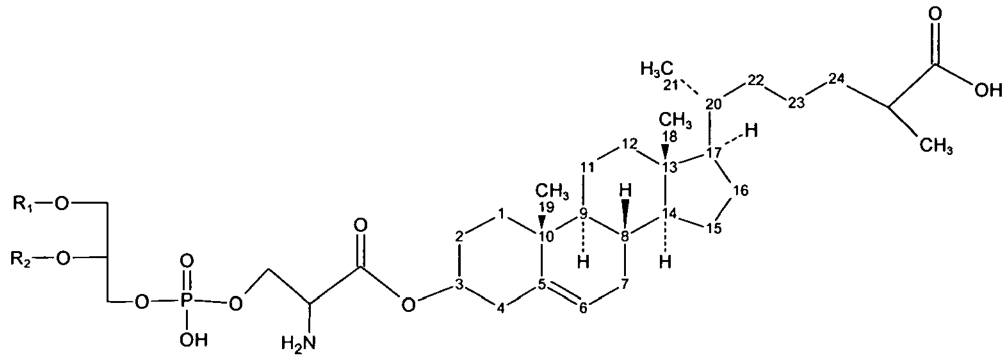

wherein $R_1$ and $R_2$ independently are $C_8$-$C_{30}$ alkyl or acyl chains with 0, 1 or 2 ethylenically unsaturated bonds and n=6-40,

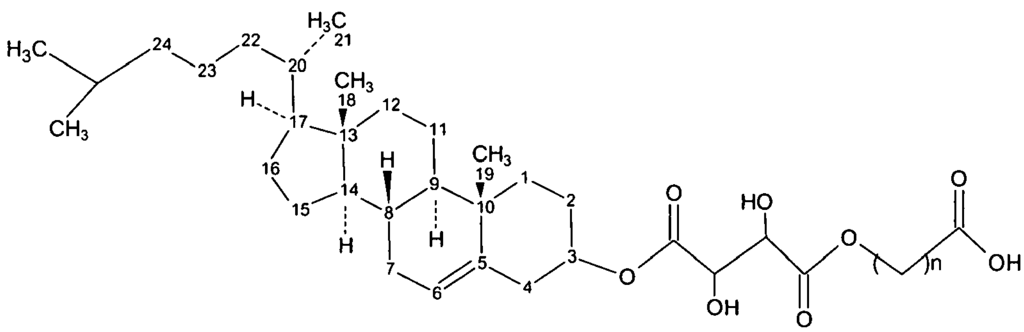

wherein n=6-40,

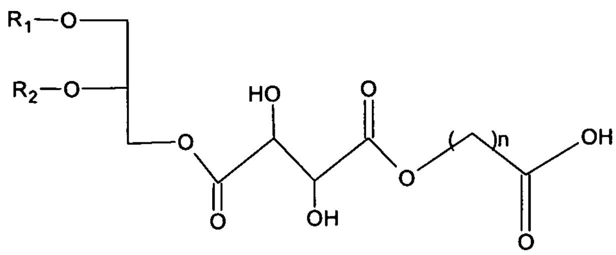

wherein $R_1$ and $R_2$ independently are $C_8$-$C_{30}$ alkyl or acyl chains with 0, 1 or 2 ethylenically unsaturated bonds and n=6-40,

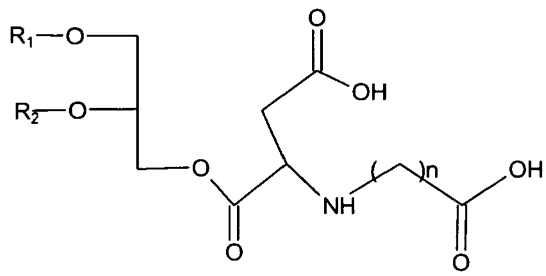

wherein $R_1$ and $R_2$ independently are $C_8$-$C_{30}$ alkyl or acyl chains with 0, 1 or 2 ethylenically unsaturated bonds and n=6-40,

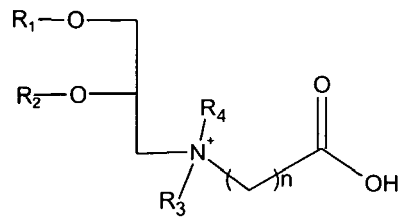

wherein $R_1$ and $R_2$ independently are $C_8$-$C_{30}$ alkyl or acyl chains with 0, 1 or 2 ethylenically unsaturated bonds, $R_3$ and $R_4$ are independently H or $C_1$-$C_6$ alkyls and n=11-40.

**81.** Lipid assemblies comprising one or more lipids as claimed in any of claim 68 to 80 wherein said lipid assemblies are selected from the group comprising liposomes of various size and lamellarity, micelles, inverted micelles, cubic or hexagonal lipid phases, cochleates, emulsions, double emulsions or other multimeric assemblies that are substantially build from lipids, oils or amphiphiles.

**82.** Lipid assemblies as claimed in claim 81 wherein said lipid assemblies sequester at least one active pharmaceutical ingredient.

**83.** Lipid assemblies as claimed in claim 82 wherein said active pharmaceutical ingredient is a nucleic acid-based drug, selected from the group comprising DNA plasmids, polynucleotides and oligonucleotides.

**84.** Amphoteric liposomes comprising one or more lipids with one or more transfection enhancer elements according to the general formula (I)

Lipid- Hydrophobic element - pH sensitive hydrophilic element (I)

wherein said pH-responsive hydrophilic element
comprises weak acids having a pKa of between 2 and 6, preferred of between 3 and 5
or
is a zwitterionic structure comprising a combination of weak or strong acidic groups with weak bases having a pKa of between 3 and 8, preferred of between 4.5 and 7.
and
wherein said hydrophobic element comprises linear, branched or cyclic chains with a minimum chain length of 6 elements.

**85.** Amphoteric liposomes as claimed in claim 84 wherein said liposomes sequester at least one active pharmaceutical ingredient.

**86.** Amphoteric liposomes as claimed in claim 85 wherein said active pharmaceutical ingredient is a nucleic acid -based drug, selected from the group comprising DNA plasmids, polynucleotides and oligonucleotides.

**87.** A pharmaceutical composition comprising pharmaceutically active ingredients sequestered in lipid assemblies or amphoteric liposomes as claimed in any of claims 81 to 86 and a pharmaceutically acceptable vehicle therefore.

**88.** Use of a pharmaceutical composition as claimed in claim 87, for the treatment or prophylaxis of inflammatory, immune or autoimmune disorders and/or cancer of humans or non-human animals.

**89.** A method of treating a human or non-human animal by administering a pharmaceutical composition as claimed in any of claims 87 to 88, wherein said lipid assemblies sequestering an active agent and targeting specific organ or organs, tumours or sites of infection or inflammation.

*FIG. 1*

log D for zwitter ion structures

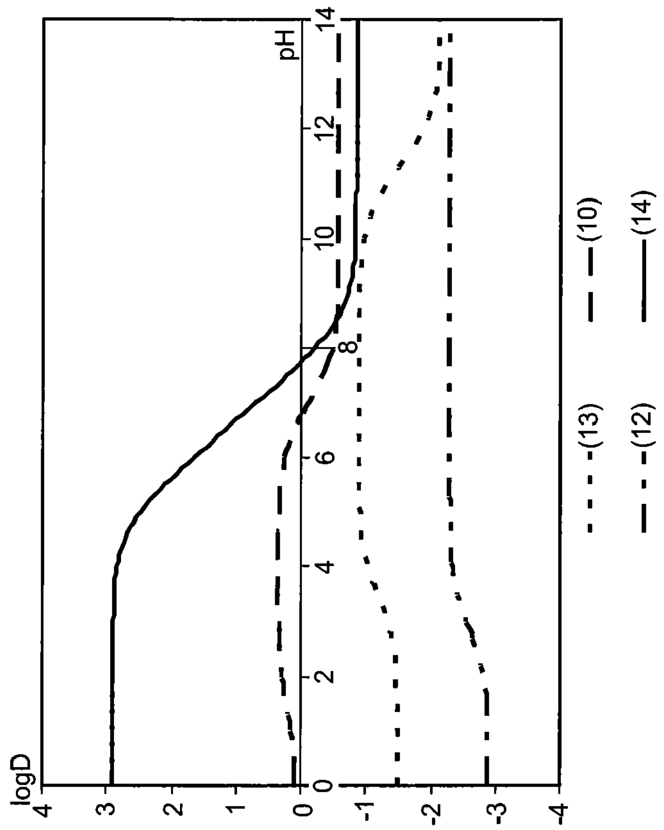

***FIG. 2***
logD vs . pH

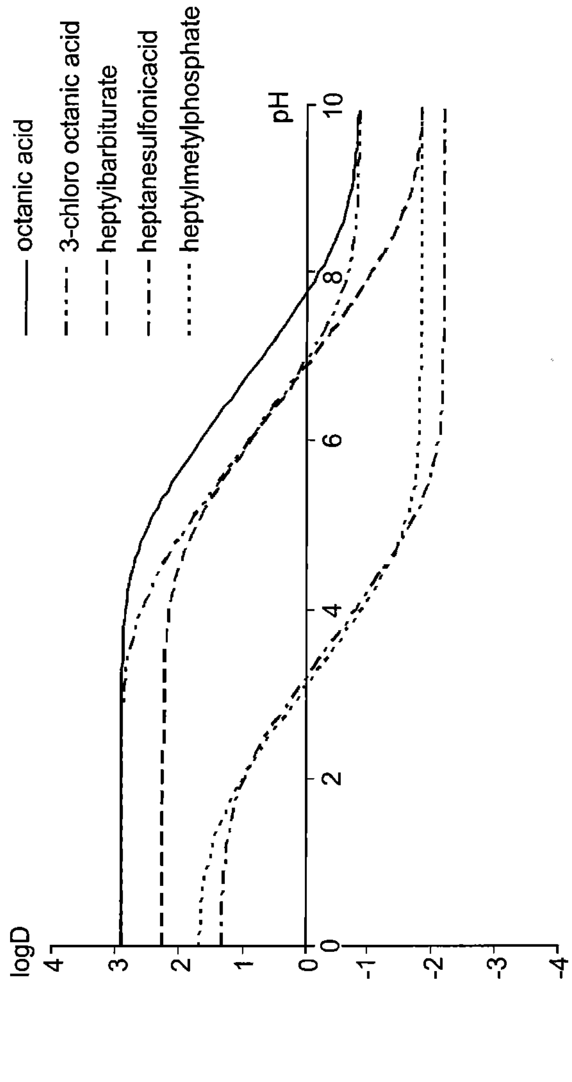

***FIG. 3***
logD vs . pK

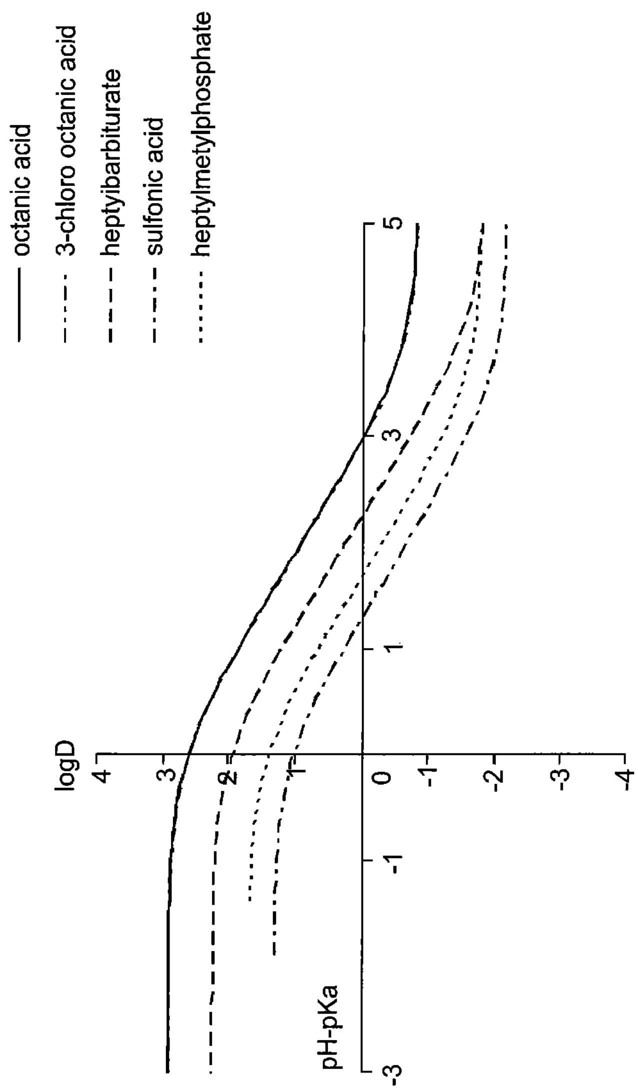

*FIG. 4*

increment logD vs . pH

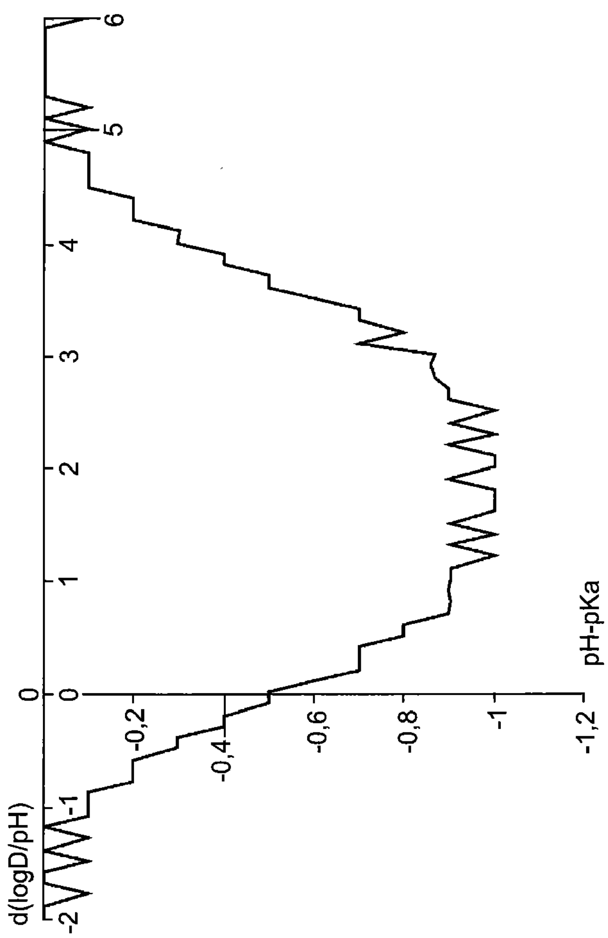

*FIG. 5*

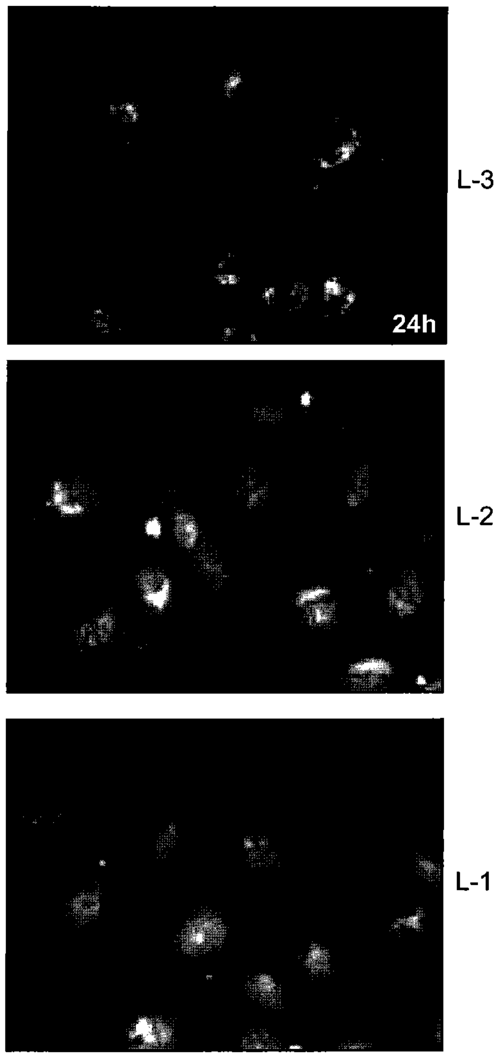

FIG. 6

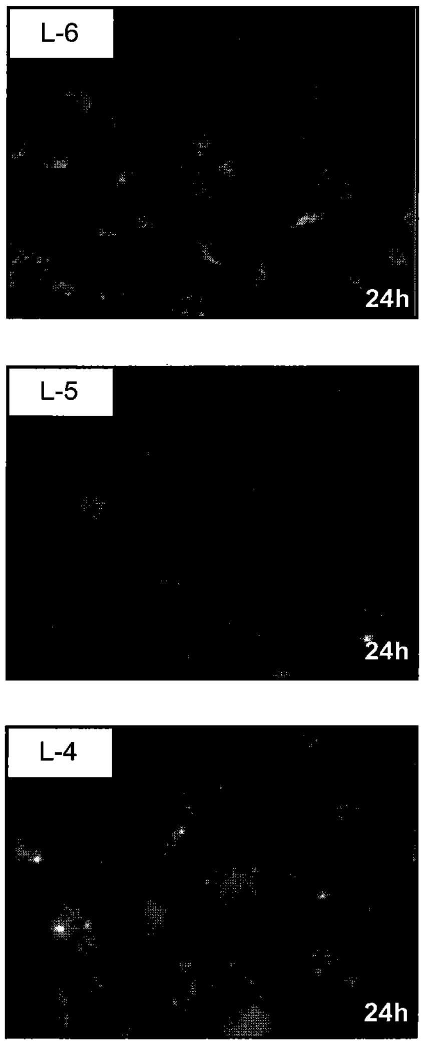

FIG. 7

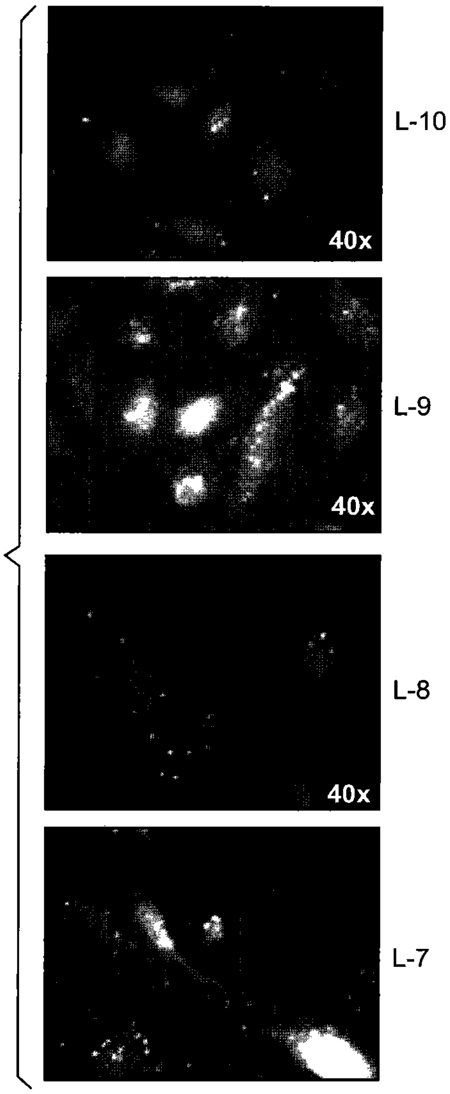

FIG. 8

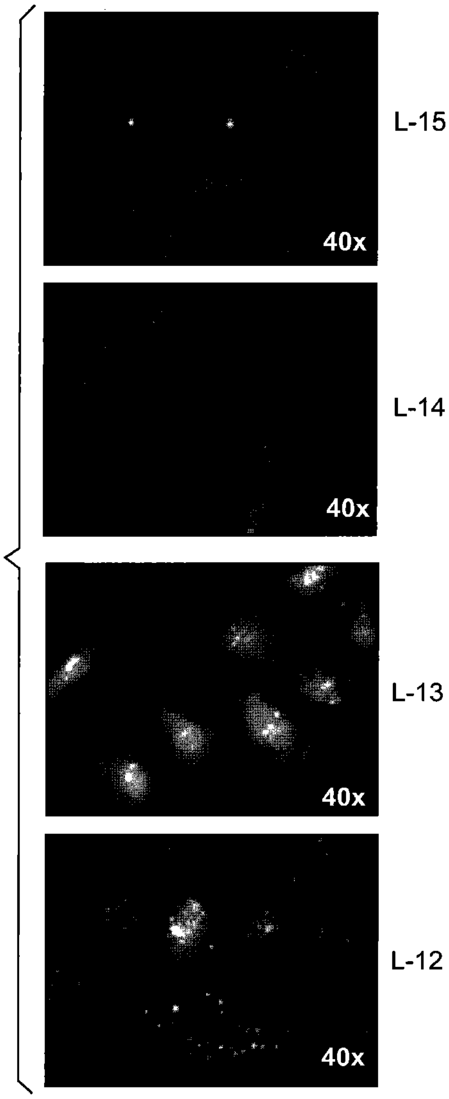

FIG. 9

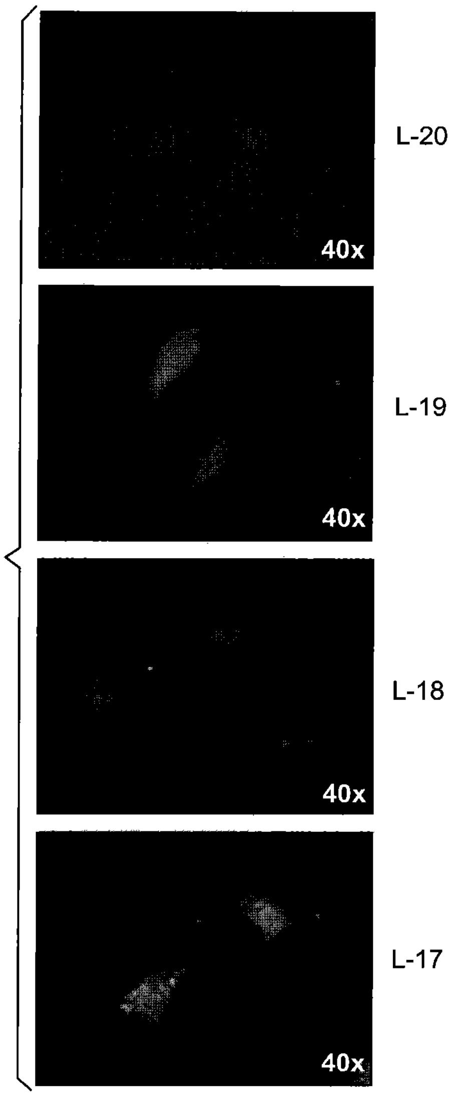

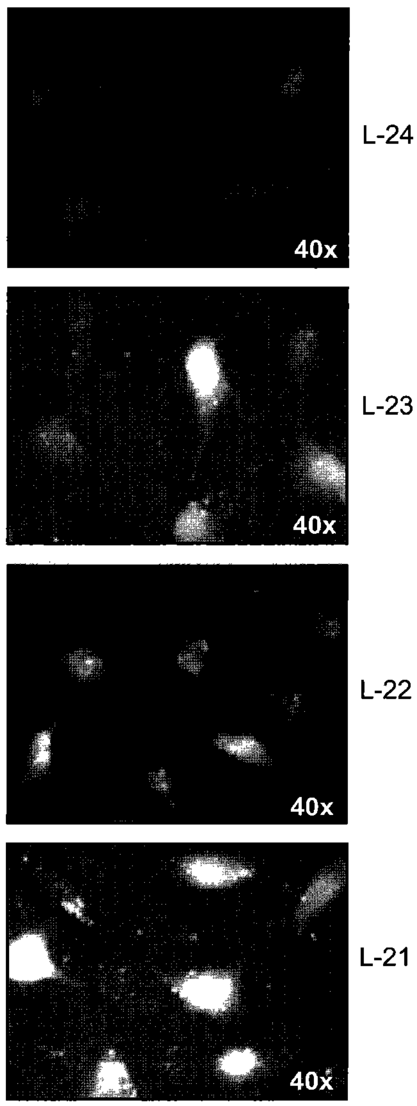


FIG. 10

FIG. 11

C-1 C-2

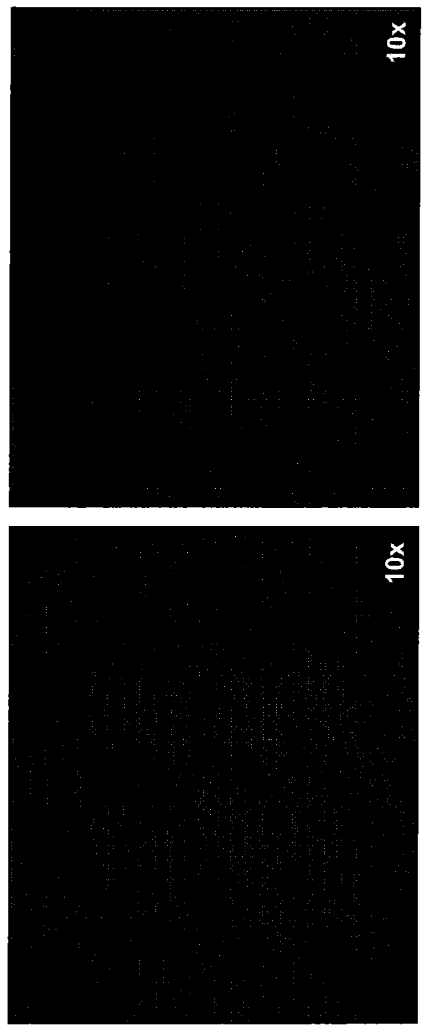

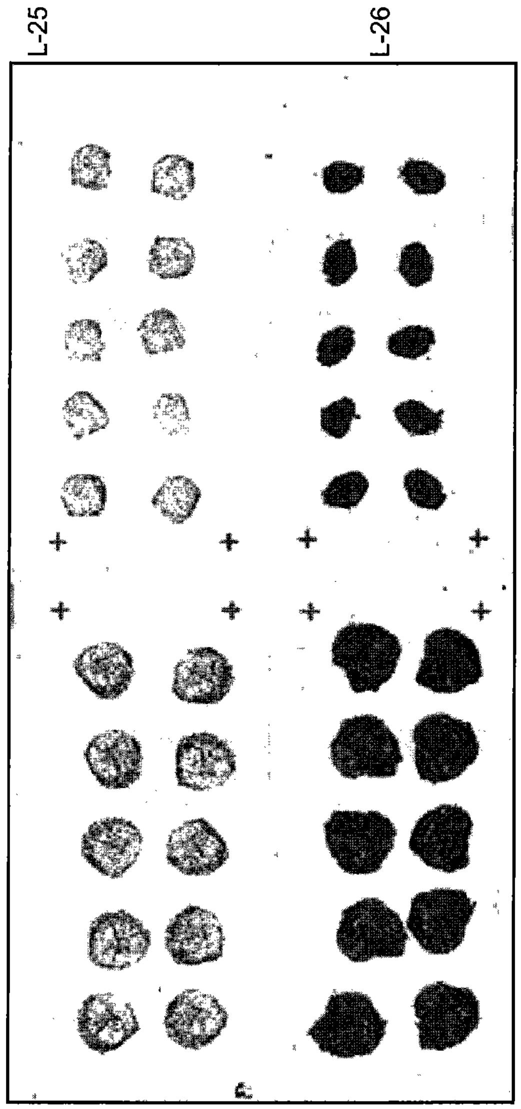


FIG. 12

European Patent Office

# PARTIAL EUROPEAN SEARCH REPORT

which under Rule 45 of the European Patent Convention shall be considered, for the purposes of subsequent proceedings, as the European search report

Application Number
EP 06 25 6450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 96/05218 A (COMMW SCIENT IND RES ORG [AU]; WHITTAKER ROBERT [AU]; CAMERON FIONA HE) 22 February 1996 (1996-02-22)<br>* page 1, line 3 - line 10 *<br>* page 1, line 21 - page 3, line 17 *<br>* page 4, line 35 - page 5, line 2 *<br>----- | 1-89 | INV.<br>A61K48/00<br>A61K47/48 |
| X | US 6 989 434 B1 (GEBEYEHU GULILAT [US] ET AL) 24 January 2006 (2006-01-24)<br>* column 1, line 14 - line 18 *<br>* column 12, line 53 - line 67 *<br>* column 15, line 58; claim 60 *<br>scheme 4, scheme 8 8<br>* column 17, line 31 - line 33; figures XII,XVIII *<br>-----<br>-/-- | 1-89 | |

TECHNICAL FIELDS SEARCHED (IPC)

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 5 July 2007 | Bendl, Ernst |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 06 25 6450

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | GUO X ET AL: "STERIC STABILIZATION OF FUSOGENIC LIPOSOMES BY A LOW-PH SENSITIVE PEG-DIORTHO ESTER-LIPID CONJUGATE" BIOCONJUGATE CHEMISTRY, ACS, WASHINGTON, DC, US, vol. 2, no. 12, March 2001 (2001-03), pages 291-300, XP001095697 ISSN: 1043-1802 * abstract * ----- | 1-89 | |
| X | US 6 124 270 A (HAENSLER JEAN [FR]) 26 September 2000 (2000-09-26) * column 1, line 7 - line 9 * * column 6 - column 7 * ----- | 1-89 | |
| X | WO 91/16024 A (VICAL INC [US]) 31 October 1991 (1991-10-31) * page 1, line 6 - line 12 * * page 17, line 20 - page 18, line 15 * * page 20, line 10 - line 25 * * page 21; claim 1 * ----- | 1-89 | TECHNICAL FIELDS SEARCHED (IPC) |

2

EPO FORM 1503 03.82 (P04C10)

European Patent Office

**INCOMPLETE SEARCH SHEET C**

Application Number
EP 06 25 6450

Although claims 1-67 are directed to a method of treatment of the human/animal body (Article 52(4) EPC), the search has been carried out and based on the alleged effects of the compound/composition.

-----

Claim(s) searched incompletely:
1-89

Reason for the limitation of the search:

The initial phase of the search revealed a very large number of documents relevant to the issue of novelty. So many documents were retrieved that it is impossible to determine which parts of the claims may be said to define subject-matter for which protection might legitimately be sought (Article 84 EPC). For these reasons, a meaningful search of the whole claimed subject matter of all claims could not be carried out (Rule 45 EPC). The extent of the search was consequently limited.

The search of all claims was restricted to the general concept of using compounds of formula (I) for enhancing transfection.

# ANNEX TO THE EUROPEAN SEARCH REPORT ON EUROPEAN PATENT APPLICATION NO. EP 06 25 6450

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2007

| Patent document cited in search report | Publication date | Patent family member(s) | Publication date |
|---|---|---|---|
| WO 9605218 A | 22-02-1996 | AT 253588 T | 15-11-2003 |
| | | CA 2197653 A1 | 22-02-1996 |
| | | CN 1158619 A | 03-09-1997 |
| | | DE 69532081 D1 | 11-12-2003 |
| | | DE 69532081 T2 | 09-09-2004 |
| | | DK 777679 T3 | 01-12-2003 |
| | | EP 0777679 A1 | 11-06-1997 |
| | | FI 970648 A | 07-04-1997 |
| | | JP 10509862 T | 29-09-1998 |
| | | JP 3763577 B2 | 05-04-2006 |
| | | NZ 290757 A | 29-09-2000 |
| | | US 5906922 A | 25-05-1999 |
| US 6989434 B1 | 24-01-2006 | NONE | |
| US 6124270 A | 26-09-2000 | AT 223716 T | 15-09-2002 |
| | | AU 704369 B2 | 22-04-1999 |
| | | AU 5651796 A | 30-10-1996 |
| | | CA 2192597 A1 | 17-10-1996 |
| | | DE 69623559 D1 | 17-10-2002 |
| | | DE 69623559 T2 | 07-08-2003 |
| | | DK 769942 T3 | 06-01-2003 |
| | | EP 0769942 A1 | 02-05-1997 |
| | | ES 2181885 T3 | 01-03-2003 |
| | | FR 2732895 A1 | 18-10-1996 |
| | | WO 9632102 A1 | 17-10-1996 |
| | | JP 10501822 T | 17-02-1998 |
| | | PT 769942 T | 31-01-2003 |
| WO 9116024 A | 31-10-1991 | AT 181319 T | 15-07-1999 |
| | | AU 7854791 A | 11-11-1991 |
| | | CA 2079814 A1 | 20-10-1991 |
| | | DE 69131347 D1 | 22-07-1999 |
| | | DE 69131347 T2 | 30-03-2000 |
| | | DK 523189 T3 | 13-12-1999 |
| | | EP 0523189 A1 | 20-01-1993 |
| | | ES 2134775 T3 | 16-10-1999 |
| | | GR 3030974 T3 | 31-12-1999 |
| | | JP 2538474 B2 | 25-09-1996 |
| | | US 5459127 A | 17-10-1995 |
| | | US 5264618 A | 23-11-1993 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02066490 A
- WO 02066012 A
- WO 03070735 A
- WO 03070220 A
- WO 03066489 A
- US 4458066 A
- WO 8604232 A, Kung
- WO 9719675 A, Wheeler
- US 6294191 B
- WO 06105361 A
- WO 0164330 A
- WO OS105152 A
- WO 06069782 A
- WO OS007196 A
- WO 0234236 A
- US 5965434 A
- US 5635487 A
- US 6756054 B
- WO 06016097 A
- US 5785992 A
- WO 04035523 A
- WO 02066120 A
- WO 02066489 A
- WO 9821322 A
- DE 19753182
- EP 1392341 A
- WO 9932619 A
- WO 02055693 A

### Non-patent literature cited in the description

- **THOMAS et al.** *Nature Reviews, Genetics,* 2003, vol. 4, 346-358
- **FILION et al.** *BBA,* 1997, vol. 1329 (2), 345-356
- **DASS.** *J. Pharm. Pharmacol,* 2002, vol. 54 (5), 593-601
- **HIRKO et al.** *Curr. Med. Chem,* 2003, vol. 10 (14), 1185-1193
- **MILLER N ; VILE R.** *FASEB J,* 1995, vol. 9, 190-199
- **STEGMANN et al.** *EMBO J.,* 1990, vol. 9 (13), 4231-4241
- **NARANG et al.** *Meth. Enzymol,* 1979, vol. 68, 90-99
- **BROWN et al.** *Method Enzymol.,* 1979, vol. 68, 109-151
- **BEAUCAGE et al.** *Tetrahedron Lett.,* 1981, vol. 22, 1859-1862
- **MATTEUCCI et al.** *J. Am. Chem. Soc.,* 1981, vol. 103, 3185-3191
- **KULIKOWSKA et al.** *Biochim. Acta Pol,* 2004, vol. 51, 493-531
- **G. T. HERMANSON.** Bioconjugate Techniques. Academic Press, 1996
- **DEGRIP et al.** *Biochem. J.,* 1998, vol. 330, 667-674
- **MORRISSEY et al.** *Nature Biotechnology,* 2005, vol. 23 (8), 1002-1007
- **WHEELER et al.** *Gene Therapy,* 1999, vol. 6 (2), 271-281
- **BUDKER et al.** *Nature Biotechnology,* 1996, vol. 14 (6), 760-764
- **SPAGNOU et al.** *Biochemistry,* 2004, vol. 43 (42), 13348-13356
- **CHO-CHUNG et al.** *Curr. Opin. Mol. Ther.,* 1999